(19) **Europäisches Patentamt / European Patent Office / Office européen des brevets**

(11) **EP 4 029 945 A1**

(12) **EUROPEAN PATENT APPLICATION**
published in accordance with Art. 153(4) EPC

(43) Date of publication:
**20.07.2022 Bulletin 2022/29**

(21) Application number: **20863502.9**

(22) Date of filing: **11.09.2020**

(51) International Patent Classification (IPC):
*C12P 1/00* (2006.01)     *A23K 10/14* (2016.01)
*A23K 10/16* (2016.01)     *A23K 10/38* (2016.01)
*A23K 20/121* (2016.01)    *A23K 20/137* (2016.01)
*A23K 20/142* (2016.01)    *A23K 20/158* (2016.01)
*A23K 20/163* (2016.01)    *A23K 50/40* (2016.01)
*A23L 5/40* (2016.01)      *A23L 27/00* (2016.01)
*A23L 27/10* (2016.01)     *A23L 27/20* (2016.01)
*C12N 1/16* (2006.01)

(52) Cooperative Patent Classification (CPC):
**A23K 10/14; A23K 10/16; A23K 10/38;
A23K 20/121; A23K 20/137; A23K 20/142;
A23K 20/158; A23K 20/163; A23K 50/40;
A23L 5/40; A23L 27/00; A23L 27/10; A23L 27/20;
A23L 31/15; C12N 1/16;** (Cont.)

(86) International application number:
**PCT/JP2020/034473**

(87) International publication number:
**WO 2021/049619 (18.03.2021 Gazette 2021/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.09.2019 JP 2019167481**

(71) Applicants:
• **Asahi Group Foods, Ltd.
Tokyo, 130-8602 (JP)**

• **Asahi Group Holdings, Ltd.
Tokyo 130-8602 (JP)**

(72) Inventor: **OTSUSHI, Noboru
Tokyo 150-0022 (JP)**

(74) Representative: **Hoffmann Eitle
Patent- und Rechtsanwälte PartmbB
Arabellastraße 30
81925 München (DE)**

(54) **YEAST CELL WALL-DERIVED DECOMPOSITION-CONTAINING COMPOSITION, PRODUCTION METHOD THEREFOR, AND USAGE THEREFOR**

(57) Provided is a method for producing a yeast cell wall-derived decomposition product-containing composition, the method including enzymatically treating a cell wall of yeast with exoglucanase.

**EP 4 029 945 A1**

**(Cont. next page)**

FIG. 3

(52) Cooperative Patent Classification (CPC): (Cont.)
**C12P 1/00; C12P 7/62; C12P 17/12;** Y02P 60/87

**Description**

Technical Field

The present invention relates to a yeast cell wall-derived decomposition product-containing composition, a method for producing the yeast cell wall-derived decomposition product-containing composition, and a beer-like fragrance-imparting composition, a roast flavor-imparting composition, a roast color-imparting composition, food or drink, and a pet food palatability enhancer, each of which contains the yeast cell wall-derived decomposition product-containing composition.

Background Art

A yeast cell body contains various nutrients such as proteins, vitamins, minerals, nucleic acids, glutathione, and dietary fibers, and has widely been used in various fields such as a food field, a bio field, and a cosmetic field.

Use of the yeast cell body includes, for example, use of a yeast extract obtained by extracting ingredients contained in the yeast cell body, and use of the yeast cell wall formed as a residue after separation of a yeast extract in the production of the yeast extract. Among them, the yeast extract is often used because the yeast extract is easily processable by virtue of, for example, its high solubility in water and the ability to be concentrated to about 65% by mass to about 70% by mass.

Meanwhile, the yeast cell wall is insoluble in water and has such a property as very high stability to, for example, heat, and further the yeast cell wall contains abundant polysaccharides. Even in the state of slurry containing the yeast cell wall, therefore, its viscosity considerably increases when the concentration of the slurry is about 15% by mass or higher, which causes difficulties in processing such as making highly concentrating impossible. At present, the yeast cell wall has not been sufficiently effectively used.

In recent years, in the food field and the cosmetic field, there have been increasing demands worldwide for organic, naturally occurring products (natural), additive free, etc., and product developments have been being conducted to meet these demands. Particularly in the European Union (EU), the guideline for traceability in the general food law of CIAA (Confederation of the food and drink industries of the EU) defines criteria for attaching "clean label" indicating that the labeled product is a naturally occurring product (natural) (see Non-Patent Literature 1).

Reaction flavors with flavors such as a beef flavor or a roast flavor have been proposed as products using a yeast extract that can be labeled with "clean label" (see Patent Literature 1). The reaction flavors are obtained as products of the Maillard reaction. The Maillard reaction is a reaction where a reducing monosaccharide and an amino acid as monomers are heated to produce a brown substance. In the EU, when a reducing monosaccharide such as glucose and an amino acid are externally added as sub-materials, they should be displayed as additives, which makes it impossible to label the resultant product with "clean label". The resultant product does not meet the above demands and is problematically unfavorable.

In view thereof, the above proposed reaction flavors are produced in the following manner. Specifically, a yeast extract or a yeast autolysate is step-wise treated with enzymes such as endoprotease, $\alpha$-$\alpha$-trehalase, glucoamylase, endoglucanase, and exoglucanase to obtain yeast-derived products containing yeast-derived amino acids and reducing sugars. The yeast-derived products are centrifuged to remove insoluble ingredients (i.e., yeast cell walls). The resultant yeast extract is incubated under predetermined conditions to cause the Maillard reaction of the yeast-derived amino acids and reducing sugars.

In the above proposed method, it is expected that as a result of treating the yeast extract or yeast autolysate containing the yeast cell walls with endoprotease and then glucanase, certain amounts of amino acids and reducing sugars are produced from the yeast cell walls as well. However, the yeast cell walls are very low in solubility ratio as described above, and the above proposed method has a problem that only part of the ingredients contained in the yeast cell walls can be solubilized to form a lot of precipitates derived from the yeast cell walls, and there is a need to remove insoluble ingredients by centrifugation.

Since only part of the yeast cell wall can be solubilized, there is a need for an operation to remove insoluble ingredients, which causes a problem of low operation and production efficiency. The yeast cell wall is difficult to process and is very poor in the reducing monosaccharide and the amino acid as monomers, which causes another problem that the reaction flavors as in the above proposal cannot be obtained.

Meanwhile, the yeast cell wall is obtained as a residue after separation of the yeast extract, and if its processing properties such as solubility are improved, the yeast cell wall is advantageously available at low cost.

A flavor composition has been proposed that uses the yeast cell wall and is capable of enhancing or imparting a fatty and creamy mouthfeel (see PTL 2). This flavor composition is produced by contacting a slurry of the yeast cell walls with endoglucanase (laminaripentaose-producing-$\beta$-1,3-glucanase) and endoprotease, followed by separating a liquid fraction by solid/liquid separation for removing insoluble ingredients. Thus, there is a problem that its solubility ratio is not sufficiently satisfactory. This method produces amino acids derived from the yeast cell wall, but does not produce reducing monosaccharides as sugars. Since the action of exoglucanase produces non-reducing polysaccharides (pentose oligosaccharides formed by β-1,3 glucoside bonds of glucoses, this flavor composition cannot be used to obtain Maillard reaction products.

Maillard reaction products having flavors such as a meat flavor and a roast flavor are known to contribute to not only improvement in palatability of humans but also improvement in palatability of pets (see, for example, PTL 3 and NPL 2).

Under such circumstances, there are currently strong demands for provision of a method for producing a yeast cell wall-derived decomposition product-containing composition where the method is capable of increasing the solubility ratio and dispersion stability of the yeast cell wall, needs no separation/removal operation of an insoluble fraction of the yeast cell wall, and achieves high operation and production efficiency; and provision of a yeast cell wall-derived decomposition product-containing composition that is producible from only the raw materials derived from the yeast cell wall and has favorable flavors such as a meat flavor and a roast flavor.

Citation List

Patent Literature

PTL 1: Japanese Patent No. 5982696
PTL 2: Japanese Patent Application Laid-Open (JP-A) No. 2018-531586
PTL 3: JP-A No. 2017-86079

Non-Patent Literature

NPL 1: CIAA GUIDELINES, on Regulation (EC) No. 1334/2008, on Flavourings and Certain Food Ingredients with Flavouring Properties for Use in and on Foods
NPL 2: Latest Trends in Pet Foods and Drugs, "Chapter 4 Palatability for Pet Foods", December 16, 2013, pp. 29-35, CMC Publishing

Summary of Invention

Technical Problem

The present invention aims to solve the above existing problems and has an object to achieve the following.

Specifically, an object of the present invention is to provide a method for producing a yeast cell wall-derived decomposition product-containing composition where the method is capable of increasing the solubility ratio and dispersion stability of the yeast cell wall, needs no separation/removal operation of an insoluble fraction of the yeast cell wall, and achieves high operation and production efficiency

Another object of the present invention is to provide a cell wall-derived decomposition product-containing composition that has excellent solubility and dispersion stability.

Another object of the present invention is to provide a yeast cell wall-derived decomposition product-containing composition that has excellent solubility and dispersion stability, is favorable in a roast flavor and a fat and oil feeling, and is favorable in a roast color.

Another object of the present invention is to provide a beer-like fragrance-imparting composition that has excellent solubility and dispersion stability, has less off-flavors, and has a favorable beer-like fragrance.

Another object of the present invention is to provide a roast flavor-imparting composition that has excellent solubility and dispersion stability and is favorable in a roast flavor and a fat and oil feeling.

Another object of the present invention is to provide a roast color-imparting composition that has excellent solubility and dispersion stability and is favorable in a roast color.

Another object of the present invention is to provide food or drink containing the yeast cell wall-derived decomposition product-containing composition.

Another object of the present invention is to provide a pet food palatability enhancer containing the yeast cell wall-derived decomposition product-containing composition.

Solution to Problem

Means for solving the above problems are as follows.

<1> A method for producing a yeast cell wall-derived decomposition product-containing composition, the method including
enzymatically treating a cell wall of yeast with exoglucanase.
<2> The method according to <1>, wherein the enzymatically treating further includes a protease treatment of the cell wall of yeast.
<3> The method according to <2>, wherein the enzymatically treating further includes an exopeptidase treatment of the cell wall of yeast.
<4> The method according to any one of <1> to <3>, further including performing a heat treatment to an enzymatically treated product obtained in the enzymatically treating.
<5> The method according to <4>, further including adjusting pH of the enzymatically treated product to be alkaline, wherein the heat treatment is heat-treating an alkaline enzymatically treated product obtained in the adjusting.
<6> A yeast cell wall-derived decomposition product-containing composition including

a decomposition product derived from a cell wall of yeast,
wherein a volume of a precipitate is 50 mL or less, the precipitate forming when 100 mL of the yeast cell wall-derived decomposition product-containing composition having a solid content concentration of 10% by mass is left to stand still at 25°C under normal pressure for 48 hours.

<7> The yeast cell wall-derived decomposition product-containing composition according to <6>, wherein relative to a solid content mass of the yeast cell wall-derived decomposition product-containing composition, a content ratio of a total free amino acid is 7% by mass or more and a content ratio of a reducing sugar is 1% by mass or more.
<8> The yeast cell wall-derived decomposition product-containing composition according to <6> or <7>, wherein relative to a solid content mass of the yeast cell wall-derived decomposition product-containing composition, a content ratio of a total free amino acid is 12% by mass or more and a content ratio of a reducing sugar is 10% by mass or more.
<9> The yeast cell wall-derived decomposition product-containing composition according to any one of <6> to <8>, wherein the yeast cell wall-derived decomposition product-containing composition includes at least one compound selected from the group consisting of ethyl caproate, ethyl caprylate, ethyl caprate, phenylethyl alcohol, and capric acid.
<10> The yeast cell wall-derived decomposition product-containing composition according to <9>, wherein the decomposition product derived from the cell wall of yeast is a decomposition product derived from a cell wall of beer yeast.
<11> A yeast cell wall-derived decomposition product-containing composition including

a decomposition product derived from a cell wall of yeast,
wherein the yeast cell wall-derived decomposition product-containing composition includes at least one compound selected from the group consisting of pyrazine, methylpyrazine, 2,5-dimethylpyrazine, 2,6-dimethylpyrazine, 2,3-dimethylpyrazine, and furaneol.

<12> A beer-like fragrance-imparting composition including
the yeast cell wall-derived decomposition product-containing composition according to <9> or <10>.
<13> A roast flavor-imparting composition including
the yeast cell wall-derived decomposition product-containing composition according to <11>.
<14> A roast color-imparting composition including
the yeast cell wall-derived decomposition product-containing composition according to <11>.
<15> Food or drink including
the yeast cell wall-derived decomposition product-containing composition according to any one of <6> to <11>.
<16> A pet food palatability enhancer including
the yeast cell wall-derived decomposition product-containing composition according to any one of <6> to <11>.

Advantageous Effects of Invention

According to the present invention, it is possible to solve the above existing problems to achieve the above object, and provide a method for producing a yeast cell wall-derived decomposition product-containing composition where the method is capable of increasing the solubility ratio and dispersion stability of the yeast cell wall, needs no separation/removal operation of an insoluble fraction of the yeast cell wall, achieves high operation and production efficiency, and is performable at low cost.

According to the present invention, it is possible to solve the above existing problems to achieve the above object, and provide a cell wall-derived decomposition product-containing composition that has excellent solubility and dispersion stability.

According to the present invention, it is possible to solve the above existing problems to achieve the above object, and provide a yeast cell wall-derived decomposition product-containing composition that has excellent solubility and dispersion stability, is favorable in a roast flavor and a fat and oil feeling, and is favorable in a roast color.

According to the present invention, it is possible to solve the above existing problems to achieve the above object, and provide a beer-like fragrance-imparting composition that has excellent solubility and dispersion stability, has less off-flavors, and has a favorable beer-like fragrance.

According to the present invention, it is possible to solve the above existing problems to achieve the above object, and provide a roast flavor-imparting composition that has excellent solubility and dispersion stability and is favorable in a roast flavor and a fat and oil feeling.

According to the present invention, it is possible to solve the above existing problems to achieve the above object, and provide a roast color-imparting composition that has excellent solubility and dispersion stability and is favorable in a roast color.

According to the present invention, it is possible to solve the above existing problems to achieve the above object, and provide food or drink containing the yeast cell wall-derived decomposition product-containing composition.

According to the present invention, it is possible to solve the above existing problems to achieve the above object, and provide a pet food palatability enhancer containing the yeast cell wall-derived decomposition product-containing composition.

Brief Description of Drawings

FIG. 1 is chart presenting the results of solid-phase micro extraction (SPME)-gas chromatography mass spectrometry of the yeast cell wall (autolysis-type yeast cell wall) in Test Example 7-1;

FIG. 2 is a chart presenting the results of solid-phase micro extraction (SPME)-gas chromatography mass spectrometry of the autolysis-type yeast cell wall-derived decomposition product-containing composition (1E) (Preparation Example 3-1) in Test Example 7-1;

FIG. 3 is a chart presenting the results of solid-phase micro extraction (SPME)-gas chromatography mass spectrometry of the autolysis-type yeast cell wall-derived decomposition product-containing composition (1M) (Preparation Example 5-1) in Test Example 7-1;

FIG. 4 is a chart of an overlap for comparison of the charts of FIG. 1 to FIG. 3, where the dashed line indicates the chart of the yeast cell wall (autolysis-type yeast cell wall), the thick solid line indicates the chart of the autolysis-type yeast cell wall-derived decomposition product-containing composition (1E) (Preparation Example 3-1), and the thin solid line indicates the chart of the autolysis-type yeast cell wall-derived decomposition product-containing composition (1M) (Preparation Example 5-1);

FIG. 5 is an enlarged chart of FIG. 4;

FIG. 6 is an enlarged chart of the peaks of the pyrazines in FIG. 4;

FIG. 7 is an enlarged chart of the peak of the furaneol in FIG. 4;

FIG. 8 is a chart of an overlap for comparison of charts of solid-phase micro extraction (SPME)-gas chromatography mass spectrometry of an autolysis-type yeast cell wall-derived decomposition product-containing composition (1E)

(Preparation Example 3-1) and an autolysis-type yeast cell wall-derived decomposition product-containing composition (4E) (Preparation Example 3-4) in Test Example 7-2, where the dotted line indicates the chart of the autolysis-type yeast cell wall-derived decomposition product-containing composition (1E)

(Preparation Example 3-1) and the thick solid line indicates the chart of the autolysis-type yeast cell wall-derived decomposition product-containing composition (4E) (Preparation Example 3-4);

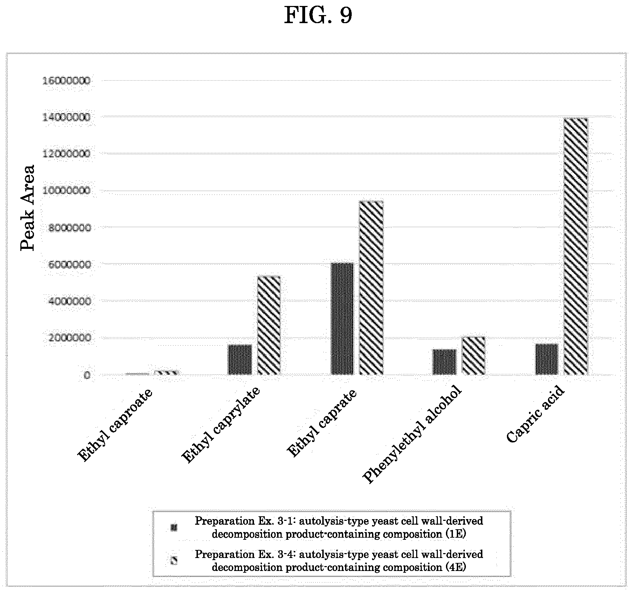

FIG. 9 is a graph of peak areas detected by solid-phase micro extraction (SPME)-gas chromatography mass spectrometry of an autolysis-type yeast cell wall-derived decomposition product-containing composition (1E) (Preparation Example 3-1) and an autolysis-type yeast cell wall-derived decomposition product-containing composition (4E) (Preparation Example 3-4) in Test Example 7-2, where the lefthand columns (black solid columns) indicate the peak areas of the respective ingredients of the autolysis-type yeast cell wall-derived decomposition product-containing composition (1E) (Preparation Example 3-1) and the right-hand columns (black shaded columns) indicate the peak areas of the respective ingredients of the autolysis-type yeast cell wall-derived decomposition product-containing composition (4E) (Preparation Example 3-4);

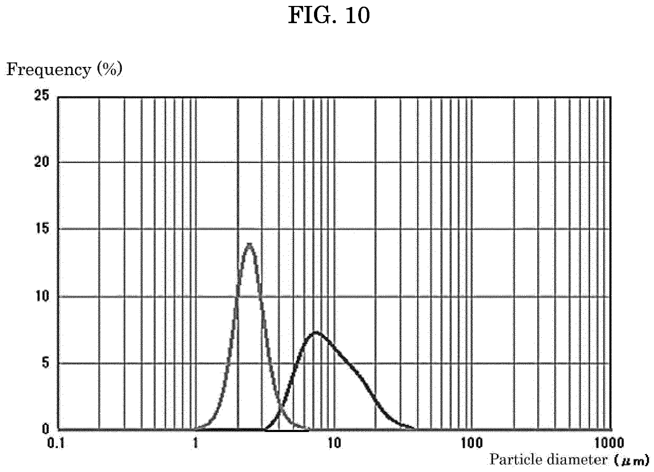

FIG. 10 is a chart presenting the results of the particle size distribution measurement of insoluble fractions of the autolysis-type yeast cell wall slurry (Preparation Example 1-1) in Test Example 8 and the autolysis-type yeast cell wall-derived decomposition product-containing composition (1E) (Preparation Example 3-1), where the black line indicates the results of the particle size distribution measurement of the insoluble fractions of the autolysis-type yeast cell wall slurry (Preparation Example 1-1) and the gray line indicates the results of the particle size distribution measurement of the autolysis-type yeast cell wall-derived decomposition product-containing composition (1E) (Preparation Example 3-1), and the vertical axis is frequency (%) and the horizontal axis is particle diameter ($\mu$m).

Description of Embodiments

(Yeast cell wall-derived decomposition product-containing composition, and a method for producing the composition)

A method of the present invention for producing a yeast cell wall-derived decomposition product-containing composition includes an enzymatically treating step, preferably further includes a pH adjusting step and a heat treating step, and if necessary further includes other steps.

In the present specification, the yeast cell wall-derived decomposition product-containing composition obtained in the enzymatically treating step may be referred to as "enzymatically treated product". Also, in the present specification, the yeast cell wall-derived decomposition product-containing composition obtained in the heat treating step may be referred to as "heat-treated product".

The yeast cell wall-derived decomposition product-containing composition of the present invention includes a decomposition product derived from a cell wall of yeast, wherein a volume of a precipitate is 50 mL or less, the precipitate forming when 100 mL of the yeast cell wall-derived decomposition product-containing composition having a solid content concentration of 10% by mass is left to stand still at 25°C under normal pressure for 48 hours.

The yeast cell wall-derived decomposition product-containing composition of the present invention can be suitably produced by the method of the present invention for producing a yeast cell wall-derived decomposition product-containing composition (enzymatically treated product).

The yeast cell wall-derived decomposition product-containing composition of the present invention includes a decomposition product derived from a cell wall of yeast and includes at least one compound selected from the group consisting of pyrazine, methylpyrazine, 2,5-dimethylpyrazine, 2,6-dimethylpyrazine, 2,3-dimethylpyrazine, and furaneol.

The yeast cell wall-derived decomposition product-containing composition of the present invention can be suitably produced by the method of the present invention for producing a yeast cell wall-derived decomposition product-containing composition (heat-treated product).

Hereinafter, the yeast cell wall-derived decomposition product-containing composition of the present invention will be described together with the method of the present invention for producing a yeast cell wall-derived decomposition product-containing composition.

<Enzymatically treating step>

The enzymatically treating step is a step of treating a cell wall of yeast (hereinafter may be abbreviated as "yeast cell wall") with exoglucanase.

The enzymatically treating step preferably further includes at least one of a protease treatment and an exopeptidase treatment of the yeast cell wall, more preferably includes both treatments of the protease treatment and the exopeptidase treatment.

By decomposing the yeast cell wall in the enzymatically treating step, it is possible to obtain a yeast cell wall-derived decomposition product-containing composition (enzymatically treated product) having excellent solubility.

Here, the solubility ratio in the present invention is calculated according to the following Formula (1).

$$\text{Solubility ratio (\%)} = \text{Solid content mass A/Solid content mass B} \times 100 \ldots$$

Formula (1)

In the Formula (1), the "Solid content mass B" denotes a mass of a dry product obtained by drying X g of a target sample at 105°C for 5 hours.

Also, in the Formula (1), the "Solid content mass A" denotes a mass of a dry product obtained by centrifuging X g of a target sample at 5,000 G for 5 minutes and drying the obtained supernatant at 105°C for 5 hours.

Examples of the target sample include the yeast cell wall, the enzymatically treated product, and the heat-treated product.

«Yeast cell wall»

In the present invention, the yeast cell wall is an insoluble fraction that is obtained as a heavy liquid after separation of a yeast cell extract, which is obtained by subjecting a yeast cell (which may be referred to as, for example, "yeast" or "yeast body") to a treatment for extracting a yeast extract therefrom. Incidentally, a soluble fraction obtained as a supernatant after the separation of the extract is a yeast extract. Therefore, the yeast cell wall in the present invention does not include the form of a yeast cell wall containing the yeast extract.

The kind of the yeast to be a raw material of the yeast cell wall is not particularly limited and may be appropriately selected depending on the intended purpose. Examples of the kind thereof include baker's yeast, beer yeast, wine yeast, *sake* yeast, soy sauce yeast, torula yeast, and yeast for bioethanol.

The genus of the yeast is not particularly limited and may be appropriately selected depending on the intended purpose. Examples of the genus include the genus Saccharomyces, the genus Candida, and the genus Kluvveromvces. Among them, the genus Saccharomyces is preferable.

Specific examples of the genus Saccharomyces include Saccharomyces cerevisiae.

The yeast cell wall used may be prepared from one kind of yeast. Alternatively, the yeast cell walls prepared from two or more kinds of yeast may be used in combination.

The yeast cell wall used may be a commercially available product. Alternatively, the yeast cell wall used may be prepared by a known method such as appropriate culturing for the purpose of use in the method for producing a yeast cell wall-derived decomposition product-containing composition. Furthermore, from the viewpoints of utilizing wastes and reducing cost for disposing of wastes, the yeast cell wall used may be a product generated as extra wastes in the brewing industry for beer, whisky, wine, distilled spirits, *sake*, *miso*, soy sauce, etc.

A method of the treatment to extract a yeast extract from the yeast cell for preparing the yeast cell wall is not particularly limited and may be appropriately selected from known extraction methods of a yeast extract. Examples of the method include an autolysis method, a hot-water extraction method, an enzymatic decomposition method, an alkali decomposition method, a freeze-thaw method, and a mechanical breakage method. One of these extraction methods may be performed, or two or more thereof may be performed in combination.

The autolysis method is specifically a method of making the yeast body soluble by use of, for example, proteases that are intrinsically present in the body.

The hot-water extraction method is specifically a method of making the yeast body soluble through immersion in hot water for a certain period of time. The temperature and immersion time in the hot-water extraction method are not particularly limited and may be appropriately selected depending on the intended purpose.

The enzymatic decomposition method is specifically a method of making the yeast body soluble by the addition of an enzyme preparation derived from microorganisms or plants. The enzyme preparation used in the enzymatic decomposition method is not particularly limited and may be appropriately selected from known enzyme preparations depending on the intended purpose.

The acid or alkali decomposition method is specifically a method of making the yeast body soluble by the addition of an acid or an alkali.

The freeze-thaw method is specifically a method of breaking the yeast body by at least one freeze-thaw process.

The mechanical breakage method is specifically a method of breaking the yeast body by mechanical stimuli. The mechanical stimuli used in the mechanical breakage method are not particularly limited and may be appropriately selected depending on the intended purpose. Examples thereof include ultrasonic waves, homogenizing under high pressure, grinding with a micro grinder, and milling by mixing with solids such as glass beads.

Among them, the yeast cell wall is preferably a yeast cell wall obtained by the autolysis method (hereinafter may be referred to as "autolysis-type yeast cell wall"), a yeast cell wall obtained by the hot-water extraction method (hereinafter may be referred to as "hot-water extraction-type yeast cell wall"), or a yeast cell wall obtained by the enzymatic decomposition method (hereinafter may be referred to as "enzymatic decomposition-type yeast cell wall").

A method for separating the yeast cell wall from the extract of the yeast cell is not particularly limited and may be appropriately selected depending on the intended purpose. Examples of the method include a method of centrifugation.

Conditions of the centrifugation are not particularly limited and may be appropriately selected depending on the intended purpose, but are preferably conditions at 4,700 G to 5,500 G for 5 minutes, more preferably conditions at 5,000 G for 5 minutes.

A form of the yeast cell wall used in the enzymatically treating step is not particularly limited and may be appropriately selected depending on the intended purpose. Examples of the form include a slurry (muddy) obtained by separation from the extract of the yeast cell, a suspension obtained by suspending the slurry in a solvent, a paste obtained by squeezing and concentrating the slurry, a dry product obtained by drying the paste followed by further concentrating, and powder obtained by pulverizing the dry product.

Among them, the form of the yeast cell wall is preferably a slurry (muddy) or a suspension obtained by suspending the slurry in a solvent because they do not require such treatments as squeezing and drying to result in increases in working efficiency and production efficiency. The suspension obtained by suspending the slurry in a solvent is more preferable because it becomes less viscous than the slurry to result in further increases in working efficiency and production efficiency.

The concentration of the slurry in the suspension is not particularly limited and may be appropriately selected depending on the viscosity of the slurry. In general, however, the slurry of the yeast cell wall has a very high viscosity, and thus in many cases, the concentration of the slurry in the suspension is at most about 15% by volume to about 16% by volume for the autolysis-type yeast cell wall and is at most about 15% by volume to about 20% by volume for the hot-water extraction-type yeast cell wall.

The content ratio of the protein in the yeast cell wall is not particularly limited and may be appropriately selected depending on, for example, the extraction method of the yeast extract for preparing the yeast cell wall.

The content ratio of the protein in the autolysis-type yeast cell wall is typically about 20% by mass to about 30% by mass.

The content ratio of the protein in the hot-water extraction-type yeast cell wall is typically about 45% by mass to about 55% by mass.

The content ratio of the protein in the enzymatic decomposition-type yeast cell wall may be varied depending on the kind of the enzyme used, but is often close to the content ratio of the protein in the autolysis-type yeast cell wall.

The content ratio of the protein in the yeast cell wall is a content ratio relative to a solid content mass of the yeast cell wall and can be measured by a combustion method (improved Dumas method).

The solid content mass of the yeast cell wall in the present invention refers to a mass of a dry product obtained by drying the yeast cell wall at 105°C for 5 hours.

The content ratio of the saccharide in the yeast cell wall is not particularly limited and may be appropriately selected depending on, for example, the extraction method of the yeast extract for preparing the yeast cell wall.

The content ratio of the saccharide in the autolysis-type yeast cell wall is typically 45% by mass or more in many cases, and the upper limit thereof is about 65% by mass.

The content ratio of the saccharide in the hot-water extraction-type yeast cell wall is typically 50% by mass or less, and the lower limit thereof is about 20% by mass.

The content ratio of the saccharide in the enzymatic decomposition-type yeast cell wall may be varied depending on the kind of the enzyme used, but is often close to the content ratio of the saccharide in the autolysis-type yeast cell wall.

The content ratio of the saccharide in the yeast cell wall is a content ratio relative to a solid content mass of the yeast cell wall and can be measured by the following Formula (2).

$$\text{Content ratio of saccharide in the yeast cell wall} = 100 - (A+B+C+D) \ldots \text{Formula (2)}$$

In the Formula (2), "A" denotes a content ratio of the protein in the yeast cell wall measured by the combustion method (improved Dumas method). "B" denotes a content ratio of the moisture in the yeast cell wall measured by the atmospheric heating drying method. "C" denotes a content ratio of the lipid in the yeast cell wall measured by the acid decomposition method. "D" denotes a content ratio of the ash in the yeast cell wall measured by the direct ashing method.

«Exoglucanase»

An exoglucanase is an exo-type enzyme that cleaves the end of a cellulose chain in the yeast cell wall. The exoglucanase is not particularly limited and may be appropriately selected. Preferably, it is an exoglucanase that is usable for food applications or is of grade usable for food applications.

The kind of the exoglucanase is not particularly limited and may be appropriately selected, but is preferably glucanase including at least one selected from the group consisting of exo-1,3-β-glucanase, exo-1,4-β-glucanase, and exo-1,6-β-glucanase.

A source of the exoglucanase is not particularly limited and may be appropriately selected depending on the intended purpose. Preferably, it is derived from the genus Talaromyces; the genus Rasamsonia such as Rasamsonia emersonii; Disporotrichum such as Disporotrichum dimorphosporum; the genus Streptomyces such as Streptomyces violaceoruber; or the genus Trichoderma such as Trichoderma longibrachiatum or Trichoderma ressei. More preferably, it is derived from Rasamsonia emersonii, Disporotrichum dimorphosporum, or the genus Talaromyces. Particularly preferably, it is derived from the genus Talaromyces. These may be used alone or in combination.

The exoglucanase used may be a commercially available product. Alternatively, it may be appropriately prepared for use by a known method from, for example, bacteria containing the exoglucanase.

Examples of the commercially available product of the exoglucanase include FILTRASE (registered trademark) BRX (product of DSM Japan, Ltd.), FILTRASE (registered trademark) BR-XL (product of DSM Japan, Ltd.), DENAZYME GEL-L1/R (product of Nagase ChemteX Corporation), and SUMIZYME TG (product of SHINNIHON CHEMICALS Corporation). FILTRASE (registered trademark) BRX (product of DSM Japan, Ltd.), FILTRASE (registered trademark) BR-XL (product of DSM Japan, Ltd.), and DENAZYME GEL-L1/R (product of Nagase ChemteX Corporation) are commercially available examples having a pure glucanase activity only, and SUMIZYME TG (product of SHINNIHON CHEMICALS Corporation) is a commercially available example having a composite activity including, for example, a protease activity as well.

In general, many of the commercially available product of the exoglucanase include endoglucanase as well. In the enzymatically treating step, a commercially available product including endoglucanase in addition to the exoglucanase may be used.

Endoglucanase is an endo-type enzyme that randomly cleaves an inside portion of the cellulose chain in the yeast cell wall.

The kind of endoglucanase is not particularly limited and may be appropriately selected. Examples of the kind include endo-1,3-β-glucanase, endo-1,4-β-glucanase, and endo-1,6-β-glucanase. These may be used alone or in combination.

A source of the endoglucanase is not particularly limited and is, for example, similar sources to those of the exoglucanase.

The lower limit of an amount of the exoglucanase added relative to the yeast cell wall is not particularly limited as long as it is not 0% by mass and may be appropriately selected depending on the intended purpose. It is preferably 0.05% by mass or more, more preferably 0.1% by mass or more, particularly preferably 0.5% by mass or more, relative to the solid content mass of the yeast cell wall.

The upper limit of an amount of the exoglucanase added relative to the yeast cell wall is not particularly limited and may be appropriately selected depending on the intended purpose. It is preferably 5% by mass or less, more preferably 2% by mass or less, particularly preferably 1% by mass or less, relative to the solid content mass of the yeast cell wall.

The lower limit and the upper limit of the amount of the exoglucanase relative to the yeast cell wall can appropriately be combined. The amount of the exoglucanase added relative to the yeast cell wall is preferably from 0.05% by mass to 5% by mass, more preferably from 0.1% by mass to 2% by mass, particularly preferably from 0.5% by mass to 1% by mass, relative to the solid content mass of the yeast cell wall.

When the yeast cell wall is treated with the exoglucanase, an enzymatically treated product of the decomposed yeast cell wall increases in solubility ratio, which is advantageous.

Also, when the yeast cell wall is treated with the exoglucanase, it is possible to produce a reducing sugar derived from the yeast cell wall in a large amount, which is advantageous. The reducing sugar derived from the yeast cell wall is able to function in, for example, the below-described heat treating step, preferably in the course of the Maillard reaction.

The reducing monosaccharide is not particularly limited and may be appropriately selected depending on the intended purpose. Examples of the reducing monosaccharide include ribose, xylose, glucose, mannose, fructose, galactose, arabinose, and rhamnose. Among them, glucose is a monosaccharide inherent in yeast that is producible from a sugar in the yeast cell wall. Glucose is therefore preferable because there is no need to add a sugar later to the enzymatically treated product. It is also advantageous that foods produced using such an enzymatically treated product can be displayed as a product with clean label and natural in the EU.

«Protease»

A protease acts on and hydrolyzes a peptide bond in a protein or a polypeptide chain in the yeast cell wall. The protease is not particularly limited and may be appropriately selected. Preferably, it is a protease that is usable for food applications or is of grade usable for food applications.

The kind of protease is not particularly limited and may be appropriately selected. It is preferably an endo-type protease that randomly cleaves an inside portion in the protein or the polypeptide chain in the yeast cell wall.

A source of the protease is not particularly limited and may be appropriately selected depending on the intended purpose. It is preferably derived from the genus Bacillus.

The protease may be a commercially available product. Alternatively, it may be appropriately prepared for use by a known method from, for example, bacteria containing a protease.

Examples of the commercially available product of the protease include Alcalase (registered trademark) 2.4 L FG (product of Novozymes Japan, Ltd.).

The lower limit of an amount of the protease added relative to the yeast cell wall is not particularly limited and may be appropriately selected depending on the intended purpose. It is preferably 0.05% by mass or more, more preferably 0.1% by mass or more, particularly preferably 0.2% by mass or more, relative to the solid content mass of the yeast cell wall.

The upper limit of an amount of the protease added relative to the yeast cell wall is not particularly limited and may be appropriately selected depending on the intended purpose. It is preferably 5% by mass or less, more preferably 2% by mass or less, particularly preferably 1% by mass or less, relative to the solid content mass of the yeast cell wall.

The lower limit and the upper limit of the amount of the protease added relative to the yeast cell wall may be appropriately combined. The amount of the protease added relative to the yeast cell wall is preferably from 0.05% by mass to 5% by mass, more preferably from 0.1% by mass to 2% by mass, particularly preferably from 0.2% by mass to 1% by mass, relative to the solid content mass of the yeast cell wall.

The timing at which the protease is added to the yeast cell wall is not particularly limited and may be appropriately selected depending on the intended purpose. The protease may be added so as to act in the same period as the exoglucanase. Alternatively, it may be added so as to act in a different period from the exoglucanase.

A method for adding the protease so as to act in the same period as the exoglucanase is not particularly limited and may be appropriately selected depending on the intended purpose. Examples of the method include adding the protease and the exoglucanase at the same time.

A method for adding the protease so as to act in a different period from the exoglucanase is not particularly limited and may be appropriately selected depending on the intended purpose. Preferably, allowing the exoglucanase to act is followed by allowing the protease to act. Examples of the method include adding the exoglucanase and then the protease.

When the yeast cell wall is treated with exoglucanase and the protease at the same time, the time efficiency increases, which is advantageous.

<<Exopeptidase>>

Exopeptidase acts on and hydrolyzes a peptide bond in the vicinity of the terminal of a protein or a polypeptide chain in the yeast cell wall. When the yeast cell wall is treated with exopeptidase, a large number of amino acids derived from the yeast cell wall can be produced.

Therefore, exopeptidase is preferably used when the method for producing a yeast cell wall-derived decomposition product-containing composition includes the below-described heat treating step.

The exopeptidase is not particularly limited and may be appropriately selected. Preferably, it is exopeptidase that is usable for food applications or is of grade usable for food applications.

A source of exopeptidase is not particularly limited and may be appropriately selected depending on the intended purpose. Preferably, it is derived from the genus <u>Aspergillus</u>.

The exopeptidase used may be a commercially available product. Alternatively, it may be appropriately prepared for use by a known method from, for example, bacteria containing exopeptidase.

Examples of the commercially available product of exopeptidase include Flavourzyme (registered trademark) 1000L (product of Novozymes Japan, Ltd.).

The lower limit of an amount of exopeptidase added relative to the yeast cell wall is not particularly limited and may be appropriately selected depending on the intended purpose. It is preferably 0.05% by mass or more, more preferably 0.1% by mass or more, particularly preferably 0.2% by mass or more, relative to the solid content mass of the yeast cell wall.

The upper limit of an amount of exopeptidase added relative to the yeast cell wall is not particularly limited and may be appropriately selected depending on the intended purpose. It is preferably 5% by mass or less, more preferably 2% by mass or less, particularly preferably 1% by mass or less.

The lower limit and the upper limit of the amount of exopeptidase added relative to the yeast cell wall may be appropriately combined. The amount of the exopeptidase added relative to the yeast cell wall is preferably from 0.05% by mass to 5% by mass, more preferably from 0.1% by mass to 2% by mass, particularly preferably from 0.2% by mass to 1% by mass, relative to the solid content mass of the yeast cell wall.

In the enzymatically treating step, the timing when exopeptidase is added to the yeast cell wall is not particularly limited. It is preferably added in the same period as the protease or after the protease.

Performing the exopeptidase treatment after the protease treatment is preferable because of increased production efficiency of amino acids derived from the yeast cell wall and of increased working efficiency.

Free or lower-molecular-weight, yeast cell wall-derived sugars and amino acids produced by treating the yeast cell wall with exopeptidase can be used as a source of nutrients for microorganisms or also can be used as an application of seasonings. Further, in the below-described heat treating step, they can preferably function in the course of the Maillard reaction. In order to cause the Maillard reaction industrially and efficiently, both the reducing sugars and the amino acids are preferably monomers. When the method for producing a yeast cell wall-derived decomposition product-containing composition includes the heat treating step, advantageously, the Maillard reaction can be caused, without externally adding any amino acids or reducing sugars such as glucose as auxiliary raw materials, only by the yeast cell wall-derived amino acids produced through enzymatic decomposition and the yeast cell wall-derived reducing sugars produced through enzymatic decomposition, and also it is possible to obtain a heat-treated product derived from the yeast cell wall having a favorable roast flavor and a roast color. Especially in the EU, the obtained products can be attached with clean label and natural, which is advantageous.

Nonetheless, in the method for producing a yeast cell wall-derived decomposition product-containing composition, the Maillard reaction may be caused by appropriately externally adding at least one of amino acids and reducing sugars depending on the intended purpose.

The amino acid is not particularly limited and may be appropriately selected depending on the intended purpose. Examples of the amino acid include free amino acids such as lysine, arginine, histidine, aspartic acid, glutamic acid, serine, threonine, aspartic acid, glutamine, alanine, glycine, valine, isoleucine, leucine, phenylalanine, tyrosine, tryptophan, methionine, cysteine, and proline; and some peptides and tripeptides such as glutathione.

The pH in the enzymatically treating step is not particular limited and may be appropriately selected depending on, for example, the optimum pH of each enzyme.

In the enzymatically treating step, the pH in the case of adding the exoglucanase and the protease so as to act in the same period is preferably from 4 to 9, more preferably from 5 to 7.

In the enzymatically treating step, the pH in the case of adding the exoglucanase, the protease, and the exopeptidase so as to act in the same period is also preferably from 4 to 9, more preferably from 5 to 7.

The temperature of the enzymatically treating step is not particularly limited and may be appropriately selected depending on, for example, the optimum temperature of each enzyme.

The temperature in the case of adding the exoglucanase and the protease so as to act in the same period in the enzymatically treating step is preferably from 30°C to 70°C, more preferably from 40°C to 60°C.

Also, the temperature in the case of adding the exoglucanase, the protease, and the exopeptidase so as to act in the same period in the enzymatically treating step preferably from 30°C to 70°C, more preferably from 40°C to 60°C.

The period of the enzymatically treating step is not particularly limited and may be appropriately selected depending on, for example, the extent of decomposition, the intended purpose, and the amount of each enzyme added.

In the enzymatically treating step, the period of the enzymatically treating step in the case of adding the exoglucanase and the protease so as to act in the same period is preferably from 1 hour to 40 hours, more preferably from 12 hours to 30 hours.

Also, in the enzymatically treating step, the period of the enzymatically treating step in the case of adding the exoglucanase, the protease, and the exopeptidase so as to act in the same period is preferably from 1 hour to 40 hours, more preferably from 12 hours to 30 hours.

The enzymatically treating step may be performed while being left to stand or under stirring. It is preferable to perform the enzymatically treating step under stirring because the decomposition efficiency of the yeast cell wall by each enzyme is high.

The speed of the stirring is not particularly limited and may be appropriately selected depending on the intended purpose as long as the yeast cell wall and each of the enzymes can be allowed to react.

-Yeast cell wall-derived decomposition product-containing composition (enzymatically treated product)-

The yeast cell wall-derived decomposition product-containing composition (enzymatically treated product) obtained by the enzymatically treating step has a much lower viscosity than that of the slurry of the yeast cell wall. The slurry of the yeast cell wall has a very high viscosity because of the abundance of glucan which is a thickening polysaccharide in the yeast cell wall. The yeast cell wall-derived decomposition product-containing composition (enzymatically treated product), however, has a lower viscosity because most of the glucan in the yeast cell wall is decomposed to disappear and be soluble or monomerized by the enzymatic treatment in the enzymatically treating step. The yeast cell wall-derived decomposition product-containing composition (enzymatically treated product), therefore, can be solubilized in a solvent or concentrated to result in remarkable increases in processing ability, fluidity, and handling ability, which is advantageous.

Furthermore, the yeast cell wall-derived decomposition product-containing composition (enzymatically treated product) has a remarkably increased dispersion stability as compared with the slurry of the yeast cell wall.

In the yeast cell wall-derived decomposition product-containing composition (enzymatically treated product), a volume of a precipitate is 50 mL or less, the precipitate forming when 100 mL of the yeast cell wall-derived decomposition product-containing composition having a solid content concentration of 10% by mass is left to stand still at 25°C under normal pressure for 48 hours. The volume of the precipitate is preferably 40 mL or less, more preferably 30 mL or less, further preferably 20 mL or less, particularly preferably 15 mL or less. When the volume of the precipitate of the yeast cell wall-derived decomposition product-containing composition (enzymatically treated product) is 50 mL or less, most of the yeast cell wall-derived decomposition product-containing composition (enzymatically treated product) is solubilized, and the insoluble fraction has a favorable dispersion stability.

The volume of the precipitate of the yeast cell wall-derived decomposition product-containing composition can be measured with, for example, a measuring cylinder.

In the present invention, the normal pressure refers to a pressure without being particularly reduced or increased. Usually, the normal pressure refers to a pressure equal to the atmospheric pressure (101.325 kPa).

Here, the solid mass concentration in the present invention is calculated according to the following Formula (3).

$$\text{Solid mass concentration (\%)} = \text{solid content mass/mass of the enzymatically treated product} \times 100 \ ... \ (3)$$

In the Formula (3), the "solid content mass" denotes a mass of a dry product obtained by drying X g of the enzymatically treated product at 105°C for 5 hours.

Also, in the Formula (3), the "mass of the enzymatically treated product" denotes a mass of X g of the enzymatically treated product.

The particle size distribution (d10) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 4 μm or less, more preferably 3 μm or less, particularly preferably 2 μm or less.

The particle size distribution (d50) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 7 μm or less, more preferably 5 μm or less, particularly preferably 3 μm or less.

The particle size distribution (d90) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 15 μm or less, more preferably 10 μm or less, further preferably 5 μm or less, particularly preferably 4 μm or less.

The mean volume particle diameter (MV) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 9 μm or less, more preferably 7 μm or less, further preferably 5 μm or less, particularly preferably 3 μm or less.

The mean number particle diameter (MN) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 5 μm or less, more preferably 4 μm or less, further preferably 3 μm or less, particularly preferably 2.5 μm or less.

The mean area particle diameter (MA) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 7 μm or less, more preferably 5 μm or less, further preferably 3 μm or less, particularly preferably 2.5 μm or less.

The standard deviation (SD) of the insoluble fraction of the yeast cell wall-derived decomposition product-containing composition (enzymatically treated product) is not particularly limited and may be appropriately selected depending on the intended purpose. From the viewpoints of solubility and dispersion stability, it is preferably 4 μm or less, more preferably 3 μm or less, further preferably 2 μm or less, particularly preferably 1 μm or less.

The standard deviation (SD) is indicative of the distribution width of a particle size distribution.

The particle size distribution (d10, d50, and d90), the mean volume particle diameter (MV), the mean number particle diameter (MN), the mean area particle diameter (MA), and the standard deviation (SD) of the yeast cell wall-derived decomposition product-containing composition (enzymatically treated product) in the present invention refer to corresponding values measured by MICROTRAC particle size distribution analyzer (MT3300EX, product of Nikkiso Co., Ltd.).

The yeast cell wall-derived decomposition product-containing composition (enzymatically treated product) preferably contains at least one compound selected from the group consisting of ethyl caproate (Hexanoic acid, ethyl ester), ethyl caprylate (Octanoic acid, ethyl ester), ethyl caprate (Decanoic acid, ethyl ester), phenylethyl alcohol (Phenylethyl Alcohol), and capric acid (n-Decanoic acid). These compounds are ingredients responsible for beer-like fragrance. These are suitably contained when the yeast cell wall-derived decomposition product-containing composition (enzymatically treated product) is a beer yeast cell wall-derived decomposition product-containing composition.

The compounds in the yeast cell wall-derived decomposition product-containing composition can be measured by solid-phase micro extraction (SPME)-gas chromatograph mass spectrometry under the measurement conditions described in the Examples (Test Examples 7-1 and 7-2).

The yeast cell wall-derived decomposition product-containing composition (enzymatically treated product) obtained by the enzymatically treating step is roughly divided into two forms depending on the presence or absence of the exopeptidase used in the enzymatically treating step.

A first form of the enzymatically treated product is an enzymatically treated product obtained in the enzymatically treating step by treating the yeast cell wall with the exoglucanase and if necessary, further with the protease (hereinafter this may be referred to as enzymatically treated product (1)).

Therefore, the enzymatically treated product (1) includes a yeast cell wall-derived decomposition product and the exoglucanase and if necessary, further includes the protease.

The enzymatically treated product (1) has remarkably increased solubility and dispersion stability as compared with the yeast cell wall. Also, the enzymatically treated product (1) further includes a large amount of a reducing monosaccharide (glucose) derived from the yeast cell wall.

The yeast cell wall-derived decomposition product in the enzymatically treated product (1) is a product obtained by decomposing the yeast cell wall with the exoglucanase and if necessary, further with the protease.

The content of the yeast cell wall-derived decomposition product in the enzymatically treated product (1) is not particularly limited and may be appropriately selected depending on, for example, the amount of the yeast cell wall used in the enzymatically treating step.

The content ratio of the yeast cell wall-derived glucose in the enzymatically treated product (1) is not particularly limited and may be appropriately selected depending on, for example, the conditions of the enzymatically treating step. It is preferably 1% by mass or more, more preferably 10% by mass or more, further preferably 20% by mass or more, relative to the solid content mass of the enzymatically treated product (1). When the content ratio of the glucose is 1% by mass or more, the solubility ratio becomes higher to make it easier to cause the Maillard reaction in the heat treating step. The upper limit of the yeast cell wall-derived glucose in the enzymatically treated product (1) is not particularly limited and may be appropriately selected depending on, for example, the content of glucan contained in the yeast cell wall as a raw material, and the decomposition extent by the enzyme. It is usually 35% by mass or less.

The solid content mass of the enzymatically treated product in the present invention refers to a mass of a dry product obtained by drying the enzymatically treated product at 105°C for 5 hours.

Also, in the present invention, the content ratio of the glucose in the enzymatically treated product is a value that was measured by an enzyme-catalyzed reaction and a hydrogen peroxide electrode detection method using a multifunctional biosensor (BF-7D, product of Oji Scientific Instruments). Specifically, in a multifunctional biosensor, it can be measured using a glucose electrode and a sucrose electrode, and a dedicated buffer solution at 30°C.

The content ratio of the yeast cell wall-derived total free amino acid in the enzymatically treated product (1) is not particularly limited but is often comparable to the content ratio of the amino acids in the yeast cell wall. Specifically, it is about 5% by mass to about 8% by mass relative to the solid content mass of the enzymatically treated product (1), and depending on the method of an enzyme treatment, it is, for example, 5% by mass to 13% by mass.

In the present invention, the content ratio of the total free amino acid in the enzymatically treated product can be measured under the measurement conditions described in the Examples (Test Example 5) by derivatizing the amino acids in the enzymatically treated product according to the product protocol of AccQ-Tag Ultra Derivatization kit (product of Waters, Co.) followed by ultra high performance liquid chromatography.

The content of the exoglucanase and the content of the protease in the enzymatically treated product (1) are not particularly limited and may be appropriately selected depending on, for example, the amount of the exoglucanase and the amount of the protease used in the enzymatically treating step.

A second form of the enzymatically treated product is an enzymatically treated product obtained in the enzymatically treating step by treating the yeast cell wall with the exoglucanase and the exopeptidase, and if necessary, further with the protease (hereinafter this may be referred to as enzymatically treated product (2)). Therefore, the enzymatically treated product (2) includes a yeast cell wall-derived decomposition product, the exoglucanase, and the exopeptidase, and if necessary, further includes the protease.

Similar to the enzymatically treated product (1), the enzymatically treated product (2) has remarkably increased solubility ratio and dispersion stability as compared with the yeast cell wall. Also, the enzymatically treated product (2) further includes large amounts of the yeast cell wall-derived total free amino acid and a reducing monosaccharide derived from the yeast cell wall. Therefore, the enzymatically treated product (2) can be suitably used as a heat treating target (raw material) in the heat treating step.

The yeast cell wall-derived decomposition product in the enzymatically treated product (2) is a product obtained by decomposing the yeast cell wall with the exoglucanase and the exopeptidase, and if necessary, further with the protease.

The content of the yeast cell wall-derived decomposition product in the enzymatically treated product (2) is not particularly limited and may be appropriately selected depending on, for example, the amount of the yeast cell wall used in the enzymatically treating step.

The content of the yeast cell wall-derived glucose in the enzymatically treated product (2) is not particularly limited and may be appropriately selected depending on, for example, the conditions of the enzymatically treating step. It is preferably 1% by mass or more, more preferably 10% by mass or more, further preferably 20% by mass or more, relative to the solid content mass of the enzymatically treated product (2). When the content ratio of the glucose is 1% by mass or more, the solubility ratio becomes higher to make it easier to cause the Maillard reaction in the heat treating step. The upper limit of the yeast cell wall-derived glucose in the enzymatically treated product (2) is not particularly limited and may be appropriately selected depending on, for example, the content of glucan contained in the yeast cell wall as a raw material, and the decomposition extent by the enzyme. It is usually 35% by mass or less.

The content ratio of the yeast cell wall-derived total free amino acid in the enzymatically treated product (2) is not particularly limited and may be appropriately selected depending on, for example, the conditions of the enzymatically treating step. The solid content mass of the enzymatically treated product (2) is preferably 7% by mass or more, more preferably 10% by mass or more, further preferably 12% by mass or more. When the content ratio of the total free amino acids is 7% by mass or more, the Maillard reaction can be efficiently caused in the heat treating step. The upper limit of the content ratio of the total free amino acids derived from yeast cell wall in the enzymatically treated product (2) is not particularly limited and may be appropriately selected depending on, for example, the content ratio of a protein contained in the yeast cell wall as a raw material. It is usually 35% by mass or less. Usually, the content ratio of a protein contained in the yeast cell wall as a raw material is 60% by mass or less. Because the amino acid decomposition efficiency is not as high as the sugars, the content ratio of the total free amino acids derived from the yeast cell wall in the enzymatically treated product (2) is at most about 30% by mass.

Also, when the content ratio of the total free amino acid is 12% by mass or more relative to the solid content mass of the enzymatically treated product and the content ratio of the reducing sugar is 10% by mass or more, the enzymatically treated product can find more applications as a raw material, which is preferable.

The content of the exoglucanase, the content of the protease, and the content of the exopeptidase in the enzymatically treated product (2) are not particularly limited and may be appropriately selected depending on, for example, the amount of the exoglucanase, the amount of the protease, and the amount of the exopeptidase used in the enzymatically treating step.

The solubility ratios of the enzymatically treated product (1) and the enzymatically treated product (2) are not particularly limited and may be appropriately selected depending on the intended purpose. The solubility ratios calculated according to the above Formula (1) are preferably 60% or more, more preferably 80% or more. The solubility ratios of the yeast cell wall calculated according to the above Formula (1) are typically from about 18% to about 27% or are equal to or less than about 18%. Thus, the enzymatically treated product (1) and the enzymatically treated product (2) have very increased solubility ratios as compared with the yeast cell wall, which is advantageous.

The enzymatically treated product (1) and the enzymatically treated product (2) each may further contain other ingredients, if necessary.

The other ingredients are not particularly limited and may be appropriately selected depending on the intended purpose. Examples of the other ingredients include solvents, pH adjusters, fungicides such as salts, sugars, reducing monosaccharides, thiamine, ascorbic acid (vitamins), amino acids, peptides, acids, alkalis, emulsifiers, other food additives, and sulfur-containing compounds. These may be used alone or in combination.

The content of the other ingredients in the enzymatically treated product is not particularly limited and may be appropriately selected depending on the intended purpose.

A form of the enzymatically treated product is not particularly limited and may be appropriately selected depending on the intended purpose. Examples of the form include a liquid obtained in the enzymatically treating step, a liquid suspension obtained by suspending the liquid enzymatically treated product in a solvent, a paste obtained by squeezing and concentrating the liquid enzymatically treated product, a dry product obtained by drying the paste followed by further concentrating, and powder obtained by pulverizing the dry product.

Applications of the enzymatically treated product are not particularly limited and may be appropriately selected depending on the intended purpose. The enzymatically treated product has high solubility ratio and dispersion stability. Thus, similar to the applications of the typical yeast extract, the enzymatically treated product can be widely used in various fields such as the foods field, the bio field, and the cosmetics field. Specifically, it can be suitably used in, for example, foods and drinks, alcohols, raw materials for pet food (especially a pet food palatability enhancer), and media.

<pH adjusting step>

When the method for producing a yeast cell wall-derived decomposition product-containing composition includes the below-described heat treating step, the method for producing a yeast cell wall-derived decomposition product-containing composition preferably includes a pH adjusting step.

The pH adjusting step is a step of adjusting the pH of the enzymatically treated product obtained in the enzymatically treating step to a pH in conformity to the intended purpose.

In the pH adjusting step, the enzymatically treated product to be adjusted for pH is the enzymatically treated product (2) obtained by treating the yeast cell wall with the exoglucanase and the exopeptidase, and if necessary, further with the protease.

The timing at which the pH adjusting step is performed is not particularly limited and may be appropriately selected depending on the intended purpose as long as it is after the enzymatically treating step or before the heat treating step.

Performing the pH adjusting step before the heat treating step is advantageous because the solubility ratio and the dispersion stability of the yeast cell wall are further increased.

Also, performing the pH adjusting step before the heat treating step is advantageous because of further increase in consumption of the total free amino acid (the Maillard reaction) in the subsequent heat treating step. This makes it possible to obtain a yeast cell wall-derived decomposition product-containing composition that is further favorable in a roast flavor, a fat and oil feeling, and a roast color.

A method for adjusting the pH of the enzymatically treated product is not particularly limited and may be appropriately selected from known method depending on the intended purpose. Examples of the method include a method of adding a pH adjuster to the enzymatically treated product.

The pH adjuster is not particularly limited and may be appropriately selected depending on the intended purpose. Examples of the pH adjuster include alkalis such as sodium hydroxide, potassium hydroxide, sodium citrate, sodium acetate, and sodium phosphate. These may be used alone or in combination.

The pH of the enzymatically treated product in the pH adjusting step is preferably 8 or higher but 10 or lower. When the pH thereof is in such a range, the efficiency of the Maillard reaction increases in the subsequent heat treating step for obtaining a roast flavor and coloring, which is preferable.

<Heat treating step>

In order to obtain a yeast cell wall-derived decomposition product-containing composition that is favorable in a roast flavor and a fat and oil feeling and is favorable in a roast color, the method for producing a yeast cell wall-derived decomposition product-containing composition preferably includes the heat treating step.

The heat treating step is a step of heat-treating the enzymatically treated product obtained in the enzymatically treating step. When the method for producing a yeast cell wall-derived decomposition product-containing composition includes the pH adjusting step, the heat treating step is a step of heat-treating an alkaline enzymatically treated product obtained in the pH adjusting step.

A heat-treating target in the heat treating step is the enzymatically treated product (2) obtained by treating the yeast cell wall with the exoglucanase and the exopeptidase, and if necessary, further with the protease.

In the heat treating step, the Maillard reaction is caused by the yeast cell wall-derived total free amino acid produced through enzymatic decomposition and the yeast cell wall-derived reducing monosaccharide produced through enzymatic decomposition in the enzymatically treated product (2). As a result, it is possible to obtain a yeast cell wall-derived decomposition product-containing composition (heat-treated product) that is favorable in a roast flavor and a roast color.

The exoglucanase, the protease, and the exopeptidase used in the enzymatically treating step are deactivated in the heat treating step. Therefore, the enzymatically treated product (1) may be used as a heat-treating target in the heat treating step in order to deactivate these enzymes. Also, the heat-treating target in the heat treating step may be a product that has undergone the below-described enzyme deactivating step.

In order to cause the Maillard reaction in the heat treating step, both the reducing sugars and the amino acids should be monomers. In general, when the amount of amino acids is larger than the amount of reducing monosaccharides in the Maillard reaction, it is easier to obtain a roast flavor and a roast color intensity, which is advantageous. The case where the amount of reducing monosaccharides is larger than the amount of amino acids is advantageous in terms of a meat flavor and tastes.

In the heat treating step, preferable contents of the yeast cell wall-derived total free amino acid and the yeast cell wall-derived reducing monosaccharide (glucose) in the enzymatically treated product (2) are as described in the above section "-Yeast cell wall-derived decomposition product-containing composition (enzymatically treated product)-".

The pH in the heat treating step is not particularly limited and may be appropriately selected depending on the intended purpose. It is preferably a pH at which the Maillard reaction can occur.

The pH at which the Maillard reaction can occur is preferably from 4 to 12, more preferably from 8 to 10. When the pH in the heat treating step is 4 or higher, the rate of the Maillard reaction becomes faster, whereas when the pH is 12 or lower, off-flavor does not easily occur to make it possible to obtain a heat-treated product favorable in a roast flavor and a roast color. Meanwhile, when the pH in the heat treating step is lower than 4, the rate of the Maillard reaction may become slower, whereas when the pH is higher than 12, off-flavor may occur to make it impossible to obtain a heat-treated product favorable in a roast flavor and a roast color. In the EU, a pH of 8 or lower is preferably in terms of being displayable as a product with clean label and natural and in terms of the other regulations in the EU.

The heating temperature in the heat treating step is not particularly limited and may be appropriately selected depending on the intended purpose. It is preferably a heating temperature at which the Maillard reaction can occur.

The heating temperature at which the Maillard reaction can occur industrially efficiently in a short time is preferably from 60°C to 180°C, more preferably from 90°C to 120°C. When the heating temperature in the heat treating step is 60°C or higher, the rate of the Maillard reaction becomes faster, whereas when the heating temperature is 180°C or lower, side reactions do not easily occur to make it possible to obtain a heat-treated product favorable in a roast flavor and a roast color. Meanwhile, when the heating temperature in the heat treating step is lower than 60°C, the rate of the Maillard reaction may become slower, whereas when the heating temperature is higher than 180°C, other reactions and decompositions may occur to make it impossible to obtain a heat-treated product favorable in a roast flavor and a roast color. When the heating temperature in the heat treating step is in the more preferable range, a clean label can be attached in the EU, which is advantageous. Also, from the viewpoint of the other regulations in the EU, it is preferably 180°C or lower.

The heating period in the heat treating step is not particularly limited and may be appropriately selected depending on, for example, the heating temperature in the heat treating step. It is preferably a heating period that can efficiently cause the Maillard reaction in a short time.

The period that can cause the Maillard reaction is preferably from 1 minute to 360 minutes, more preferably from 30 minutes to 180 minutes. When the heating period in the heat treating step is shorter than 1 minute, it may be impossible to cause the Maillard reaction sufficiently. When it is longer than 360 minutes, off-flavor may occur and also such a long period may be disadvantageous in terms of production efficiency.

The heat treating step may be performed while being left to stand or under stirring. It is preferable to perform the heat treating step under stirring because the ingredients in the yeast cell wall-derived decomposition product-containing composition (heat-treated product) obtained by the heating treatment become stable and uniform.

The speed of the stirring is not particularly limited and may be appropriately selected depending on the intended purpose.

The heat treating step may be performed using an apparatus. The apparatus is not particularly limited and may be appropriately selected from known apparatuses as long as the yeast cell wall can be treated. Examples of the apparatus include typical food manufacturing machines such as a kneader capable of stirring at normal pressure and a stirring device capable of increasing and reducing the pressure.

Specific examples of the apparatus usable in the heat treating step include an autoclave, a cooking mixer (product of KAJIWARA INC.), and Flex-Mix Processor (product of SPX FLOW Technology).

Also, a tank used in the enzymatically treating step may be used in the heat treating step as is.

-Yeast cell wall-derived decomposition product-containing composition (heat-treated product)-

The yeast cell wall-derived decomposition product-containing composition (heat-treated product) obtained by the heat treating step includes a yeast cell wall-derived decomposition product. The yeast cell wall-derived decomposition product in the heat-treated product is a product obtained by decomposing the yeast cell wall by the heat treatment. The heat-treated product has an increased solubility ratio and is favorable in a roast flavor and a fat and oil feeling, and is favorable in a roast color, as compared with the yeast cell wall.

The yeast cell wall-derived decomposition product-containing composition (heat-treated product) includes at least one compound selected from the group consisting of pyrazine, methylpyrazine, 2,5-dimethylpyrazine, 2,6-dimethylpyrazine, 2,3-dimethylpyrazine, and furaneol. The roast flavor of the heat-treated product is attributed to these compounds.

The compounds in the heat-treated product can be measured by solid-phase micro extraction (SPME)-gas chromatograph mass spectrometry under the measurement conditions described in the Examples (Test Examples 7-1 and 7-2).

The heat-treated product obtained using as a raw material the yeast cell wall including a high content of a sugar such as the autolysis-type yeast cell wall advantageously has a further increased solubility ratio. In addition, a higher extent of the Maillard reaction provides a more favorable roast flavor and a roast color having a high extent of browning.

The heat-treated product obtained using as a raw material the yeast cell wall including a high content of a protein such as the hot-water extraction-type yeast cell wall advantageously has a further increased solubility ratio similar to the heat-treated product obtained using as a raw material the yeast cell wall including a high content of a sugar. The heat-treated product obtained using as a raw material the yeast cell wall including a high content of a protein contains a large amount of peptides and amino acids derived from the yeast cell wall. Thus, as compared with the heat-treated product obtained using as a raw material the yeast cell wall including a high content of a sugar, advantageously, it is weakly roasted due to the low ratio of the reducing sugar to thereby provide a meat flavor-like roast flavor.

As described above, the flavor and the roast color of the heat-treated product can be appropriately adjusted by adjusting the formulation of ingredients in the yeast cell wall as a raw material, preferably the yeast cell wall-derived total free amino acid and the content ratios of the yeast cell wall-derived reducing sugars (mass ratios) in the enzymatically treated product (2), the conditions of the Maillard reaction in the heat treating step, etc. Therefore, the method for producing a yeast cell wall-derived decomposition product-containing composition can advantageously produce appropriate heat-treated products that are different depending on the intended purpose.

The roast flavor of the heat-treated product includes at least one of a roast flavor, a meat flavor, and a fat and oil feeling.

In the present invention, the "roast flavor" refers to a roast taste (roasting taste), a roast fragrance and a nuts fragrance (mainly a fragrance attributed to pyrazines), a cereal fragrance, a coffee fragrance, and a bitter taste.

In the present invention, the "fat and oil feeling" refers to one accompanying a flavor that imparts the so-called fat feeling such as fat and oil and a phospholipid.

More specifically, the roast flavor of the heat-treated product has flavors derived from plants such as cacao mass, cocoa butter, cocoa powder, chocolate, peanut, roast peanut, and coffee; flavors derived from birds such as chicken, turkey meat, pheasant meat, goose meat, swan meat, and duck meat; flavors derived from mammals such as beef, pork, lamb meat, mutton, goat meat, and horse meat; and associated fragrances thereof or cooking fragrances thereof.

The solubility ratio of the heat-treated product is not particularly limited and may be appropriately selected depending on the intended purpose. The solubility ratio calculated according to the above Formula (1) is preferably 80% or higher, more preferably 85% or higher. The solubility ratio of the yeast cell wall calculated according to the above Formula (1), as described above, is typically from about 18% to about 27%, or is equal to or lower than about 18%. As compared with the yeast cell wall, therefore, the heat-treated product has a remarkably increased solubility ratio, which is advantageous. Also, the heat-treated product has an increased solubility ratio as compared with the enzymatically treated product, which is further advantageous.

The content ratio of the yeast cell wall-derived decomposition product in the heat-treated product is not particularly limited and may be appropriately selected depending on, for example, the amount of the enzymatically treated product (2) used in the heat-treated product.

In the heat treating step, the yeast cell wall-derived total free amino acid in the enzymatically treated product (2) is consumed by the Maillard reaction. As a result, the content of the yeast cell wall-derived total free amino acid in the heat-treated product is lower than the content of the yeast cell wall-derived total free amino acid in the enzymatically treated product (2).

When the content of the yeast cell wall-derived total free amino acid in the heat-treated product is almost equal to the content of the yeast cell wall-derived total free amino acid in the yeast cell wall, it is meant that almost all or all the yeast cell wall-derived total free amino acid produced in the enzymatically treating step has been consumed in the heat treating step.

It is not necessarily preferable that all the yeast cell wall-derived total free amino acid in the heat-treated product is consumed by the Maillard reaction. Whether to consume all may be appropriately selected depending on the intended purpose. For example, for the purposes of, for example, use as seasonings and impartment of tastes, the heat treatment may be performed so as to leave the total free amino acid.

A method for performing the heat treatment so as to leave the total free amino acid is not particularly limited and may be appropriately selected depending on the intended purpose. The method is made possible by appropriately adjusting various conditions such as the pH, the heating temperature, and the heating period in the heat treating step.

In the heat treating step, the yeast cell wall-derived reducing monosaccharide in the enzymatically treated product (2) is consumed by the Maillard reaction together with the total free amino acid. As a result, the content of the yeast cell wall-derived reducing monosaccharide in the heat-treated product is lower than the content of the yeast cell wall-derived reducing monosaccharide in the enzymatically treated product (2).

When the content of the yeast cell wall-derived reducing monosaccharide in the heat-treated product is almost equal to the content of the yeast cell wall-derived reducing monosaccharide in the yeast cell wall, it is meant that almost all or all the yeast cell wall-derived reducing monosaccharide produced in the enzymatically treating step has been consumed in the heat treating step to thereby provide various flavors and coloring.

It is not necessarily preferable that all the yeast cell wall-derived reducing monosaccharide in the heat-treated product is consumed by the Maillard reaction. Whether to consume all may be appropriately selected depending on the intended purpose. For example, for the purpose of, for example, use of a source of nutrients in media and microorganisms, the heat treatment may be performed so as to leave the reducing monosaccharide.

A method for performing the heat treatment so as to leave the reducing monosaccharide is not particularly limited and may be appropriately selected depending on the intended purpose. The method is made possible by appropriately adjusting various conditions such as the pH, the heating temperature, and the heating period in the heat treating step.

The color of the enzymatically treated product (2) is turned into brown (browning) in the heat treating step. Thus, the color can be used as an indicator for evaluating the extent of roast of the heat-treated product.

A method for evaluating the color of the heat-treated product is not particularly limited and may be appropriately selected from known methods that can evaluate the extent of browning. Examples of the method include a method of evaluating in the L*a*b* color system and a method of evaluating an optical density at 470 nm (OD470).

The method of evaluating in the L*a*b* color system is specifically according to JIS Z-8722, and uses a color difference meter to make measurement under the measurement conditions described in the Examples (Test Example 2), to be able to determine the L* value, the a* value, and the b* value of the heat-treated product.

The L*a*b* color system is an indicator used for representing the color of an object and is standardized by the International Commission on Illumination (CIE) in 1976. In the L*a*b* color system, lightness is indicated by the L* value and chromaticity (hue and saturation) is indicated by the a* value and the b* value. The greater L* value means being lighter. The a* value and the b* value indicate directions of a color. The a* and the -a* indicate the red direction and the green direction, respectively, and the b* and the -b* indicate the yellow direction and the blue direction, respectively.

In the present invention, the L* value is a value for evaluating the extent of roast of the heat-treated product based on the color (lightness). The L* value of 0 is black and the L* value of 100 is white.

The heat-treated product may further include other ingredients, if necessary.

The other ingredients are not particularly limited and may be appropriately selected depending on the intended purpose. Examples of the other ingredients include solvents, pH adjusters, fungicides such as salts, glycerin, propylene glycol, and dextrin. These may be used alone or in combination.

The content of the other ingredients in the heat-treated product is not particularly limited and may be appropriately selected depending on the intended purpose.

A form of the heat-treated product is not particularly limited and may be appropriately selected depending on the intended purpose. Examples of the form include a liquid obtained in the heat treating step, a liquid suspension obtained by suspending the liquid heat-treated product in a solvent, a paste obtained by squeezing and concentrating the liquid heat-treated product, a dry product obtained by drying the paste followed by further concentrating, and powder obtained by pulverizing the dry product.

Applications of the heat-treated product are not particularly limited and may be appropriately selected depending on the intended purpose. The heat-treated product is excellent in solubility and dispersion stability and is favorable in a roast flavor and a fat and oil feeling, and is favorable in a roast color, and thus can be used for at least one selected from the group consisting of roast flavor enhancement, roast flavor impartment, roast color enhancement, roast color impartment, fat and oil feeling enhancement, and fat and oil feeling impartment in food or drink, pet food, and a pet food palatability enhancer.

Also, the heat-treated product can be used as additives or raw materials thereof such as perfumes and perfume raw materials for at least one selected from the group consisting of roast flavor enhancement, roast flavor impartment, roast color enhancement, roast color impartment, fat and oil feeling enhancement, and fat and oil feeling impartment.

<Other steps>

The other steps are not particularly limited and may be appropriately selected depending on the intended purpose. Examples of the other steps include a sterilizing step, a neutralizing step, an enzyme-deactivating step, a cooling step, a centrifuging or filtrating step, a concentrating or drying step, a pulverizing step, and an emulsifying step.

-Sterilizing step-

The sterilizing step is a step of sterilizing the yeast cell wall as a raw material before the enzymatically treating step.

A method for sterilizing the yeast cell wall in the sterilizing step is not particularly limited and may be appropriately selected from known methods. Examples of the method include a method of heating under pressurized conditions and the ultrahigh temperature (UHT) method.

The pressure, temperature, and period in the method of heating under pressurized conditions are not particularly limited and may be appropriately selected depending on the intended purpose as long as the yeast cell wall can be sterilized.

-Neutralizing step-

The neutralizing step is a step of neutralizing the enzymatically treated product obtained in the enzymatically treating step or heat-treated product obtained in the heat treating step.

Depending on the applications of the enzymatically treated product and the heat-treated product, they may be neutralized in the neutralizing step.

A method of the neutralizing is not particularly limited and may be appropriately selected from known methods. Examples of the method include a method of appropriately adding an alkali such as sodium hydroxide, potassium hydroxide, sodium citrate, sodium acetate, or sodium phosphate or an acid such as hydrochloric acid, sulfuric acid, citric acid, or formic acid, to the enzymatically treated product or the heat-treated product. The pH of the enzymatically treated product or the heat-treated product after the neutralization is preferably from 4 to 8, more preferably from 5 to 7.

-Enzyme-deactivating step-

The enzyme-deactivating step is a step of deactivating the enzymes in the enzymatically treated product obtained in the enzymatically treating step.

As described for the heat treating step, the enzymes used in the enzymatically treating step and remaining in the enzymatically treated product are deactivated in the heat treating step. In the method for producing a yeast cell wall-derived decomposition product-containing composition, however, it is not necessarily perform the enzymatically treating step and the heat treating step successively. In such a case, the enzyme-deactivating step may be performed after the enzymatically treating step to deactivate the enzymes in the enzymatically treated product.

A method for deactivating the enzymes in the enzymatically treated product is not particularly limited and may be appropriately selected depending on the intended purpose. Examples of the method include a method of adjusting the pH of the enzymatically treated product to a pH at which the enzymes are deactivated.

The timing at which the enzyme-deactivating step is performed is not particularly limited and may be appropriately selected depending on the intended purpose as long as it is after the enzymatically treating step. From the viewpoint of preventing over-progression of the enzymatic reaction in order to obtain a desired enzymatically treated product, it is preferable to perform the enzyme-deactivating step just after the enzymatically treating step.

-Cooling step-

The cooling step is a step of cooling the heat-treated product obtained in the heat treating step to ambient temperature (15°C to 25°C) or a temperature that is equal to or lower than the ambient temperature.

A method of the cooling is not particularly limited and may be appropriately selected from known methods. Examples of the method include air cooling and water cooling.

-Centrifuging or filtrating step-

The centrifuging or filtrating step is a step of centrifuging or filtrating the enzymatically treated product obtained in the enzymatically treating step or the heat-treated product in the heat treating step, to remove impurities.

The enzymatically treated product or the heat-treated product are much superior to conventional products in terms of solubility and dispersion stability. When the enzymatically treated product and the heat-treated product are required to be clear in their usage forms (e.g., foods and drinks such as beer, beer-taste drinks, non-alcohol drinks, RTDs (Ready to drinks: low-alcohol drinks, for example), spirits, and whisky), impurities are removed in the centrifuging or filtrating step to allow the enzymatically treated product or the heat-treated product to be clearer.

-Concentrating or drying step-

The concentrating or drying step is a step of concentrating or drying the enzymatically treated product obtained in the enzymatically treating step or the heat-treated product in the heat treating step.

A method of the concentrating or drying is not particularly limited and may be appropriately selected from known methods. Examples of the method include a method of drying by the spray dry method, the air dry method, or the use of a dry drum, the freeze-dry method, and the vacuum dry method.

The concentration ratio of the enzymatically treated product or the heat-treated product is not particularly limited and may be appropriately selected depending on the intended purpose. The enzymatically treated product or the heat-treated product has an increased solubility ratio as compared with the yeast cell wall. Thus, even if the enzymatically treated product or the heat-treated product is concentrated to a high concentration of from about 40% by mass to about 50% by mass, the enzymatically treated product or the heat-treated product can be formed into a liquid or paste having favorable fluidity, which is advantageous in terms of increases in working efficiency and production efficiency.

-Pulverizing step-

The pulverizing step is a step of pulverizing the enzymatically treated product obtained in the enzymatically treating step or the heat-treated product obtained in the heat treating step, to form powder. The pulverizing step is preferably subjected to the concentrated or dry product of the enzymatically treated product or the concentrated or dry product of the heat-treated product obtained in the concentrating or drying step.

A method of the pulverizing is not particularly limited and may be appropriately selected from known methods. Examples of the method include slicing, cutting, and drilling. These may be used alone or in combination.

A unit used for the pulverizing is not particularly limited and may be appropriately selected from known pulverizers. Examples of the unit include food processors.

-Emulsifying step-

The emulsifying step is a step of emulsifying the enzymatically treated product obtained in the enzymatically treating step or the heat-treated product obtained in the heat treating step.

A raw material used for emulsifying the enzymatically treated product or the heat-treated product is not particularly limited and may be appropriately selected from common fat and oil raw materials. Examples of the raw material include plant fats and oils such as sunflower oil, soybean oil, and rapeseed oil; and animal fats and oils such as pig fat, lard, beef tallow, and chicken oil.

An amount of the fat and oil raw material added relative to the enzymatically treated product or the heat-treated product is not particularly limited and may be appropriately selected depending on the intended purpose.

A method for emulsifying the enzymatically treated product or the heat-treated product is not particularly limited and may be appropriately selected from known emulsifying methods. Examples of the method include a method of vigorously stirring with a common mixer and a method of using a homogenizer or high-pressure emulsifier.

(Beer-like fragrance-imparting composition)

A beer-like fragrance-imparting composition of the present invention includes the yeast cell wall-derived decomposition product-containing composition (enzymatically treated product) and if necessary, further includes other ingredients.

<Yeast cell wall-derived decomposition product-containing composition>

The yeast cell wall-derived decomposition product-containing composition is the yeast cell wall-derived decomposition product-containing composition (enzymatically treated product) of the present invention. It is preferably a beer yeast cell wall-derived decomposition product-containing composition.

The present inventor has unexpectedly found that the beer yeast cell wall-derived decomposition product-containing composition has a strong beer-like fragrance although having the low extent of off-flavor. This beer-like fragrance is believed to be attributed to at least one compound selected from the group consisting of ethyl caproate, ethyl caprylate, ethyl caprate, phenylethyl alcohol, and capric acid in the yeast cell wall-derived decomposition product-containing composition.

In the present invention, the "beer-like fragrance" refers to a similar fragrance to a fragrance (hereinafter may be referred to as a brewing fragrance) derived from an alcohol drink obtained by using beer yeast to ferment a substance containing malt, hop, and water as main raw materials and optionally containing other materials such as rice, corn, and starch as auxiliary raw materials in a certain range (preferably at a mass equal to or less than 1/2 the mass of the malt).

Usually, capric acid is not a compound that is directly related to the brewing fragrance, but is known to be an important factor in imparting thickness and body to beer, beer-taste drinks, non-alcohol drinks, RTDs (e.g., low-alcohol drinks), spirits, and whisky. Therefore, capric acid is a very important and indispensable substance in the beer-like fragrance-imparting composition.

In the present invention, the "off-flavor" refers to a feeling perceived as "abnormal odor" when a person judges that it is far from "normal odor" due to, for example, deterioration-related odor or oxidation-related odor, or a negative indicator.

The method for producing a yeast cell wall-derived decomposition product-containing composition is not particularly limited and may be appropriately selected depending on the intended purpose. The beer yeast cell wall-derived decomposition product-containing composition is preferably produced by the same method as the method for producing a yeast cell wall-derived decomposition product-containing composition except for selectively using beer yeast as the kind of the yeast as a raw material of the yeast cell wall. In this case, the beer yeast cell wall-derived decomposition product-containing composition has properties corresponding to the enzymatically treated product (the enzymatically treated product (1) or the enzymatically treated product (2)) and preferable forms thereof and the like are also similar thereto.

The beer yeast is not particularly limited and may be appropriately selected depending on the intended purpose.

The beer yeast used may be extra yeast formed through beer brewing or may be a by-product thereof. The extra yeast formed through beer brewing or the by-product thereof found limited applications due to its unique fragrance without any treatment. The yeast cell wall formed from the beer yeast obtained by the autolysis method conventionally found further limited applications of use due to its rotten odor and hop's oxidation-related odor. Meanwhile, the beer-like fragrance-imparting composition of the present invention includes the beer yeast cell wall-derived decomposition product-containing composition and advantageously has less off-flavor and a favorable beer-like fragrance.

The content of the yeast cell wall-derived decomposition product-containing composition (enzymatically treated product) in the beer-like fragrance-imparting composition is not particularly limited and may be appropriately selected depending on the intended purpose. The beer-like fragrance-imparting composition may be the yeast cell wall-derived decomposition product-containing composition (enzymatically treated product) itself.

<Other ingredients>

The other ingredients in the beer-like fragrance-imparting composition are not particularly limited and may be appropriately selected depending on the intended purpose. Examples of the other ingredients include solvents, pH adjusters, fungicides such as salts, glycerin, and propylene glycol. These may be used alone or in combination. The beer-like fragrance-imparting composition may be a beer-like fragrance-imparting composition including enzymes such as the exoglucanase, the protease, and the exopeptidase derived from the yeast cell wall-derived decomposition product-containing composition.

The content of the other ingredients in the beer-like fragrance-imparting composition is not particularly limited and may be appropriately selected depending on the intended purpose.

Applications of the beer-like fragrance-imparting composition are not particularly limited and may be appropriately selected depending on the intended purpose. The beer-like fragrance-imparting composition is excellent in solubility and dispersion stability, has less off-flavor, and has a favorable beer-like fragrance. Thus, the beer-like fragrance-imparting composition can be used for at least one of beer-like fragrance enhancement and beer-like fragrance impartment in, for example, food or drink, pet food, and a pet food palatability enhancer.

Also, the beer-like fragrance-imparting composition can be used as additives or raw materials thereof such as perfumes, perfume raw materials, food materials, and extraction raw materials for at least one of beer-like fragrance enhancement and beer-like fragrance impartment.

For example, the beer-like fragrance-imparting composition can be added during brewing, preparation, or mixing of, for example, beer, beer-taste drinks, non-alcohol drinks, RTDs, spirits, or whisky.

Moreover, such beer-like fragrance and yeast odor are also known to have the effect of attracting insects and mites. Therefore, the beer-like fragrance-imparting composition can also be used as an insect pesticide or a mite pesticide.

(Roast flavor-imparting composition and roast color-imparting composition)

A roast flavor-imparting composition of the present invention includes the yeast cell wall-derived decomposition product-containing composition (heat-treated product) of the present invention and if necessary, further includes other ingredients.

A roast color-imparting composition of the present invention includes the yeast cell wall-derived decomposition product-containing composition (heat-treated product) of the present invention and if necessary, further includes other ingredients.

<Yeast cell wall-derived decomposition product-containing composition>

Because the yeast cell wall-derived decomposition product-containing composition in the roast flavor-imparting composition or the roast color-imparting composition is the yeast cell wall-derived decomposition product-containing composition (heat-treated product) of the present invention, preferable forms and the like are as described in the above section "-Yeast cell wall-derived decomposition product-containing composition (heat-treated product)-".

Therefore, a method for producing the yeast cell wall-derived decomposition product-containing composition (heat-treated product) is not particularly limited and may be appropriately selected depending on the intended purpose. It is preferably produced by the same method as the method for producing the yeast cell wall-derived decomposition product-containing composition.

The content of the yeast cell wall-derived decomposition product-containing composition (heat-treated product) in the roast flavor-imparting composition or the roast color-imparting composition is not particularly limited and may be appropriately selected depending on the intended purpose. The roast flavor-imparting composition or the roast color-imparting composition may be the yeast cell wall-derived decomposition product-containing composition (heat-treated product) itself.

<Other ingredients>

The other ingredients in the roast flavor-imparting composition or the roast color-imparting composition are not particularly limited and may be appropriately selected depending on the intended purpose. Examples of the other ingredients include solvents, pH adjusters, fungicides such as salts, glycerin, and propylene glycol. These may be used alone or in combination.

The content of the other ingredients in the roast flavor-imparting composition or the roast color-imparting composition is not particularly limited and may be appropriately selected depending on the intended purpose.

Applications of the roast flavor-imparting composition or the roast color-imparting composition are not particularly limited and may be appropriately selected depending on the intended purpose. The roast flavor-imparting composition or the roast color-imparting composition is excellent in solubility and dispersion stability and is favorable in a roast flavor, a fat and oil feeling, and a roast color, and thus can be used for at least one selected from the group consisting of roast flavor enhancement, roast flavor impartment, roast color enhancement, roast color impartment, fat and oil feeling enhancement, and fat and oil feeling impartment in, for example, food or drink, pet food, and a pet food palatability enhancer.

Also, the roast flavor-imparting composition or the roast color-imparting composition can be used as additives or raw materials thereof such as perfumes and perfume raw materials for at least one selected from the group consisting of roast flavor enhancement, roast flavor impartment, roast color enhancement, roast color impartment, fat and oil feeling enhancement, and fat and oil feeling impartment.

(Food or drink)

A food or drink of the present invention includes at least one of the yeast cell wall-derived decomposition product-containing composition (enzymatically treated product) of the present invention and the yeast cell wall-derived decomposition product-containing composition (heat-treated product) of the present invention and if necessary, further includes other ingredients.

The food or drink refers to those which are less harmful to human health and which are given orally or through the gastrointestinal tract in the ordinary social life. They are not limited to categories of foods or drinks, drugs, and quasi drugs within the administrative boundaries, but include a wide variety of orally-given common foods, healthy foods, health-promoting foods, cosmetic foods, quasi drugs, and drugs.

The food or drink is not particularly limited and may be appropriately selected depending on the intended purpose. Examples of the food or drink include drinks such as beer, beer-taste drinks, non-alcohol drinks, RTDs, spirits, whisky, refreshing drinks, carbonated drinks, energy drinks, fruit drinks, and lactic drinks (including concentrated liquids and powder for preparation of these drinks); frozen desserts such as ice cream, ice sherbet, and ice shavings; noodles such as buckwheat noodles, wheat noodles, vermicelli, coats of Chinese dumplings, coats of pork dumplings, Chinese noodles, and instant noodles; snacks such as candies, gum, chocolate, tabletted snacks, munches, biscuits, jelly, jam, cream, peanuts cream, baked confectionery, and bread; marine products such as crab, salmon, Japanese littleneck, tuna, sardine, shrimps, prawns, bonito, mackerel, whale, oyster, saury, squid, bloody clam, scallop, abalone, sea chestnut, salmon caviar, and *Sulculus diversicolor supertexta*; marine/livestock processed foods such as fish minced and steamed, ham and sausage; dairy products such as processed milk and fermented milk; fats and oils or processed foods thereof such as salad oil, *Tempura* oil, margarine, mayonnaise, shortening, whip cream and dressing; seasonings such as sugar, salt, vinegar, soy sauce, *miso,* sauce, basting, consommé stock, and bouillon; retort pouch foods such as curry, stew, *Oyako-don* (a bowl of rice topped with boiled chicken and eggs), rice porridge, Zosui (rice soup), *Chuka-don* (a bowl of rice with a chop-suey-like mixture on it), *Katsu-don* (a rice bowl with pork cutlets), *Ten-don* (a *tempura* rice bowl), *Una-don* (an eel rice bowl), *hayashi rice* (hashed beef with rice), *Oden* (a dish containing several ingredients such as boiled eggs and radish), mapo doufu, *Gyu-don* (a beef rice bowl), meat sauce, egg soup, rice omelet, Chinese dumplings, pork dumplings, hamburger steak and meat balls; prepared foods such as salad and pickles; healthy, cosmetic, and dietary supplemental foods in various forms; and pharmaceutical drugs and quasi drugs such as tablets, granules, capsules, drinkable preparations, jelly preparations and troches. The food or drink is not limited to the above examples.

The content of at least one of the yeast cell wall-derived decomposition product-containing composition (heat-treated product) and the yeast cell wall-derived decomposition product-containing composition (enzymatically treated product) in the food or drink is not particularly limited and may be appropriately selected depending on the intended purpose.

Examples of the other ingredients include auxiliary materials and additives that are commonly used for producing food or drink.

The auxiliary materials and additives are not particularly limited and may be appropriately selected depending on the intended purpose. Examples of the auxiliary materials and additives include glucose, fructose, xylose, ribose, sucrose, maltose, sorbitol, stevioside, rubusoside, corn syrup, lactose, citric acid, tartaric acid, malic acid, succinic acid, lactic acid, L-ascorbic acid, dl-$\alpha$-tocopherol, sodium erythorbate, glycerin, propylene glycol, glycerin fatty acid esters, polyglycerin fatty acid esters, sucrose fatty acid esters, sorbitan fatty acid esters, gum arabic, carrageenan, casein, gelatin, pectin, agar, vitamins B, nicotinic-acid amide, calcium pantothenate, amino acids, glutathione, calcium salts, dyes, perfumes, and preservatives.

The content of the other ingredients in the food or drink is not particularly limited and may be appropriately selected depending on the intended purpose.

A method for producing the food or drink is not particularly limited and may be appropriately selected from known methods for producing food or drink as long as at least one of the yeast cell wall-derived decomposition product-containing composition (heat-treated product) and the yeast cell wall-derived decomposition product-containing composition (enzymatically treated product) is incorporated therein.

The food or drink of the present invention includes at least one of the yeast cell wall-derived decomposition product-containing composition (enzymatically treated product) and the yeast cell wall-derived decomposition product-containing composition (heat-treated product). Thus, the food or drink of the present invention can be formed into food or drink having not only the intrinsic flavor or roast color of the food or drink itself but also an imparted or enhanced roast flavor, beer-like fragrance, or roast color based on the roast flavor, the beer-like fragrance, or the roast color of these.

Also, the at least one of the yeast cell wall-derived decomposition product-containing composition (enzymatically treated product) and the yeast cell wall-derived decomposition product-containing composition (heat-treated product) is derived from the yeast cell wall and is able to impart or enhance a roast flavor, a beer-like fragrance, or a roast color without incorporation of the other additives. Thus, in the EU, a clean label and natural can be attached to a product and displayed, which is advantageous.

(Pet food palatability enhancer)

A pet food palatability enhancer of the present invention includes at least one of the yeast cell wall-derived decomposition product-containing composition (enzymatically treated product) of the present invention and the yeast cell wall-derived decomposition product-containing composition (heat-treated product) of the present invention and if necessary, further includes other ingredients.

In the present invention, the "pet food palatability enhancer" is one raw material for pet food and means a substance having the effect of enhancing palatability by the addition of it to pet food in a small amount (e.g., the effect of driving pets to eat it and the effect of increasing the amount of a diet taken).

In general, yeast materials, fish powder, etc. obtained from raw materials such as yeast, a Maillard substance, an amino acid, and a sugar are added to pet food as a pet food palatability enhancer in an amount of from about 1% by mass to about 2% by mass. Some known palatability enhancers for pet food use yeast, a yeast extract, a yeast cell wall, etc. However, conventional palatability enhancers for pet food are obtained by causing the Maillard reaction by the sole addition of such yeast materials as yeast, a yeast extract, and a yeast cell wall or by the addition of a mixture containing amino acids (e.g., lysine, cysteine, and methionine) and sugars (e.g., a reducing sugar, such as xylose) added to the yeast materials. When these substances are added to pet food, they are displayed as additives.

Meanwhile, the pet food palatability enhancer of the present invention includes at least one of the yeast cell wall-derived decomposition product-containing composition (enzymatically treated product) of the present invention and the yeast cell wall-derived decomposition product-containing composition (heat-treated product) of the present invention. Thus, it can supply a pet food palatability enhancer or pet food containing it without additives, and a clean label and natural can be attached to a product and displayed, which is advantageous. Such a pet food palatability enhancer did not exist hitherto.

<Yeast cell wall-derived decomposition product-containing composition>

The yeast cell wall-derived decomposition product-containing composition is at least one of the yeast cell wall-derived decomposition product-containing composition (enzymatically treated product) of the present invention and the yeast cell wall-derived decomposition product-containing composition (heat-treated product) of the present invention. Therefore, preferable forms and the like are as described in the section "-Yeast cell wall-derived decomposition product-containing composition (enzymatically treated product)-" and the section "-Yeast cell wall-derived decomposition product-containing composition (heat-treated product)-". Among them, the pet food palatability enhancer preferably contains the yeast cell wall-derived decomposition product-containing composition (enzymatically treated product) containing at least one compound selected from the group consisting of ethyl caproate, ethyl caprylate, ethyl caprate, phenylethyl alcohol, and capric acid or the yeast cell wall-derived decomposition product-containing composition (heat-treated product) containing at least one compound selected from the group consisting of pyrazine, methylpyrazine, 2,5-dimethylpyrazine, 2,6-dimethylpyrazine, 2,3-dimethylpyrazine, and furaneol. It is more preferable to contain the yeast cell wall-derived decomposition product-containing composition (heat-treated product) containing at least one compound selected from the group consisting of pyrazine, methylpyrazine, 2,5-dimethylpyrazine, 2,6-dimethylpyrazine, 2,3-dimethylpyrazine, and furaneol. It is preferable that the pet food palatability enhancer contain at least one of the yeast cell wall-derived decomposition product-containing composition (enzymatically treated product) and the yeast cell wall-derived decomposition product-containing composition (heat-treated product) each containing these compounds (fragrance ingredients) in terms of being able to stimulate a sense of smell of a pet to increase the incitement and the amount of a diet taken; i.e., enhance palatability.

A method for producing the yeast cell wall-derived decomposition product-containing composition (enzymatically treated product) or a method for producing the yeast cell wall-derived decomposition product-containing composition (heat-treated product) is not particularly limited and may be appropriately selected depending on the intended purpose. They are preferably produced by the same method as the method for producing the yeast cell wall-derived decomposition product-containing composition.

The content of the at least one of the yeast cell wall-derived decomposition product-containing composition (enzymatically treated product) and the yeast cell wall-derived decomposition product-containing composition (heat-treated product) in the pet food palatability enhancer is not particularly limited and may be appropriately selected depending on the intended purpose. The pet food palatability enhancer may be the at least one itself of the yeast cell wall-derived decomposition product-containing composition (enzymatically treated product) and the yeast cell wall-derived decomposition product-containing composition (heat-treated product).

<Other ingredients>

Examples of the other ingredients include raw materials, auxiliary raw materials, or additives that are usually used for producing the pet food palatability enhancer.

The content of the other ingredients in the pet food palatability enhancer is not particularly limited and may be appropriately selected depending on the intended purpose.

A method for producing the pet food palatability enhancer is not particularly limited and may be appropriately selected depending on known methods for producing a pet food palatability enhancer as long as at least one of the yeast cell wall-derived decomposition product-containing composition (enzymatically treated product) and the yeast cell wall-derived decomposition product-containing composition (heat-treated product) is incorporated therein.

A form of the pet food palatability enhancer is not particularly limited and may be appropriately selected depending on the intended purpose. Examples of the form include a form of incorporating it during the production of pet food (e.g., kneading it into pellets of pet food) and a form of sprinkling or coating it on the finished pet food.

The amount of the pet food palatability enhancer to be added to pet food is not particularly limited and may be appropriately selected depending on the intended purpose. The upper limit thereof is preferably less than 1% by mass, more preferably 0.5% by mass or less, particularly preferably 0.3% by mass or less, relative to the total amount of the pet food. The lower limit of the amount of the pet food palatability enhancer to be added to pet food is preferably 0.1% by mass or more but 0.2% by mass or less.

The upper limit and the lower limit of the amount of the pet food palatability enhancer to be added to pet food may be appropriately combined. The amount thereof is preferably 0.1% by mass or more but less than 1% by mass, more preferably 0.1% by mass or more but 0.5% by mass or less, particularly preferably 0.2% by mass or more but 0.3% by mass or less. When the amount of from about 1% by mass to 2% by mass is desired for the mixing step, the above concentration or therearound may be achieved through dilution with, for example, other palatability enhancers, dispersants, and excipients.

A target pet of the pet food palatability enhancer is not particularly limited and may be appropriately selected depending on the intended purpose. Examples of the target pet include animals other than humans, such as monkeys, pigs, cows, sheep, goats, dogs, cats, mice, rats, hamsters, birds, and fish.

A method for confirming palatability of the pet is not particularly limited and may be appropriately selected from known methods. For example, evaluation by the 2-point evaluation method is common. In the 2-point evaluation method, the target pet is given simultaneously pet food containing the pet food palatability enhancer and a control pet food (e.g., pet food free of the pet food palatability enhancer or pet food containing a commercially available pet food palatability enhancer) to determine, as pet food having higher palatability, the pet food that the target pet first puts in its mouth (hereinafter may be referred to as "FC; First Choice" or "first bite") and that the target pet intakes more,.

The pet food palatability enhancer includes at least one of the yeast cell wall-derived decomposition product-containing composition (enzymatically treated product) and the yeast cell wall-derived decomposition product-containing composition (heat-treated product). Thus, advantageously, the pet food can be formed into pet food having not only the intrinsic flavor or color of the pet food itself but also an imparted or enhanced feature that is at least one of a beer-like fragrance, a roast flavor, a fat and oil feeling, and a roast color based on the flavor thereof (i.e., beer-like fragrance) or at least one of the roast flavor (e.g., meat flavor), the fat and oil feeling, and the roast color thereof.

Examples

The present invention will be described in detail by way of Preparation Examples and Test Examples below. However, the present invention should not be construed as being limited to these Preparation Examples and Test Examples.

(Preparation Example 1-1: Autolysis-type yeast cell wall slurry)

A slurry of the yeast cell wall derived from beer yeast belonging to the genus Saccharomyces (autolysis-type yeast cell wall) was prepared to have a slurry concentration of 10% by mass. Specifically, 100 g of the yeast cell wall (autolysis-type yeast cell wall) (product name: yeast cell wall, product of Asahi Group Foods, Ltd.) was suspended in 900 g of water to prepare an "autolysis-type yeast cell wall slurry".
The "yeast cell wall" (product of Asahi Group Foods, Ltd.) used as the yeast cell wall (autolysis-type yeast cell wall) is powder that was spray dried.

(Preparation Example 1-2: Autoclave-treated product (1) of the autolysis-type yeast cell wall)

The autolysis-type yeast cell wall slurry prepared in Preparation Example 1-1 (slurry concentration: 10% by mass) was heat-treated in an autoclave at 120°C for 15 minutes to obtain an "autoclave-treated product (1) of the autolysis-type yeast cell wall".

(Preparation Example 1-3: Autoclave-treated product (2) of the autolysis-type yeast cell wall)

The autolysis-type yeast cell wall slurry prepared in Preparation Example 1-1 (slurry concentration: 10% by mass) was heat-treated in an autoclave at 120°C for 30 minutes to obtain an "autoclave-treated product (2) of the autolysis-type yeast cell wall".

(Preparation Example 2-1: Hot-water extraction-type yeast cell wall slurry)

A slurry of the yeast cell wall derived from baker's yeast belonging to the genus Saccharomyces (hot-water extraction-type yeast cell wall) was prepared to have a slurry concentration of 16% by mass. Specifically, 160 g of the yeast cell wall (hot-water extraction-type yeast cell wall) (product name: HG-YCW, product of Asahi Group Foods, Ltd.) was suspended in 840 g of water to prepare a "hot-water extraction-type yeast cell wall slurry".
The "HG-YCW" (product of Asahi Group Foods, Ltd.) used as the yeast cell wall (hot-water extraction-type yeast cell wall) is powder that was spray dried.

(Preparation Example 2-2: Autoclave-treated product (1) of the hot-water extraction-type yeast cell wall)

The hot-water extraction-type yeast cell wall slurry of Preparation Example 2-1 (slurry concentration: 16% by mass) was heat-treated in an autoclave at 120°C for 15 minutes to obtain an "autoclave-treated product (1) of the hot-water extraction-type yeast cell wall".

(Preparation Example 2-3: Autoclave-treated product (2) of the hot-water extraction-type yeast cell wall)

The hot-water extraction-type yeast cell wall slurry of Preparation Example 2-1 (slurry concentration: 16% by mass) was heat-treated in an autoclave at 120°C for 30 minutes to obtain an "autoclave-treated product (2) of the hot-water extraction-type yeast cell wall".

(Preparation Example 3-1: Autolysis-type yeast cell wall-derived decomposition product-containing composition (1E))

1 kg of the autoclave-treated product (1) of the autolysis-type yeast cell wall obtained in Preparation Example 1-2 was placed in a jar having a volume of 2 L and adjusted to pH 5.7 using sodium hydroxide. Then, the following three kinds of enzymes; i.e., a glucanase derived from the genus Talaromyces (FILTRASE (registered trademark) BRX, product of DSM Japan, Ltd.), a protease derived from the genus Bacillus (Alcalase (registered trademark) 2.4 L FG, product of Novozymes Japan, Ltd.), and an exopeptidase derived from the genus Aspergillus (Flavourzyme (registered trademark) 1000L, product of Novozymes Japan, Ltd.) were added at the same time so as to be 0.5% by mass each, relative to the solid content mass of the autoclave-treated product (1) of the autolysis-type yeast cell wall. The resultant mixture was enzymatically treated for 24 hours under stirring at 50°C and 200 rpm to obtain an "autolysis-type yeast cell wall-derived decomposition product-containing composition (1E)". The pH of the autolysis-type yeast cell wall-derived decomposition product-containing composition (1E) after the enzymatic treatment for 24 hours was found to be 5.5.

The pH was measured with a pH meter (model: F-52/glass electrode, HORIBA, Ltd.). In the following Preparation Examples, the pH was measured in the same manner.

(Preparation Example 3-2: Autolysis-type yeast cell wall-derived decomposition product-containing composition (2E))

An "autolysis-type yeast cell wall-derived decomposition product-containing composition (2E)" was obtained in the same manner as in Preparation Example 3-1 except that for the enzymatic treatment, the exopeptidase derived from the genus Aspergillus was not used and only two kinds of the enzymes; i.e., the glucanase derived from the genus Talaromyces (FILTRASE (registered trademark) BRX, product of DSM Japan, Ltd.) and the protease derived from the genus Bacillus (Alcalase (registered trademark) 2.4 L FG, product of Novozymes Japan, Ltd.) were added at the same time so as to be 0.5% by mass each, relative to the solid content mass of the autoclave-treated product (1) of the autolysis-type yeast cell wall. The pH of the autolysis-type yeast cell wall-derived decomposition product-containing composition (2E) after the enzymatic treatment for 24 hours was found to be 5.5. (Preparation Example 3-3: Autolysis-type yeast cell wall-derived decomposition product-containing composition (3E))

In Preparation Example 3-3, the protease, the glucanase, and the exopeptidase were used for the enzymatic treatment at two separate steps in the following manner.

As the first step, 1 kg of the autoclave-treated product (1) of the autolysis-type yeast cell wall obtained in Preparation Example 1-2 was placed in a jar having a volume of 2 L and adjusted to pH 8.0 using sodium hydroxide. Then, the protease derived from the genus Bacillus (Alcalase (registered trademark) 2.4 L FG, product of Novozymes Japan, Ltd.) was added so as to be 0.5% by mass relative to the solid content mass of the autoclave-treated product (1) of the autolysis-type yeast cell wall. The resultant mixture was enzymatically treated for 6 hours under stirring at 65°C and 200 rpm. The pH of the autoclave-treated product (1) of the autolysis-type yeast cell wall after the enzymatic treatment with the protease was found to be 7.3.

As the second step, the autoclave-treated product (1) of the autolysis-type yeast cell wall treated with the protease was adjusted to pH 5.5 with hydrochloric acid. Then, the glucanase derived from the genus Talaromyces (FILTRASE (registered trademark) BRX, product of DSM Japan, Ltd.) and the exopeptidase derived from the genus Aspergillus (Flavourzyme (registered trademark) 1000L, product of Novozymes Japan, Ltd.) were added so as to be 0.5% by mass each, relative to the solid content mass of the autoclave-treated product (1) of the autolysis-type yeast cell wall. The resultant mixture was enzymatically treated for 18 hours under stirring at 50°C and 200 rpm to obtain an "autolysis-type yeast cell wall-derived decomposition product-containing composition (3E)". The pH of the autolysis-type yeast cell wall-derived decomposition product-containing composition (3E) after the enzymatic treatment with the glucanase and the exopeptidase was found to be 5.5.

(Preparation Example 3-4: Autolysis-type yeast cell wall-derived decomposition product-containing composition (4E))

1 kg of the autoclave-treated product (1) of the autolysis-type yeast cell wall obtained in Preparation Example 1-2 was placed in a jar having a volume of 2 L and adjusted to pH 5.5 using sodium hydroxide. Then, the glucanase derived from the genus Talaromyces (FILTRASE (registered trademark) BRX, product of DSM Japan, Ltd.) was added at the same time so as to be 0.5% by mass relative to the solid content mass of the autoclave-treated product (1) of the autolysis-type yeast cell wall. The resultant mixture was enzymatically treated for 24 hours under stirring at 50°C and 200 rpm to obtain an "autolysis-type yeast cell wall-derived decomposition product-containing composition (4E)". The pH of the autolysis-type yeast cell wall-derived decomposition product-containing composition (4E) after the enzymatic treatment for 24 hours was found to be 5.5.

(Preparation Example 4-1: Hot-water extraction-type yeast cell wall-derived decomposition product-containing composition (1E))

1 kg of the autoclave-treated product (1) of the hot-water extraction-type yeast cell wall obtained in Preparation Example 2-2 was placed in a jar having a volume of 2 L and adjusted to pH 5.7 using sodium hydroxide. Then, the following three kinds of enzymes; i.e., the glucanase derived from the genus Talaromyces (FILTRASE (registered trademark) BRX, product of DSM Japan, Ltd.), the protease derived from the genus Bacillus (Alcalase (registered trademark) 2.4 L FG, product of Novozymes Japan, Ltd.), and the exopeptidase derived from the genus Aspergillus (Flavourzyme (registered trademark) 1000L, product of Novozymes Japan, Ltd.) were added at the same time so as to be 0.5% by mass each, relative to the solid content mass of the autoclave-treated product (1) of the hot-water extraction-type yeast cell wall. The resultant mixture was enzymatically treated for 24 hours under stirring at 50°C and 200 rpm to obtain a "hot-water extraction-type yeast cell wall-derived decomposition product-containing composition (1E)". The pH of the hot-water extraction-type yeast cell wall-derived decomposition product-containing composition (1E) after the enzymatic treatment for 24 hours was found to be 5.5.

(Preparation Example 4-2: Hot-water extraction-type yeast cell wall-derived decomposition product-containing composition (2E))

A hot-water extraction-type yeast cell wall-derived decomposition product-containing composition (2E) was obtained in the same manner as in Preparation Example 4-1 except that for the enzymatic treatment, the exopeptidase derived from the genus Aspergillus was not used and only two kinds of the enzymes; i.e., the glucanase derived from the genus Talaromyces (FILTRASE (registered trademark) BRX, product of DSM Japan, Ltd.) and the protease derived from the genus Bacillus (Alcalase (registered trademark) 2.4 L FG, product of Novozymes Japan, Ltd.) were added at the same time so as to be 0.5% by mass each, relative to the solid content mass of the autoclave-treated product (1) of the hot-water extraction-type yeast cell wall. The pH of the hot-water extraction-type yeast cell wall-derived decomposition product-containing composition (2E) after the enzymatic treatment for 24 hours was found to be 5.5.

(Preparation Example 4-3: Hot-water extraction-type yeast cell wall-derived decomposition product-containing composition (3E))

In Preparation Example 4-3, the protease, the glucanase, and the exopeptidase were used for the enzymatic treatment at two separate steps in the following manner.
As the first step, 1 kg of the autoclave-treated product (1) of the hot-water extraction-type yeast cell wall obtained in Preparation Example 2-2 was placed in a jar having a volume of 2 L and adjusted to pH 8 using sodium hydroxide. Then, the protease derived from the genus Bacillus (Alcalase (registered trademark) 2.4 L FG, product of Novozymes Japan, Ltd.) was added so as to be 0.5% by mass relative to the solid content mass of the autoclave-treated product (1) of the hot-water extraction-type yeast cell wall. The resultant mixture was enzymatically treated for 6 hours under stirring at 65°C and 200 rpm. The pH of the autoclave-treated product (1) of the hot-water extraction-type yeast cell wall after the enzymatic treatment with the protease was found to be 7.0.
As the second step, the autoclave-treated product (1) of the hot-water extraction-type yeast cell wall treated with the protease was adjusted to pH 5.5 with hydrochloric acid. Then, the glucanase derived from the genus Talaromyces (FILTRASE (registered trademark) BRX, product of DSM Japan, Ltd.) and the exopeptidase derived from the genus Aspergillus (Flavourzyme (registered trademark) 1000L, product of Novozymes Japan, Ltd.) were added so as to be 0.5% by mass each, relative to the solid content mass of the autoclave-treated product (1) of the hot-water extraction-type yeast cell wall. The resultant mixture was enzymatically treated for 18 hours under stirring at 50°C and 200 rpm to obtain a "hot-water extraction-type yeast cell wall-derived decomposition product-containing composition (3E)". The pH of the hot-water extraction-type yeast cell wall-derived decomposition product-containing composition (3E) after the enzymatic treatment with the glucanase and the exopeptidase was found to be 5.5.

(Preparation Example 4-4: Hot-water extraction-type yeast cell wall-derived decomposition product-containing composition (4E))

1 kg of the autoclave-treated product (1) of the hot-water extraction-type yeast cell wall obtained in Preparation Example 2-1 was placed in a jar having a volume of 2 L and adjusted to pH 5.5 using sodium hydroxide. Then, the glucanase derived from the genus Talaromyces (FILTRASE (registered trademark) BRX, product of DSM Japan, Ltd.) was added at the same time so as to be 0.5% by mass relative to the solid content mass of the autoclave-treated product (1) of the hot-water extraction-type yeast cell wall. The resultant mixture was enzymatically treated for 24 hours under stirring at 50°C and 200 rpm to obtain a "hot-water extraction-type yeast cell wall-derived decomposition product-containing composition (4E)". The pH of the hot-water extraction-type yeast cell wall-derived decomposition product-containing composition (4E) after the enzymatic treatment for 24 hours was found to be 5.5.
The conditions of Preparation Examples 3-1 to 4-4 are collectively presented in Table 1 below.

Table 1

| Preparation Example | | Raw material used | Enzymatically treating step | | |
|---|---|---|---|---|---|
| | | | Glucanase | Protease | Exopeptidase |
| Preparation Example 3-1 | Autolysis-type yeast cell wall-derived decomposition product-containing composition (1E) | Autoclave -treated product (1) of the autolysis-type yeast cell wall | 0.5% by mass | 0.5% by mass | 0.5% by mass |
| Preparation Example 3-2 | Autolysis-type yeast cell wall-derived decomposition product-containing composition (2E) | Autoclave -treated product (1) of the autolysis-type yeast cell wall | 0.5% by mass | 0.5% by mass | - |
| Preparation Example 3-3 | Autolysis-type yeast cell wall-derived decomposition product-containing composition (3E) | Autoclave -treated product (1) of the autolysis-type yeast cell wall | 0.5% by mass | 0.5% by mass | 0.5% by mass |
| Preparation Example 3-4 | Autolysis-type yeast cell wall-derived decomposition product-containing composition (4E) | Autoclave -treated product (1) of the autolysis-type yeast cell wall | 0.5% by mass | - | - |
| Preparation Example 4-1 | Hot-water extraction-type yeast cell wall-derived decomposition product-containing composition (1E) | Autoclave -treated product (1) of the hot-water extraction-type yeast cell wall | 0.5% by mass | 0.5% by mass | 0.5% by mass |
| Preparation Example 4-2 | Hot-water extraction-type yeast cell wall-derived decomposition product-containing composition (2E) | Autoclave -treated product (1) of the hot-water extraction-type yeast cell wall | 0.5% by mass | 0.5% by mass | - |
| Preparation Example 4-3 | Hot-water extraction-type yeast cell wall-derived decomposition product-containing composition (3E) | Autoclave -treated product (1) of the hot-water extraction-type yeast cell wall | 0.5% by mass | 0.5% by mass | 0.5% by mass |
| Preparation Example 4-4 | Hot-water extraction-type yeast cell wall-derived decomposition product-containing composition (4E) | Autoclave -treated product (1) of the hot-water extraction-type yeast cell wall | 0.5% by mass | - | - |

(Preparation Example 5-1: Autolysis-type yeast cell wall-derived decomposition product-containing composition (1M))

The autolysis-type yeast cell wall-derived decomposition product-containing composition (1E) obtained in Preparation Example 3-1 was adjusted to pH 8.0 with sodium hydroxide. Then, the resultant was heat-treated using an autoclave (LSX-300, product of TOMY SEIKO CO., LTD.) at 120°C for 30 minutes. The pH of the heat-treated product was found to be 6.4. The heat-treated product was adjusted to pH 6.0 with hydrochloric acid to obtain an "autolysis-type yeast cell wall-derived decomposition product-containing composition (1M)".

(Preparation Example 5-2: Autolysis-type yeast cell wall-derived decomposition product-containing composition (2M))

The autolysis-type yeast cell wall-derived decomposition product-containing composition (1E) obtained in Preparation Example 3-1 was adjusted to pH 8.0 with sodium hydroxide. Then, the resultant was heat-treated using an autoclave (LSX-300, product of TOMY SEIKO CO., LTD.) at 90°C for 3 hours. The pH of the heat-treated product was found to be 6.5. The heat-treated product was adjusted to pH 6.0 with hydrochloric acid to obtain an "autolysis-type yeast cell wall-derived decomposition product-containing composition (2M)".

(Preparation Example 5-3: Autolysis-type yeast cell wall-derived decomposition product-containing composition (3M))

The autolysis-type yeast cell wall-derived decomposition product-containing composition (1E) obtained in Preparation Example 3-1 was adjusted to pH 10.0 with sodium hydroxide. Then, the resultant was heat-treated using an autoclave (LSX-300, product of TOMY SEIKO CO., LTD.) at 120°C for 30 minutes. The pH of the heat-treated product was found to be 7.3. The heat-treated product was adjusted to pH 6.0 with hydrochloric acid to obtain an "autolysis-type yeast cell wall-derived decomposition product-containing composition (3M)".

(Preparation Example 5-4: Autolysis-type yeast cell wall-derived decomposition product-containing composition (4M))

The autolysis-type yeast cell wall-derived decomposition product-containing composition (1E) obtained in Preparation Example 3-1 was adjusted to pH 10.0 with sodium hydroxide. Then, the resultant was heat-treated using an autoclave (LSX-300, product of TOMY SEIKO CO., LTD.) at 90°C for 3 hours. The pH of the heat-treated product was found to be 7.6. The heat-treated product was adjusted to pH 6.0 with hydrochloric acid to obtain an "autolysis-type yeast cell wall-derived decomposition product-containing composition (4M)".

(Preparation Example 6-1: Hot-water extraction-type yeast cell wall-derived decomposition product-containing composition (1M))

The hot-water extraction-type yeast cell wall-derived decomposition product-containing composition (1E) obtained in Preparation Example 4-1 was adjusted to pH 8.0 with sodium hydroxide. Then, the resultant was heat-treated using an autoclave (LSX-300, product of TOMY SEIKO CO., LTD.) at 120°C for 30 minutes. The pH of the heat-treated product was found to be 6.7. The heat-treated product was adjusted to pH 6.0 with hydrochloric acid to obtain a "hot-water extraction-type yeast cell wall-derived decomposition product-containing composition (1M)".

(Preparation Example 6-2: Hot-water extraction-type yeast cell wall-derived decomposition product-containing composition (2M))

The hot-water extraction-type yeast cell wall-derived decomposition product-containing composition (1E) obtained in Preparation Example 4-1 was adjusted to pH 8.0 with sodium hydroxide. Then, the resultant was heat-treated using an autoclave (LSX-300, product of TOMY SEIKO CO., LTD.) at 90°C for 3 hours. The pH of the heat-treated product was found to be 6.8. The heat-treated product was adjusted to pH 6.0 with hydrochloric acid to obtain a "hot-water extraction-type yeast cell wall-derived decomposition product-containing composition (2M)".

(Preparation Example 6-3: Hot-water extraction-type yeast cell wall-derived decomposition product-containing composition (3M))

The hot-water extraction-type yeast cell wall-derived decomposition product-containing composition (1E) obtained in Preparation Example 4-1 was adjusted to pH 10.0 with sodium hydroxide. Then, the resultant was heat-treated using an autoclave (LSX-300, product of TOMY SEIKO CO., LTD.) at 120°C for 30 minutes. The pH of the heat-treated product was found to be 7.9. The heat-treated product was adjusted to pH 6.0 with hydrochloric acid to obtain a "hot-water extraction-type yeast cell wall-derived decomposition product-containing composition (3M)".

(Preparation Example 6-4: Hot-water extraction-type yeast cell wall-derived decomposition product-containing composition (4M))

The hot-water extraction-type yeast cell wall-derived decomposition product-containing composition (1E) obtained in Preparation Example 4-1 was adjusted to pH 10.0 with sodium hydroxide. Then, the resultant was heat-treated using an autoclave (LSX-300, product of TOMY SEIKO CO., LTD.) at 90°C for 3 hours. The pH of the heat-treated product was found to be 8.0. The heat-treated product was adjusted to pH 6.0 with hydrochloric acid to obtain a "hot-water extraction-type yeast cell wall-derived decomposition product-containing composition (4M)".

The conditions of Preparation Examples 5-1 to 6-4 are collectively presented in Table 2 below.

Table 2

| Preparation Example | | Raw material used | pH before heat treatment | Heat treating step | | pH after heat treatment |
|---|---|---|---|---|---|---|
| | | | | Temperature | Period | |
| Preparation Example 5-1 | Autolysis-type yeast cell wall-derived decomposition product-containing composition (1M) | Autolysis-type yeast cell wall-derived decomposition product-containing composition (1E) | 8.0 | 120°C | 30 minutes | 6.4 |
| Preparation Example 5-2 | Autolysis-type yeast cell wall-derived decomposition product-containing composition (2M) | Autolysis-type yeast cell wall-derived decomposition product-containing composition (1E) | 8.0 | 90°C | 3 hours | 6.5 |
| Preparation Example 5-3 | Autolysis-type yeast cell wall-derived decomposition product-containing composition (3M) | Autolysis-type yeast cell wall-derived decomposition product-containing composition (1E) | 10.0 | 120°C | 30 minutes | 7.3 |
| Preparation Example 5-4 | Autolysis-type yeast cell wall-derived decomposition product-containing composition (4M) | Autolysis-type yeast cell wall-derived decomposition product-containing composition (1E) | 10.0 | 90°C | 3 hours | 7.6 |
| Preparation Example 6-1 | Hot-water extraction-type yeast cell wall-derived decomposition product-containing composition (1M) | Hot-water extraction-type yeast cell wall-derived decomposition product-containing composition (1E) | 8.0 | 120°C | 30 minutes | 6.7 |
| Preparation Example 6-2 | Hot-water extraction-type yeast cell wall-derived decomposition product-containing composition (2M) | Hot-water extraction-type yeast cell wall-derived decomposition product-containing composition (1E) | 8.0 | 90°C | 3 hours | 6.8 |
| Preparation Example 6-3 | Hot-water extraction-type yeast cell wall-derived decomposition product-containing composition (3M) | Hot-water extraction-type yeast cell wall-derived decomposition product-containing composition (1E) | 10.0 | 120°C | 30 minutes | 7.9 |
| Preparation Example 6-4 | Hot-water extraction-type yeast cell wall-derived decomposition product-containing composition (4M) | Hot-water extraction-type yeast cell wall-derived decomposition product-containing composition (1E) | 10.0 | 90°C | 3 hours | 8.0 |

<Test Example 1: Solubility ratio>

The products obtained in the Preparation Examples presented in Tables 3-1 and 3-2 below were used as test samples and measured for solubility ratio in the following manner.

The mass of a dry product obtained by drying 50 g of each of the test samples at 105°C for 5 hours was defined as "solid content mass B" (g) of the test sample.

Also, the mass of a dry product obtained by centrifuging the test sample of the yeast cell wall slurry (50 g) at 5,000 G for 5 minutes and drying the obtained supernatant at 105°C for 5 hours was defined as "solid content mass A" (g) solubilized in the test sample.

The solid content mass A and the solid content mass B were used in Formula (1) below to calculate a solubility ratio of the solid content in the test sample. The results are presented in Tables 3-1 and 3-2 below.

$$\text{Solubility ratio (\%)} = \text{Solid content mass A/Solid content mass B} \times 100 \ldots \text{Formula}$$

$$(1)$$

Table 3-1

| | Test samples | | Solubility ratio |
|---|---|---|---|
| Preparation Example 1-1 | Autolysis-type yeast cell wall slurry | | 26.8% |
| Preparation Example 1-2 | Autoclave-treated product (1) of the autolysis-type yeast cell wall | | 26.9% |
| Preparation Example 1-3 | Autoclave-treated product (2) of the autolysis-type yeast cell wall | | 26.9% |
| Preparation Example 3-1 | Autolysis-type yeast cell wall-derived decomposition product-containing composition (1E) | | 86.0% |
| Preparation Example 3-2 | Autolysis-type yeast cell wall-derived decomposition product-containing composition (2E) | | 85.8% |
| Preparation Example 3-3 | Autolysis-type yeast cell wall-derived decomposition product-containing composition (3E) | | 79.8% |
| Preparation Example 3-4 | Autolysis-type yeast cell wall-derived decomposition product-containing composition (4E) | | 86.5% |
| Preparation Example 5-1 | Autolysis-type yeast cell wall-derived decomposition product-containing composition (1M) | | 92.7% |
| Preparation Example 5-2 | Autolysis-type yeast cell wall-derived decomposition product-containing composition (2M) | | 92.3% |
| Preparation Example 5-3 | Autolysis-type yeast cell wall-derived decomposition product-containing composition (3M) | | 93.5% |
| Preparation Example 5-4 | Autolysis-type yeast cell wall-derived decomposition product-containing composition (4M) | | 93.1% |

Table 3-2

| | Test samples | | Solubility ratio |
|---|---|---|---|
| Test Example 2-1 | Hot-water extraction-type yeast cell wall slurry | | 22.6% |
| Test Example 2-2 | Autoclave-treated product (1) of the hot-water extraction-type yeast cell wall | | 22.8% |
| Test Example 2-3 | Autoclave-treated product (2) of the hot-water extraction-type yeast cell wall | | 22.8% |
| Preparation Example 4-1 | Hot-water extraction-type yeast cell wall-derived decomposition product-containing composition (1E) | | 80.8% |
| Preparation Example 4-2 | Hot-water extraction-type yeast cell wall-derived decomposition product-containing composition (2E) | | 80.7% |

(continued)

| Test samples | | Solubility ratio |
|---|---|---|
| Preparation Example 4-3 | Hot-water extraction-type yeast cell wall-derived decomposition product-containing composition (3E) | 75.8% |
| Preparation Example 4-4 | Hot-water extraction-type yeast cell wall-derived decomposition product-containing composition (4E) | 64.9% |
| Preparation Example 6-1 | Hot-water extraction-type yeast cell wall-derived decomposition product-containing composition (1M) | 88.7% |
| Preparation Example 6-2 | Hot-water extraction-type yeast cell wall-derived decomposition product-containing composition (2M) | 89.2% |
| Preparation Example 6-3 | Hot-water extraction-type yeast cell wall-derived decomposition product-containing composition (3M) | 90.8% |
| Preparation Example 6-4 | Hot-water extraction-type yeast cell wall-derived decomposition product-containing composition (4M) | 90.2% |

It was found that the solubility ratio did not change only by subjecting the yeast cell wall to the autoclave treatment, and the solubility ratio of the yeast cell wall increased by enzymatically treating the yeast cell wall with the exoglucanase alone or with the exoglucanase and the other enzymes at the same time. In the case of enzymatically treating the yeast cell wall with the exoglucanase and the protease at two separate steps, although they were used at the respective optimum pH, the result was that the increase in the solubility ratio was slightly poorer than in the case where these enzymes were used at the same time for the enzymatic treatment. Also, as compared with the case of the enzymatic treatment with these enzymes at the same time, the volume of the precipitated residues was more in the case of the enzymatic treatment with the exoglucanase and the protease at two separate steps.

It was found that the solubility ratio of the yeast cell wall further increased by subjecting the enzymatically treated product of the yeast cell wall to the heat treatment.

<Test Example 2: Color tone>

The products obtained in the Preparation Examples presented in Tables 4-1 and 4-2 below were used as test samples and measured for color tone in the following manner.

According to JIS Z-8722, a color difference meter was used to measure the color tone of each of the test samples under the following measurement conditions to determine the L* value, the a* value, and the b* value. The results are presented in Tables 4-1 and 4-2 below.

[Measurement conditions of color tone]

- Color difference meter: ZE 6000 (product of NIPPON DENSHOKU INDUSTRIES CO., LTD.)
- Light receiving conditions: reflection according to JIS Z-8722
- Measurement method: double beam mode
- Light source: halogen lamp 12V20W (NA55919)
- Color system: L*a*b*

Table 4-1

| Test samples | | Color tone | | |
|---|---|---|---|---|
| | | L* value | a* value | b* value |
| Preparation Example 1-1 | Autolysis-type yeast cell wall slurry | 73.28 | 5.29 | 26.11 |
| Preparation Example 1-2 | Autoclave-treated product (1) of the autolysis-type yeast cell wall | 71.56 | 5.38 | 26.71 |

(continued)

| Test samples | | Color tone | | |
| --- | --- | --- | --- | --- |
| | | L* value | a* value | b* value |
| Preparation Example 1-3 | Autoclave-treated product (2) of the autolysis-type yeast cell wall | 71.42 | 5.39 | 26.61 |
| Preparation Example 3-1 | Autolysis-type yeast cell wall-derived decomposition product-containing composition (1E) | 62.49 | 6.31 | 26.57 |
| Preparation Example 3-2 | Autolysis-type yeast cell wall-derived decomposition product-containing composition (2E) | 66.01 | 6.37 | 26.25 |
| Preparation Example 3-3 | Autolysis-type yeast cell wall-derived decomposition product-containing composition (3E) | 64.23 | 7.17 | 25.74 |
| Preparation Example 3-4 | Autolysis-type yeast cell wall-derived decomposition product-containing composition (4E) | 69.98 | 5.68 | 26.52 |
| Preparation Example 5-1 | Autolysis-type yeast cell wall-derived decomposition product-containing composition (1M) | 23.95 | 18.73 | 29.32 |
| Preparation Example 5-2 | Autolysis-type yeast cell wall-derived decomposition product-containing composition (2M) | 29.81 | 12.26 | 28.72 |
| Preparation Example 5-3 | Autolysis-type yeast cell wall-derived decomposition product-containing composition (3M) | 20.13 | 20.73 | 26.23 |
| Preparation Example 5-4 | Autolysis-type yeast cell wall-derived decomposition product-containing composition (4M) | 21.52 | 19.96 | 27.95 |

Table 4-2

| Test samples | | Color tone | | |
| --- | --- | --- | --- | --- |
| | | L* value | a* value | b* value |
| Preparation Example 2-1 | Hot-water extraction-type yeast cell wall slurry | 82.11 | 4.24 | 9.48 |
| Preparation Example 2-2 | Autoclave-treated product (1) of the hot-water extraction-type yeast cell wall | 81.72 | 4.48 | 9.50 |
| Preparation Example 2-3 | Autoclave-treated product (2) of the hot-water extraction-type yeast cell wall | 81.51 | 4.42 | 9.51 |
| Preparation Example 4-1 | Hot-water extraction-type yeast cell wall-derived decomposition product-containing composition (1E) | 71.94 | 5.61 | 10.76 |
| Preparation Example 4-2 | Hot-water extraction-type yeast cell wall-derived decomposition product-containing composition (2E) | 73.01 | 5.61 | 10.29 |
| Preparation Example 4-3 | Hot-water extraction-type yeast cell wall-derived decomposition product-containing composition (3E) | 72.04 | 6.21 | 10.11 |
| Preparation Example 4-4 | Hot-water extraction-type yeast cell wall-derived decomposition product-containing composition (4E) | 76.14 | 5.25 | 9.98 |
| Preparation Example 6-1 | Hot-water extraction-type yeast cell wall-derived decomposition product-containing composition (1M) | 22.42 | 14.35 | 16.01 |
| Preparation Example 6-2 | Hot-water extraction-type yeast cell wall-derived decomposition product-containing composition (2M) | 29.11 | 10.87 | 15.59 |

(continued)

| Test samples | | Color tone | | |
|---|---|---|---|---|
| | | L* value | a* value | b* value |
| Preparation Example 6-3 | Hot-water extraction-type yeast cell wall-derived decomposition product-containing composition (3M) | 19.92 | 15.34 | 18.17 |
| Preparation Example 6-4 | Hot-water extraction-type yeast cell wall-derived decomposition product-containing composition (4M) | 22.14 | 15.24 | 15.87 |

It was found by the heat treating step, the a* value and the b* value (chromaticity) did not greatly change, but the L* value (lightness) greatly decreased to be browned (blackened). This suggests that a high extent of the Maillard reaction was occurring.

<Test Example 3: Roast fragrance>

The products obtained in the Preparation Examples presented in Tables 5-1 and 5-2 below were used as test samples and measured for roast fragrance in the following manner.

Five specialized panelists evaluated the extent of roast fragrance of each of the test samples based on the following evaluation criteria, with the fragrance of the yeast cell wall slurry used as a raw material in each Preparation Example being a control (0 points) (i.e., for the product obtained using the autolysis-type yeast cell wall as a raw material, the fragrance of the autolysis-type yeast cell wall slurry was defined as a control, while for the product obtained using the hot-water extraction-type yeast cell wall as a raw material, the fragrance of the hot-water extraction-type yeast cell wall slurry was defined as a control). The evaluation results are presented as an average point obtained by five specialized panelists according to the evaluation criteria. The results are presented in Tables 5-1 and 5-2 below.

[Evaluation criteria]

-3 points: Very weak roast fragrance
-2 points: Weak roast fragrance
-1 points: Slightly weak roast fragrance
0 points: Control
+1 point: Slightly strong roast fragrance
+2 points: Strong roast fragrance
+3 points: Very strong roast fragrance

Table 5-1

| Test samples | | Roast fragrance |
|---|---|---|
| Preparation Example 1-1 | Autolysis-type yeast cell wall slurry | 0 |
| Preparation Example 1-2 | Autoclave-treated product (1) of the autolysis-type yeast cell wall | 0 |
| Preparation Example 1-3 | Autoclave-treated product (2) of the autolysis-type yeast cell wall | 0 |
| Preparation Example 3-1 | Autolysis-type yeast cell wall-derived decomposition product-containing composition (1E) | 0 |
| Preparation Example 3-2 | Autolysis-type yeast cell wall-derived decomposition product-containing composition (2E) | 0 |

(continued)

| Test samples | | Roast fragrance |
|---|---|---|
| Preparation Example 3-3 | Autolysis-type yeast cell wall-derived decomposition product-containing composition (3E) | 0 |
| Preparation Example 3-4 | Autolysis-type yeast cell wall-derived decomposition product-containing composition (4E) | 0 |
| Preparation Example 5-1 | Autolysis-type yeast cell wall-derived decomposition product-containing composition (1M) | + 2.8 |
| Preparation Example 5-2 | Autolysis-type yeast cell wall-derived decomposition product-containing composition (2M) | + 2.2 |
| Preparation Example 5-3 | Autolysis-type yeast cell wall-derived decomposition product-containing composition (3M) | +3.0 |
| Preparation Example 5-4 | Autolysis-type yeast cell wall-derived decomposition product-containing composition (4M) | + 2.8 |

Table 5-2

| Test samples | | Roast fragrance |
|---|---|---|
| Test Example 2-1 | Hot-water extraction-type yeast cell wall slurry | 0 |
| Test Example 2-2 | Autoclave-treated product (1) of the hot-water extraction-type yeast cell wall | 0 |
| Test Example 2-3 | Autoclave-treated product (2) of the hot-water extraction-type yeast cell wall | 0 |
| Preparation Example 4-1 | Hot-water extraction-type yeast cell wall-derived decomposition product-containing composition (1E) | 0 |
| Preparation Example 4-2 | Hot-water extraction-type yeast cell wall-derived decomposition product-containing composition (2E) | 0 |
| Preparation Example 4-3 | Hot-water extraction-type yeast cell wall-derived decomposition product-containing composition (3E) | 0 |
| Preparation Example 4-4 | Hot-water extraction-type yeast cell wall-derived decomposition product-containing composition (4E) | 0 |
| Preparation Example 6-1 | Hot-water extraction-type yeast cell wall-derived decomposition product-containing composition (1M) | +2.6 |
| Preparation Example 6-2 | Hot-water extraction-type yeast cell wall-derived decomposition product-containing composition (2M) | +2.4 |
| Preparation Example 6-3 | Hot-water extraction-type yeast cell wall-derived decomposition product-containing composition (3M) | + 2.8 |
| Preparation Example 6-4 | Hot-water extraction-type yeast cell wall-derived decomposition product-containing composition (4M) | +2.6 |

It was found that any change in roast fragrance was not perceived only by subjecting the yeast cell wall to the autoclave treatment or the enzymatically treating step, and roast fragrance was obtained by performing the heat treatment after the enzymatically treating step. There was a tendency that the stronger the roast fragrance was, the more the treated product was meat flavor-like.

From the results of Test Examples 2 and 3, the autolysis-type yeast cell wall-derived decomposition product-containing compositions (1M) to (4M) and the hot-water extraction-type yeast cell wall-derived decomposition product-containing compositions (1M) to (4M) were presumed to be the Maillard reaction products.

<Test Example 4: Beer-like fragrance>

The products obtained in the Preparation Examples presented in Table 6 below were used as test samples and measured for beer-like fragrance in the following manner.

Five specialized panelists evaluated the extent of beer-like fragrance of each of the test samples based on the following evaluation criteria, with the fragrance of the yeast cell wall slurry used as a raw material in each Preparation Example being a control (0 points) (i.e., for the product obtained using the autolysis-type yeast cell wall as a raw material, the fragrance of the autolysis-type yeast cell wall slurry was defined as a control, while for the product obtained using the hot-water extraction-type yeast cell wall as a raw material, the fragrance of the hot-water extraction-type yeast cell wall slurry was defined as a control). The evaluation results are presented as an average point obtained by five specialized panelists according to the evaluation criteria. The results are presented in Table 6 below.

[Evaluation criteria]

-3 points: Very weak beer-like fragrance
-2 points: Weak beer-like fragrance
-1 points: Slightly weak beer-like fragrance
0 points: Control
+1 point: Slightly strong beer-like fragrance
+2 points: Strong beer-like fragrance
+3 points: Very strong beer-like fragrance

Table 6

| Test samples | | Beer-like fragrance |
|---|---|---|
| Preparation Example 1-1 | Autolysis-type yeast cell wall slurry (control) | 0 |
| Preparation Example 3-1 | Autolysis-type yeast cell wall-derived decomposition product-containing composition (1E) | +2.4 |
| Preparation Example 3-4 | Autolysis-type yeast cell wall-derived decomposition product-containing composition (4E) | +2.8 |
| Preparation Example 5-1 | Autolysis-type yeast cell wall-derived decomposition product-containing composition (1M) | -0.2 |
| Preparation Example 5-2 | Autolysis-type yeast cell wall-derived decomposition product-containing composition (2M) | -0.2 |
| Preparation Example 5-3 | Autolysis-type yeast cell wall-derived decomposition product-containing composition (3M) | -0.4 |
| Preparation Example 2-1 | Hot-water extraction-type yeast cell wall slurry (control) | 0 |
| Preparation Example 4-1 | Hot-water extraction-type yeast cell wall-derived decomposition product-containing composition (1E) | 0 |
| Preparation Example 6-1 | Hot-water extraction-type yeast cell wall-derived decomposition product-containing composition (1M) | 0 |
| Preparation Example 6-2 | Hot-water extraction-type yeast cell wall-derived decomposition product-containing composition (2M) | 0 |

The beer yeast cell wall slurry had such unique fragrance as rotten odor derived from microorganisms, and off-flavor such as other rotten odors and hop's oxidation-related odor. In contrast, by enzymatically treating the beer yeast cell wall slurry in the enzymatically treating step, the resultant product had reduced off-flavor and also had the unexpected effect of coming to have strong beer-like fragrance. The autolysis-type yeast cell wall-derived decomposition product-containing composition (1E) (Preparation Example 3-1) had complex, thick beer-like fragrance. The autolysis-type yeast cell wall-derived decomposition product-containing composition (4E) (Preparation Example 3-4) had sharp beer-like fragrance and was found to be able to impart superior beer-like fragrance. Meanwhile, when the baker's yeast cell wall was used as a raw material, any beer-like fragrance was not perceived. Also, by subjecting the enzymatically treated product of the beer yeast cell wall to the heat treatment, the beer-like fragrance became weaker, and weak green fragrance and strong roast fragrance were perceived.

Conventionally, the beer yeast cell wall could not be used alone as a raw material of flavor due to the presence of the above off-flavor and the absence of any substance derived from brewing and fermenting. However, it is considered that when the beer yeast cell wall is processed to have a lower molecular weight for solubilization, and further into monomers to dissolve by virtue of an increase in solubility ratio, flavor ingredients derived from brewing and fermenting elute that are enclosed in the cell wall at a molecular level.

As presented in Test Example 1, although part of the insoluble matter in the yeast cell wall remains even after the enzymatically treating step, a dispersed state close to a solubilized sate is established where there are a small amount of precipitates, differing from the conventional cases. It is presumed that these flavors are obtained only in this state.

<Test Example 5: Reducing monosaccharide content ratio and total free amino acid content ratio>

The products obtained in the Preparation Examples presented in Table 7 below were used as test samples and measured for the content ratio of glucose as the reducing saccharide and the content ratio of the total free amino acid contained in each test sample. The results are presented in Table 7 below.

Regarding the products obtained in the Preparation Examples presented in Table 8 below, the result of each of the content ratios of the total free amino acid is presented in Table 8 below.

-Measurement of solid content concentration in the test sample-

The mass (solid content mass) of a dry product obtained by drying the test sample at 105°C for 5 hours was measured to calculate a solid content concentration (T) in the test sample based on the following Formula (3).

$$\text{Solid content concentration (T) (\% by mass)} = \text{solid content mass/mass of test sample} \times 100 \ldots \text{Formula (3)}$$

-Measurement of content ratio of glucose-

The concentration (G) of glucose in the test sample was measured by an enzyme-catalyzed reaction and a hydrogen peroxide electrode detection method using a multi-functional biosensor (BF-7D, product of Oji Scientific Instruments). The measurement was performed using a glucose electrode and a sucrose electrode as electrodes, and a dedicated buffer solution at 30°C.

"Glucose content ratio" in Table 7 below refers to a content ratio of glucose relative to the solid content mass of the test sample, the content ratio being calculated based on Formula (4) below from the measured glucose concentration (G) and the solid content ratio (T) in the test sample.

$$\text{Glucose content ratio (\% by mass)} = \text{glucose concentration (G)/solid content concentration (T)} \ldots \text{Formula (4)}$$

-Measurement of content ratio of total free amino acid-

The free amino acids in the test sample were derivatized according to the product protocol of AccQ-Tag Ultra Derivatization kit (product of Waters, Co.). The concentration (F) of each free amino acid in the test sample containing the derivatized free amino acids was measured by ultra high performance liquid chromatography under the following measurement conditions.

"Free amino acid content ratio" in Table 8 below refers to a content ratio of each free amino acid relative to the solid content mass of the test sample, the content ratio being calculated based on Formula (5) below from the measured free amino acid concentration (F) and the solid content concentration (T) in the test sample.

Also, "Total free amino acid content ratio" in Table 7 below refers to a total value of the content ratios of the free amino acids relative to the solid content mass of the test sample, the total value thereof being calculated based on Formula (5) below from the measured free amino acid concentration (F) and the solid content concentration (T) in the test sample.

$$\text{Free amino acid content ratio (\% by mass)} = \text{free amino acid concentration}$$

$$\text{(F)/solid content concentration (T)} \dots \text{Formula (5)}$$

[Measurement conditions of free amino acids]

- Analyzer: Ultra Performance LC (UPLC (registered trademark)) (product of Waters, Co.)
- Detector: Photodiode array (PDA) (product of Waters, Co.)
- Column: AccQ-Tag Ultra RP Column (130 angstroms, 1.7 $\mu$m, 2.1 mm $\times$ 100 mm, product of Waters, Co.)
- Column temperature: 55°C
- Sample temperature: 10°C
- Mobile phase: AccQ-Tag Ultra Eluent A (product of Waters, Co.) as Liquid A and AccQ-Tag Ultra Eluent B (product of Waters, Co.) as Liquid B

Table 7

| Test samples | | Glucose content ratio (% by mass) | Total free amino acid content ratio (% by mass) |
|---|---|---|---|
| Preparation Example 1-2 | Autoclave-treated product (1) of the autolysis-type yeast cell wall | 0.19 | 7.20 |
| Preparation Example 3-1 | Autolysis-type yeast cell wall-derived decomposition product-containing composition (1E) | 25.50 | 14.90 |
| Preparation Example 3-2 | Autolysis-type yeast cell wall-derived decomposition product-containing composition (2E) | 24.90 | 7.30 |
| Preparation Example 3-3 | Autolysis-type yeast cell wall-derived decomposition product-containing composition (3E) | 19.20 | 12.30 |
| Preparation Example 3-4 | Autolysis-type yeast cell wall-derived decomposition product-containing composition (4E) | 25.00 | 7.20 |
| Preparation Example 5-1 | Autolysis-type yeast cell wall-derived decomposition product-containing composition (1M) | 8.80 | 7.70 |
| Test Example 2-2 | Autoclave-treated product (1) of the hot-water extraction-type yeast cell wall | 0.12 | 7.70 |
| Preparation Example 4-1 | Hot-water extraction-type yeast cell wall-derived decomposition product-containing composition (1E) | 13.80 | 15.90 |
| Preparation Example 4-2 | Hot-water extraction-type yeast cell wall-derived decomposition product-containing composition (2E) | 13.57 | 7.90 |

(continued)

| Test samples | | Glucose content ratio (% by mass) | Total free amino acid content ratio (% by mass) |
|---|---|---|---|
| Preparation Example 4-3 | Hot-water extraction-type yeast cell wall-derived decomposition product-containing composition (3E) | 10.91 | 12.60 |
| Preparation Example 6-1 | Hot-water extraction-type yeast cell wall-derived decomposition product-containing composition (1M) | 0.76 | 10.40 |

Table 8

| Amino acids | Free amino acid content ratio (% by mass) | | | |
|---|---|---|---|---|
| | Preparation Example 3-1 | Preparation Example 5-1 | Preparation Example 4-1 | Preparation Example 6-1 |
| | Autolysis-type yeast cell wall-derived decomposition product-containing composition (1E) | Autolysis-type yeast cell wall-derived decomposition product-containing composition (1M) | Hot-water extraction-type yeast cell wall-derived decomposition product-containing composition (1E) | Hot-water extraction-type yeast cell wall-derived decomposition product-containing composition (1M) |
| His | 0.36 | 0.06 | 0.35 | 0.13 |
| Asn | 0.75 | 0.39 | 0.64 | 0.38 |
| Ser | 0.80 | 0.48 | 0.81 | 0.55 |
| Gln | 0.30 | 0.02 | 0.02 | 0.00 |
| Arg | 0.87 | 0.10 | 1.16 | 0.48 |
| Gly | 0.40 | 0.20 | 0.28 | 0.17 |
| Asp | 1.01 | 0.63 | 0.69 | 0.54 |
| Glu | 1.12 | 0.61 | 1.56 | 1.06 |
| Thr | 0.79 | 0.41 | 0.98 | 0.62 |
| Ala | 1.04 | 0.68 | 0.87 | 0.68 |
| Pro | 0.24 | 0.21 | 0.23 | 0.18 |
| Cys | 0.12 | 0.03 | 0.18 | 0.07 |
| Lys | 1.48 | 0.34 | 1.73 | 1.02 |
| Tyr | 0.68 | 0.45 | 0.87 | 0.61 |
| Met | 0.35 | 0.14 | 0.03 | 0.01 |
| Val | 1.03 | 0.66 | 1.24 | 0.89 |
| Ile | 0.89 | 0.59 | 1.01 | 0.73 |
| Leu | 1.56 | 0.99 | 2.05 | 1.44 |
| Phe | 0.86 | 0.57 | 1.10 | 0.73 |
| Trp | 0.25 | 0.14 | 0.09 | 0.10 |

It was found that by the enzymatic treatment using the exoglucanase alone or the exoglucanase and the other enzymes at the same time, the content ratio of glucose increased. Thus, it is presumed that the solubility ratio of the yeast cell wall increased by the enzymatic treatment as presented in Test Example 1 because the yeast cell wall was decomposed by the enzymatic treatment.

Meanwhile, the content ratio of the total free amino acid did not increase by use of the exoglucanase alone, but did increase by use of the enzymatic treatment using the exoglucanase and the other enzymes at the same time.

In the case of performing the enzymatic treatment with the exoglucanase after the enzymatic treatment with the protease under optimum conditions at the two separate steps (Preparation Example 3-4), the decomposition of the proteins by the protease was followed by the decomposition of the sugar by the glucanase. Thus, it is expected that the content ratios of glucose and the total free amino acid would further increase. However, the content ratio of glucose was lower than in the case of the enzymatic treatment using the protease and the exoglucanase at the same time. Also, the content ratio of the total free amino acid was slightly higher than in the case of the enzymatic treatment using the protease and the exoglucanase at the same time, but was lower than in the case of the enzymatic treatment using the exoglucanase, the protease, and the exopeptidase at the same time.

Further, after the heat treatment, both the content ratio of glucose and the content ratio of the total free amino acid greatly decreased. In particular, the content ratio of the total free amino acid decreased to the same extent as the autoclave-treated product (1) of the autolysis-type yeast cell wall and the autoclave-treated product (1) of the hot-water extraction-type yeast cell wall before the enzymatic treatment. It is therefore presumed that the autolysis-type yeast cell wall-derived decomposition product-containing composition (1M) and the hot-water extraction-type yeast cell wall-derived decomposition product-containing composition (1M) are the Maillard reaction products.

<Test Example 6: Fat and oil content ratio of the yeast cell wall-derived decomposition product-containing composition>

Products obtained in Preparation Examples presented in Table 9 below, an autolysis-type yeast extract (S-Ps, product of Asahi Group Foods, Ltd.), and a hot-water extraction-type yeast extract (HG-Ps, product of Asahi Group Foods, Ltd.) were used as test samples. The present inventor asked the Japan Food Research Laboratories to analyze the content ratio of fat and oil contained in these test samples. The reported values are presented in Table 9 below.

Table 9

| Test samples | | Fat and oil content ratio (% by mass) |
|---|---|---|
| Preparation Example 5-1 | Autolysis-type yeast cell wall-derived decomposition product-containing composition (1M) | 8.80 |
| Preparation Example 6-1 | Hot-water extraction-type yeast cell wall-derived decomposition product-containing composition (1M) | 7.20 |
| | Autolysis-type yeast extract | 0.40 |
| | Hot-water extraction-type yeast extract | 0.20 |

<Test Example 7-1: Fragrance ingredients 1 of yeast cell wall-derived decomposition product-containing composition>

A yeast cell wall (autolysis-type yeast cell wall) (product name: yeast cell wall, product of Asahi Group Foods, Ltd.), the autolysis-type yeast cell wall-derived decomposition product-containing composition (1E) obtained in Preparation Example 3-1, and the autolysis-type yeast cell wall-derived decomposition product-containing composition (1M) obtained in Preparation Example 5-1 were used as test samples. Each of the test samples was analyzed for fragrance ingredients by solid-phase micro extraction (SPME)-gas chromatography mass spectrometry under the following analysis conditions.

[Analysis conditions]

--Pre-treatment conditions of test samples--

The autolysis-type yeast cell wall-derived decomposition product-containing composition (1E) and the autolysis-type yeast cell wall-derived decomposition product-containing composition (1M) were spray dried to prepare powdered yeast cell wall-derived decomposition product-containing compositions, which were used as test samples.

--Solid-phase micro extraction conditions--

- Solid-phase micro extraction (SPME) fiber: PDMS/DVB (film thickness: 65 $\mu$m, coating phase: polydimethylsiloxane dispersed in divinylbenzene, product of SUPELCO, Inc.)
- Volatile ingredients extraction device: multi-functional auto-sampler for MPS GC-MS (product of GERSTEL K.K.)
- Extraction conditions: 2.0 g of the test sample was weighed in a 20 mL-volume vial for SPME, followed by extracting volatile ingredients at 60°C for 30 minutes, to allow the volatile ingredients to adsorb on the SPME fiber.

--Gas chromatography mass spectrometry conditions--

- Measuring apparatus: Agilent6890GC-5975MSD (product of Agilent Technologies, Ltd.)
- Column: DB-WAX (60 m × 0.250 mm ID × 0.25 $\mu$m F.T, product of Agilent Technologies, Ltd.)
- Temperature conditions: retained at 38°C (for 10 minutes) → heated to 230°C at 3°C/min → retained at 230°C (for 20 minutes) → heated to 245°C at 5°C/min → retained at 245°C (for 6 minutes)
- Carrier: He gas, gas flow rate 1.0 mL/min
- Injection inlet: split/splitless injection inlet
- Injection inlet temperature: 230°C
- Injection amount: 1 $\mu$L
- Injection method: pulsed splitless (pulse pressure 250 KPa, up to 1.01 m)
- Split ratio: 5:1
- Liner: Straight, SPME taper, Ultra Inert liner
- Transfer line temperature: 230°C
- Ionization mode: EI (ionization voltage 70 eV)
- Type of ion source: extractor ion source
- Ion source temperature: 230°C
- Quadrupolar temperature: 150°C
- Scan mode: Scan
- Scan mass: m/z 29.0 to 550.0

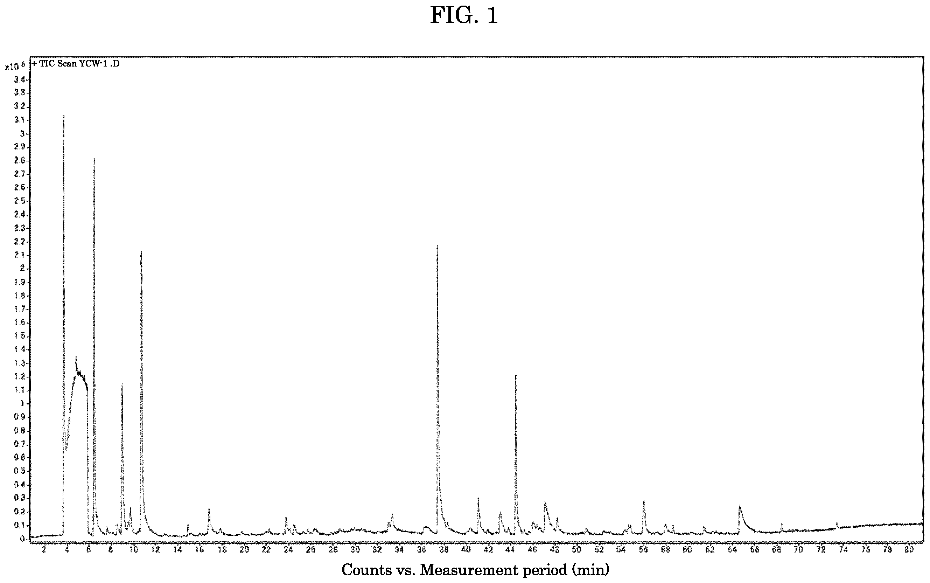

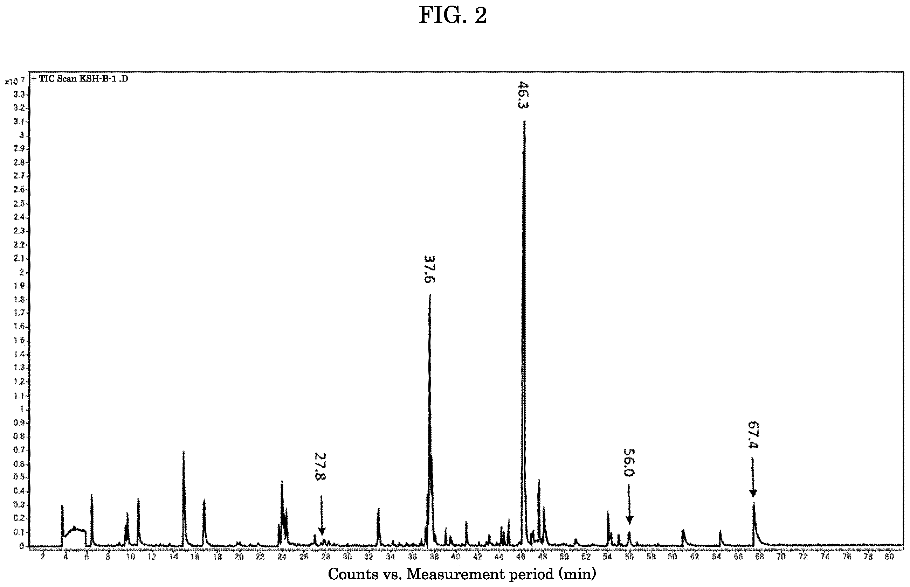

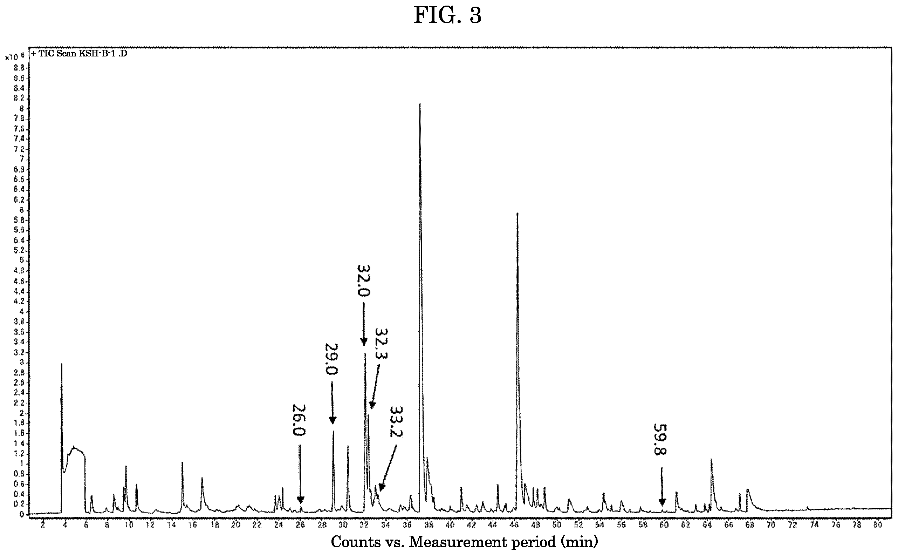

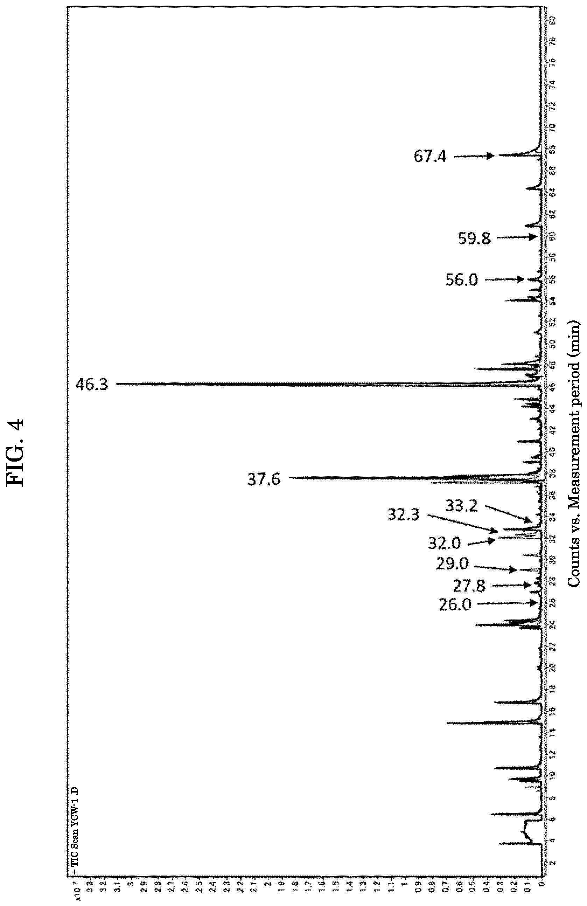

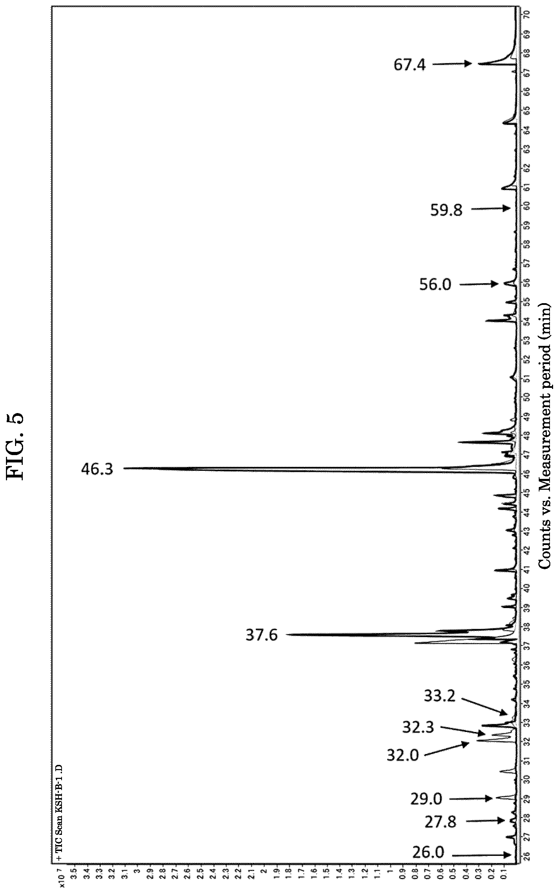

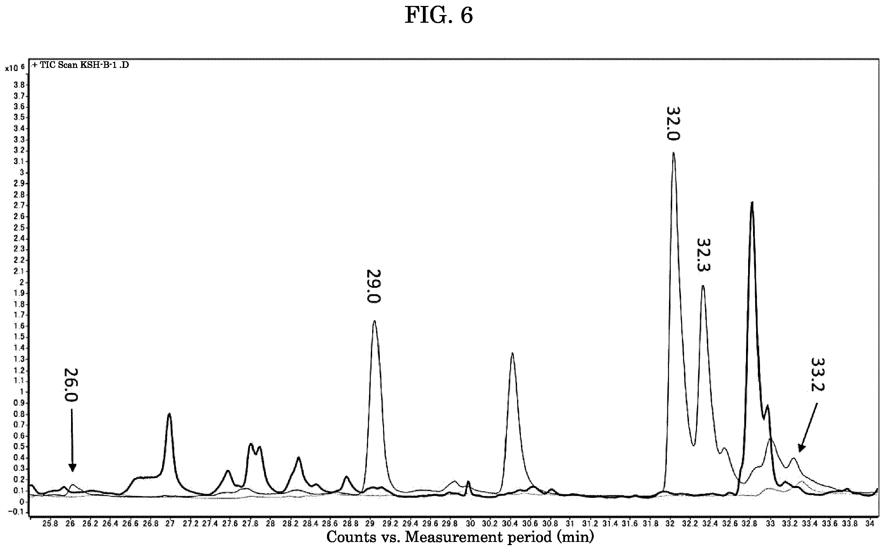

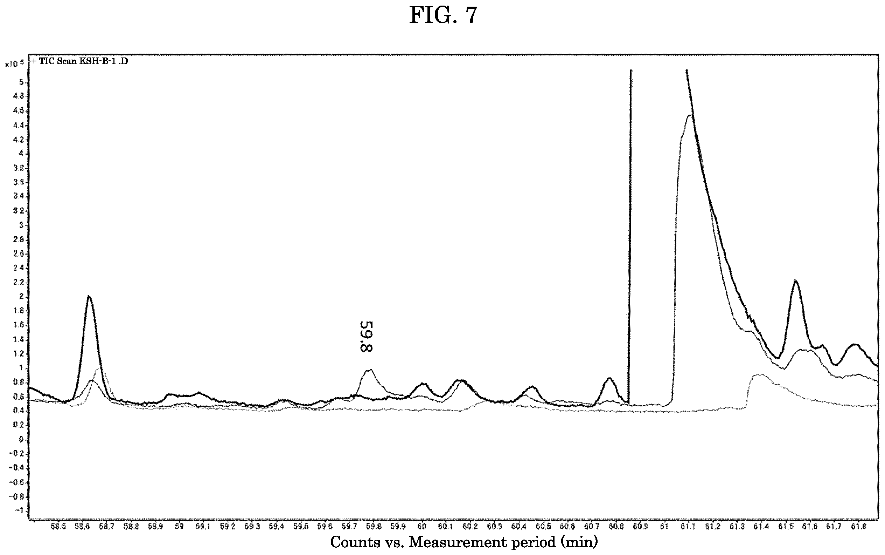

The results of the solid-phase micro extraction (SPME)-gas chromatography mass spectrometry of the test samples are presented in FIG. 1 to FIG. 7.

In the autolysis-type yeast cell wall-derived decomposition product-containing composition (1E), new peaks, which were not confirmed in the autolysis-type yeast cell wall (untreated product), were confirmed, the new peaks representing ethyl caproate (Hexanoic acid, ethyl ester), ethyl caprylate (Octanoic acid, ethyl ester), ethyl caprate (Decanoic acid, ethyl ester), phenylethyl alcohol (Phenylethyl Alcohol), and capric acid (n-Decanoic acid) (see FIG. 1, FIG. 2, and FIG. 4).

In the autolysis-type yeast cell wall-derived decomposition product-containing composition (1M), new peaks, which were not confirmed in the autolysis-type yeast cell wall (untreated product) and the autolysis-type yeast cell wall-derived decomposition product-containing composition (1E), were confirmed, the new peaks representing pyrazine (Pyrazine), methyl pyrazine (Methyl Pyrazine), 2,5-dimethyl pyrazine (2,5-Dimethyl Pyrazine), 2,6-dimethyl pyrazine (2,6-Dimethyl Pyrazine), 2,3-dimethyl pyrazine (2,3-Dimethyl Pyrazine), and furaneol (Furaneol) (see FIG. 1 to FIG. 7).

The retention times, peak heights, and peak areas of the peaks detected in each test sample are presented in Table 10-1 below.

Table 10-1

| Test samples | | Detected ingredients | Retention time (min) | Peak area |
|---|---|---|---|---|
| Preparation Example 3-1 | Autolysis-type yeast cell wall-derived decomposition product-containing composition (1E) | Ethyl caproate | 27.8 | 2489129 |
| | | Ethyl caprylate | 37.6 | 149855505 |
| | | Ethyl caprate | 46.3 | 353173994 |
| | | Phenylethyl alcohol | 56.0 | 10598725 |
| | | Capric acid | 67.4 | 45982413 |
| Preparation Example 5-1 | Autolysis-type yeast cell wall-derived decomposition product-containing composition (1M) | Pyrazine | 26.0 | 783576 |
| | | Methyl pyrazine | 29.0 | 13029499 |
| | | 2,5-Dimethyl pyrazine | 32.0 | 25537954 |
| | | 2,6-Dimethyl pyrazine | 32.3 | 14247415 |
| | | 2,3-Dimethyl pyrazine | 33.2 | 2520188 |
| | | Furaneol | 59.8 | 393837 |

The peaks of the compounds described for the autolysis-type yeast cell wall-derived decomposition product-containing composition (1E) in Table 10-1 are peaks that were not detected in the case of measuring the beer yeast cell wall (autolysis-type yeast cell wall) as a raw material under the same conditions. Thus, these are presumed to be peaks of fragrance ingredients responsible for beer-like fragrance.

Also, the peaks of the compounds described for the autolysis-type yeast cell wall-derived decomposition product-containing composition (1M) in Table 10-1 are peaks that were not detected in the case of measuring, under the same conditions, the beer yeast cell wall (autolysis-type yeast cell wall) as a raw material and the autolysis-type yeast cell wall-derived decomposition product-containing composition (1E) obtained by enzymatically treating it. Thus, these are presumed to be peaks of fragrance ingredients responsible for roast flavor.

<Test Example 7-2: Fragrance ingredients 2 of yeast cell wall-derived decomposition product-containing composition>

The autolysis-type yeast cell wall-derived decomposition product-containing composition (1E) obtained in Preparation Example 3-1 and the autolysis-type yeast cell wall-derived decomposition product-containing composition (4E) obtained in Preparation Example 3-4 were used as test samples. Each of the test samples was analyzed for fragrance ingredients by solid-phase micro extraction (SPME)-gas chromatography mass spectrometry under the following analysis conditions.

[Analysis conditions]

--Pre-treatment conditions of test samples--

The autolysis-type yeast cell wall-derived decomposition product-containing composition (1E) and the autolysis-type yeast cell wall-derived decomposition product-containing composition (4E) were spray dried to prepare powdered yeast cell wall-derived decomposition product-containing compositions, which were used as test samples.

--Solid-phase micro extraction conditions--

- Solid-phase micro extraction (SPME) fiber: PDMS/DVB (film thickness: 65 $\mu$m, coating phase: polydimethylsiloxane dispersed in divinylbenzene, product of SUPELCO, Inc.)
- Volatile ingredients extraction device: multi-functional auto-sampler for MPS GC-MS (product of GERSTEL K.K.)
- Extraction conditions: 2.0 g of the test sample was weighed in a 20 mL-volume vial for SPME, followed by extracting volatile ingredients at 60°C for 30 minutes, to allow the volatile ingredients to adsorb on the SPME fiber.

--Gas chromatography mass spectrometry conditions--

- Measuring apparatus: Agilent8890B-5977B (Single quadrupole) (product of Agilent Technologies, Ltd.)
- Column: DB-WAX UI (30 m $\times$ 0.250 mm ID $\times$ 0.25 $\mu$m F.T, product of Agilent Technologies, Ltd.)
- Temperature conditions: retained at 40°C (for 3 minutes) $\rightarrow$ heated to 250°C at 10°C/min $\rightarrow$ retained at 250°C (for 6 minutes)
- Carrier: He gas, gas flow rate 1.1 mL/min
- Injection inlet: split/splitless injection inlet
- Injection inlet temperature: 250°C
- Injection amount: 1 $\mu$L
- Injection method: pulsed splitless (pulse pressure 20 psi, for 3 minutes)
- Split ratio: 5:1
- Liner: Straight, SPME taper, Ultra Inert liner
- Transfer line temperature: 230°C
- Ionization mode: EI (ionization voltage 70 eV)
- Type of ion source: extractor ion source
- Ion source temperature: 250°C
- Quadrupolar temperature: 150°C
- Scan mode: Scan
- Scan mass: m/z 29.0 to 550.0

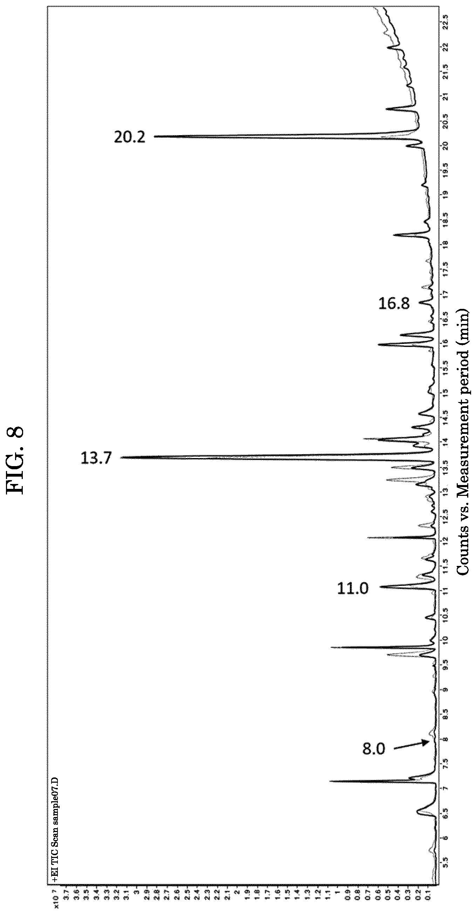

The results of the solid-phase micro extraction (SPME)-gas chromatography mass spectrometry of the test samples are presented in FIG. 8.

The retention time, peak height, and peak area detected in each of the test samples are presented in Table 10-2 below. FIG. 9 is a graph of the peak areas presented in Table 10-2 below.

Table 10-2

| Test samples | | Detected ingredients | Retention time (min) | Peak area |
|---|---|---|---|---|
| Preparation Example 3-1 | Autolysis-type yeast cell wall-derived decomposition product-containing composition (1E) | Ethyl caproate | 8.0 | 5392 |
| | | Ethyl caprylate | 11.1 | 1597774 |
| | | Ethyl caprate | 13.7 | 6043190 |
| | | Phenylethyl alcohol | 16.8 | 1349524 |
| | | Capric acid | 20.2 | 1659465 |

(continued)

| Test samples | | Detected ingredients | Retention time (min) | Peak area |
|---|---|---|---|---|
| Preparation Example 3-4 | Autolysis-type yeast cell wall-derived decomposition product-containing composition (4E) | Ethyl caproate | 8.0 | 170458 |
| | | Ethyl caprylate | 11.1 | 5337014 |
| | | Ethyl caprate | 13.7 | 9398269 |
| | | Phenylethyl alcohol | 16.8 | 2023356 |
| | | Capric acid | 20.2 | 13886483 |

The autolysis-type yeast cell wall-derived decomposition product-containing composition (4E), which had been treated with one kind of an enzyme, was found to have increased fragrance ingredients giving off beer-like fragrance, as compared with the autolysis-type yeast cell wall-derived decomposition product-containing composition (1E), which had been treated with three kinds of enzymes.

<Test Example 8: Particle size distribution of insoluble fraction of the yeast cell wall-derived decomposition product-containing composition>

The autolysis-type yeast cell wall slurry obtained in Preparation Example 1-1 and the autolysis-type yeast cell wall-derived decomposition product-containing composition (1E) obtained in Preparation Example 3-1 were used as test samples. In the following manners, the insoluble fraction in each of the test samples was measured for the particle size distribution (d10, d50, and d90), mean volume particle diameter (MV), mean number particle diameter (MN), mean area particle diameter (MA), and standard deviation (SD; an indicator for the distribution width of the measured particle size distribution). The results are presented in Table 11 and FIG. 10 below.

[Measurement conditions of particle size distribution]

•    Measuring device: MICROTRAC particle size distribution analyzer (MT3300EX, product of Nikkiso Co., Ltd.)

•    Wet mode: using water as a solvent

•    Transmittance: transmit

•    Refractive index: 1.333

•    Measurement period: 10 seconds

•    Measurement times: 3 times

•    Mode: Laser diffraction scattering method

Table 11

| Test samples | | d10 (μm) | d50 (μm) | d90 (μm) | MV (μm) | MN (μm) | MA (μm) | SD (μm) |
|---|---|---|---|---|---|---|---|---|
| Preparation Example 1-1 | Autolysis-type yeast cell wall slurry | 5.199 | 8.765 | 17.38 | 10.251 | 6.1305 | 8.3098 | 4.6586 |

(continued)

| Test samples | | d10 (μm) | d50 (μm) | d90 (μm) | MV (μm) | MN (μm) | MA (μm) | SD (μm) |
|---|---|---|---|---|---|---|---|---|
| Preparation Example 3-1 | Autolysis-type yeast cell wall-derived decomposition product-containing composition (1E) | 1.757 | 2.419 | 3.379 | 2.5161 | 2.0495 | 2.348 | 0.6128 |

<Test Example 9: Dispersion stability of the yeast cell wall-derived decomposition product-containing composition>

The autoclave-treated product (1) of the autolysis-type yeast cell wall obtained in Preparation Example 1-2, the autolysis-type yeast cell wall-derived decomposition product-containing composition (1E) obtained in Preparation Example 3-1, and the autolysis-type yeast cell wall-derived decomposition product-containing composition (4E) were used as test samples and evaluated for dispersion stability in the following manner. The results are presented in Table 12 below.

The solid content concentration of each of the test samples was calculated by the method described in Test Example 5, and then adjusted to 10% by mass.

A 100 mL-volume glass measuring cylinder (product of SHIBATA SCIENTIFIC TECHNOLOGIES, LTD.) was charged with 100 mL of the test sample having a solid content concentration of 10% by mass, and was left to stand still for 48 hours under normal pressure. Then, the volume of the precipitates was measured by reading the scale of the measuring cylinder.

Table 12

| Test samples | | Volume of precipitates (mL) |
|---|---|---|
| Preparation Example 1-2 | Autoclave-treated product (1) of the autolysis-type yeast cell wall | 78 |
| Preparation Example 3-1 | Autolysis-type yeast cell wall-derived decomposition product-containing composition (1E) | 12 |
| Preparation Example 3-4 | Autolysis-type yeast cell wall-derived decomposition product-containing composition (4E) | 11 |

It was found that the enzymatic treatment using the exoglucanase alone or using the exoglucanase and other enzymes at the same time increased not only in the solubility ratio of the yeast cell wall but also in the dispersion stability of the solid content.

<Test Example 10: Palatability test 1 for dog>

-Test Product I-

The autolysis-type yeast cell wall-derived decomposition product-containing composition (4M) having a roast fragrance, which had been prepared in Preparation Example 5-4, was sprinkled in an amount of 0.3% by mass onto dog food for animal experiments (free of a palatability enhancer, product of KITAYAMA LABES CO. LTD.), followed by homogenously mixing, to prepare pet food (hereinafter may be referred to as "Test Product I").

-Control 1-

Dog food for animal experiments (free of a palatability enhancer, product of KITAYAMA LABES CO. LTD) was used as "Control 1".

-2-Point Comparison Test-

By the 2-point comparison test below, Test Product I and Control 1 were used to test an average first choice ratio and an average intake ratio. In order to avoid occurrence of statistical difference, this 2-point comparison test was performed in the following manner with the positions; i.e., the left-hand position and the right-hand position of Test Product I and Control 1 being exchanged between the first test and the second test. Beagle dogs used in the 2-point comparison test below are dogs that underwent identification training for a palatability test and passed it, and regularly received training.

[First Test]

24 hours after the last feeding of Control 1, the first test was performed.

In the first test, the feeding amount (FA) of each of Test Product I and Control 1 was set to 250 g, and they were put in separate containers. Test Product I was set on the left-hand side and Control 1 was set on the right-hand side. Then, they were fed to beagle dogs (n=20; 2 to 11 years after birth: a mixed group of females (F), males (M), or castrated ones (XM); KITAYAMA LABES CO. LTD.). The dogs were allowed to drink water ad libitum.

With the product that they first put in their mouth (Test Product I or Control 1) being first choice (FC), each of the beagle dogs was confirmed as to which they chose first, Test Product I or Control 1. In Table 13-1, "○" indicates the product that was first chosen. In the first test, a first choice ratio was calculated from the number of the dogs that first chose Test Product I or Control 1, out of the total 20 dogs (hereinafter may be referred to as "FC1"). The results are presented in Table 13-1.

Next, 20 minutes after the start of feeding, for each of the beagle dogs, the first feeding intake (FI) (hereinafter may be referred to as "FI1") from the residual amounts of Test Product I and Control 1 after eating (hereinafter may be referred to as "residual feed amounts") (g). The feeding intake of Test Product I and the feeding intake of Control 1 were calculated as the first feeding intake. The results are presented in Table 13-2.

[Second Test]

24 hours after the first feeding, the second test was performed. In the second test, the feeding amount (FA) of each of Test Product I and Control 1 was set to 250 g, and they were put in separate containers. Control 1 was set on the left-hand side and Test Product I was set on the right-hand side. Then, they were fed to each of the same beagle dogs as in the first test. The dogs were allowed to drink water ad libitum.

Similar to the first test, each of the beagle dogs was confirmed as to which they chose first, Test Product I or Control 1. In Table 13-1, "○" indicates the product that was first chosen. In the second test, a first choice ratio was calculated from the number of the dogs that first chose Test Product I or Control 1, out of the total 20 dogs (hereinafter may be referred to as "FC2"). The results are presented in Table 13-1.

Next, similar to the first test, 20 minutes after the start of feeding, for each of the beagle dogs, the second feeding intake (FI) (hereinafter may be referred to as "FI2") from the residual feed amounts (g) of Test Product I and Control 1. The results are presented in Table 13-3.

Next, from Formula (6) below, an average first choice ratio (average FC ratio) (%) of Test Product I or Control 1 was calculated. The results are presented in Table 13-5 below.

$$\text{Average FC ratio (\%)} = (FC1 + FC2)/2 \ \dots \ \text{Formula (6)}$$

In the Formula (6), "FC1" denotes the first choice ratio of Test Product I or Control 1 in the first test and "FC2" denotes the first choice ratio of Test Product I or Control 1 in the second test.

From Formula (7) below, a feeding intake ratio (%) of Test Product I or Control 1 for each of the beagle dogs was calculated. The results are presented in Table 13-4 below. From the feeding intake ratio (%) of Test Product I or Control 1 in each of the beagle dogs, an average feeding intake ratio (%) of Test Product I or Control 1 for the 20 beagle dogs was calculated. The results are presented in Table 13-5 below.

$$\text{Feeding intake ratio (\%)} = (FI1\text{-}X + FI2\text{-}X)/(FI1\text{-}Y + FI2\text{-}Y) \times 100 \ \dots \ \text{Formula}$$

$$(7)$$

In the Formula (7), "FI1-X" denotes the feeding intake (g) of Test Product I or Control 1 in the first test, "FI2-X" denotes the feeding intake (g) of Test Product I or Control 1 in the second test, "FI1-Y" denotes the total feeding intake (g) of Test Product I and Control 1 in the first test, and "FI2-Y" denotes the total feeding intake (g) of Test Product I and Control 1 in the second test. In the Formula (7), when calculating a feeding intake ratio (%) of Test Product I, both "FI1-X" and "FI2-X" denote the feeding intake (g) of Test Product I, and when calculating a feeding intake ratio (%) of Control 1, both "FI1-X" and "FI2-X" denote the feeding intake (g) of Control 1.

Table 13-1

| Sex | Age (Year) | First Test | | Secont Test | |
|-----|-----|-----|-----|-----|-----|
| | | Test Product I | Control 1 | Control 1 | Test Product I |
| | | FC1 | FC1 | FC2 | FC2 |
| M | 2 | | ○ | | ○ |
| F | 8 | ○ | | | ○ |
| M | 2 | | ○ | | ○ |
| M | 4 | ○ | | ○ | |
| M | 7 | ○ | | | ○ |
| M | 2 | ○ | | | ○ |
| XM | 11 | ○ | | | ○ |
| M | 10 | ○ | | | ○ |
| F | 8 | ○ | | | ○ |
| F | 8 | | ○ | | ○ |
| F | 10 | ○ | | | ○ |
| F | 2 | ○ | | | ○ |
| F | 7 | | ○ | | ○ |
| F | 8 | ○ | | ○ | |
| M | 8 | ○ | | ○ | |
| F | 6 | ○ | | | ○ |
| F | 7 | ○ | | ○ | |
| F | 8 | ○ | | | ○ |
| F | 7 | ○ | | | ○ |
| F | 4 | ○ | | | ○ |
| FC (number) | | 16 | 4 | 4 | 16 |
| FC ratio (%) | | 80 | 20 | 20 | 80 |

Table 13-2

| Sex | Age (Year) | First Test | | | | | |
|-----|-----|-----|-----|-----|-----|-----|-----|
| | | Test Product I | | | Control 1 | | |
| | | Feeding amount (FA) (g) | Residual feed amount (g) | Feeding intake (FI1) (g) | Feeding amount (FA) (g) | Residual feed amount (g) | Feeding intake (FI1) (g) |
| M | 2 | 250 | 0 | 250 | 250 | 116 | 134 |
| F | 8 | 250 | 0 | 250 | 250 | 189 | 61 |
| M | 2 | 250 | 0 | 250 | 250 | 19 | 231 |
| M | 4 | 250 | 0 | 250 | 250 | 183 | 67 |
| M | 7 | 250 | 39 | 211 | 250 | 0 | 250 |
| M | 2 | 250 | 121 | 129 | 250 | 0 | 250 |

(continued)

| Sex | Age (Year) | First Test | | | | | |
|---|---|---|---|---|---|---|---|
| | | Test Product I | | | Control 1 | | |
| | | Feeding amount (FA) (g) | Residual feed amount (g) | Feeding intake (FI1) (g) | Feeding amount (FA) (g) | Residual feed amount (g) | Feeding intake (FI1) (g) |
| XM | 11 | 250 | 0 | 250 | 250 | 250 | 0 |
| M | 10 | 250 | 0 | 250 | 250 | 133 | 117 |
| F | 8 | 250 | 0 | 250 | 250 | 250 | 0 |
| F | 8 | 250 | 0 | 250 | 250 | 247 | 3 |
| F | 10 | 250 | 0 | 250 | 250 | 249 | 1 |
| F | 2 | 250 | 0 | 250 | 250 | 202 | 48 |
| F | 7 | 250 | 0 | 250 | 250 | 206 | 44 |
| F | 8 | 250 | 133 | 117 | 250 | 160 | 90 |
| M | 8 | 250 | 0 | 250 | 250 | 135 | 115 |
| F | 6 | 250 | 26 | 224 | 250 | 250 | 0 |
| F | 7 | 250 | 0 | 250 | 250 | 250 | 0 |
| F | 8 | 250 | 0 | 250 | 250 | 217 | 33 |
| F | 7 | 250 | 196 | 54 | 250 | 249 | 1 |
| F | 4 | 250 | 0 | 250 | 250 | 195 | 55 |

Table 13-3

| Sex | Age (Year) | Second Test | | | | | |
|---|---|---|---|---|---|---|---|
| | | Control 1 | | | Test Product I | | |
| | | Feeding amount (FA) (g) | Residual feed amount (g) | Feeding intake (FI2) (g) | Feeding amount (FA) (g) | Residual feed amount (g) | Feeding intake (FI2) (g) |
| M | 2 | 250 | 23 | 227 | 250 | 0 | 250 |
| F | 8 | 250 | 246 | 4 | 250 | 0 | 250 |
| M | 2 | 250 | 174 | 76 | 250 | 0 | 250 |
| M | 4 | 250 | 183 | 67 | 250 | 0 | 250 |
| M | 7 | 250 | 212 | 38 | 250 | 0 | 250 |
| M | 2 | 250 | 0 | 250 | 250 | 127 | 123 |
| XM | 11 | 250 | 249 | 1 | 250 | 0 | 250 |
| M | 10 | 250 | 194 | 56 | 250 | 0 | 250 |
| F | 8 | 250 | 248 | 2 | 250 | 0 | 250 |
| F | 8 | 250 | 249 | 1 | 250 | 0 | 250 |
| F | 10 | 250 | 250 | 0 | 250 | 0 | 250 |
| F | 2 | 250 | 0 | 250 | 250 | 191 | 59 |
| F | 7 | 250 | 220 | 30 | 250 | 0 | 250 |
| F | 8 | 250 | 146 | 104 | 250 | 201 | 49 |

(continued)

| Sex | Age (Year) | Second Test | | | | | |
|---|---|---|---|---|---|---|---|
| | | Control 1 | | | Test Product I | | |
| | | Feeding amount (FA) (g) | Residual feed amount (g) | Feeding intake (FI2) (g) | Feeding amount (FA) (g) | Residual feed amount (g) | Feeding intake (FI2) (g) |
| M | 8 | 250 | 0 | 250 | 250 | 180 | 70 |
| F | 6 | 250 | 245 | 5 | 250 | 55 | 195 |
| F | 7 | 250 | 0 | 250 | 250 | 145 | 105 |
| F | 8 | 250 | 184 | 66 | 250 | 0 | 250 |
| F | 7 | 250 | 238 | 12 | 250 | 195 | 55 |
| F | 4 | 250 | 176 | 74 | 250 | 0 | 250 |

Table 13-4

| Sex | Age (Year) | Control 1 | Test Product I |
|---|---|---|---|
| | | Feeding intake ratio (%) | Feeding intake ratio (%) |
| M | 2 | 59 | 41 |
| F | 8 | 89 | 11 |
| M | 2 | 64 | 36 |
| M | 4 | 79 | 21 |
| M | 7 | 66 | 34 |
| M | 2 | 34 | 66 |
| XM | 11 | 100 | 0 |
| M | 10 | 75 | 25 |
| F | 8 | 100 | 0 |
| F | 8 | 99 | 1 |
| F | 10 | 100 | 0 |
| F | 2 | 51 | 49 |
| F | 7 | 87 | 13 |
| F | 8 | 44 | 56. |
| M | 8 | 45 | 55 |
| F | 6 | 99 | 1 |
| F | 7 | 65 | 35 |
| F | 8 | 84 | 16 |
| F | 7 | 90 | 10 |
| F | 4 | 80 | 20 |
| Avg. feeding intake ratio (%) | | 75 | 25 |

Table 13-5

|  | Control 1 | Test Product I |
|---|---|---|
| Avg. first choice ratio (%) | 20 | 80 |
| Avg. feeding intake ratio (%) | 25 | 75 |

From the results of Table 13-5, as compared with Control 1, Test Product I was found to be significantly higher in average first choice ratio and average feeding intake ratio and also be significantly lower in residual feed amount, indicating higher palatability for dogs.

<Test Example 11: Palatability test 2 for dog>

-Control 2-

The powdered yeast cell wall (autolysis-type yeast cell wall) (product name: yeast cell wall, product of Asahi Group Foods, Ltd.), which had been used in Preparation Example 1-1, was sprinkled in an amount of 0.3% by mass onto dog food for animal experiments (free of a palatability enhancer, product of KITAYAMA LABES CO. LTD.), followed by homogenously mixing, to prepare pet food (hereinafter may be referred to as "Control 2").

-2-Point Comparison Test-

In the same manner as in the 2-point comparison test of Test Example 10 except that Control 1 was changed to Control 2, an average first choice ratio and an average feeding intake ratio were tested. The results are presented in Table 14.

Table 14

|  | Control 2 | Test Product I |
|---|---|---|
| Avg. first choice ratio (%) | 32 | 68 |
| Avg. feeding intake ratio (%) | 31 | 69 |

From the results of Table 14, as compared with Control 2, Test Product I was found to be significantly higher in average first choice ratio and average feeding intake ratio and also be significantly lower in residual feed amount, indicating higher palatability for dogs.

<Test Example 12: Palatability test 3 for dog>

-Preparation of Test Product II-

The autolysis-type yeast cell wall-derived decomposition product-containing composition (1E) having beer-like fragrance, which had been prepared in Preparation Example 3-1, was sprinkled in an amount of 0.3% by mass onto dog food for animal experiments (free of a palatability enhancer, product of KITAYAMA LABES CO. LTD.), followed by homogenously mixing, to prepare pet food (hereinafter may be referred to as "Test Product II").

-2-Point Comparison Test-

In the same manner as in the 2-point comparison test of Test Example 11 except that Test Product I was changed to Test Product II, an average first choice ratio and an average feeding intake ratio were tested. The results are presented in Table 15.

Table 15

|  | Control 2 | Test Product II |
|---|---|---|
| Avg. first choice ratio (%) | 30 | 70 |
| Avg. feeding intake ratio (%) | 31 | 69 |

From the results of Table 15, as compared with Control 2, Test Product II was found to be significantly higher in average first choice ratio and average feeding intake ratio and also be significantly lower in residual feed amount, indicating higher palatability for dogs.

<Test Example 13: Palatability test 4 for dog>

-Preparation of Test Product III-

The hot-water extraction-type yeast cell wall-derived decomposition product-containing composition (2M) having meat flavor-like roast flavor, which had been prepared in Preparation Example 6-2, was sprinkled in an amount of 0.3% by mass onto dog food for animal experiments (free of a palatability enhancer, product of KITAYAMA LABES CO. LTD.), followed by homogenously mixing, to prepare pet food (hereinafter may be referred to as "Test Product III").

-Control 3-

The powdered yeast cell wall (hot-water extraction-type yeast cell wall) (product name: HG-YCW, product of Asahi Group Foods, Ltd.), which had been used in Preparation Example 2-1, was sprinkled in an amount of 0.3% by mass onto dog food for animal experiments (free of a palatability enhancer, product of KITAYAMA LABES CO. LTD.), followed by homogenously mixing, to prepare pet food (hereinafter may be referred to as "Control 3").

-2-Point Comparison Test-

In the same manner as in the 2-point comparison test of Test Example 10 except that Test Product I was changed to Test Product III and Control 1 was changed to Control 3, an average first choice ratio and an average feeding intake ratio were tested. The results are presented in Table 16.

Table 16

|  | Control 3 | Test Product III |
|---|---|---|
| Avg. first choice ratio (%) | 30 | 70 |
| Avg. feeding intake ratio (%) | 32 | 68 |

From the results of Table 16, as compared with Control 3, Test Product III was found to be significantly higher in average first choice ratio and average feeding intake ratio and also be significantly lower in residual feed amount, indicating higher palatability for dogs.

<Test Example 14: Palatability test 1 for cat>

-Test Product IV-

The autolysis-type yeast cell wall-derived decomposition product-containing composition (4M) having a roast fragrance, which had been prepared in Preparation Example 5-4, was sprinkled in an amount of 0.3% by mass onto cat food for animal experiments (free of a palatability enhancer, product of KITAYAMA LABES CO. LTD.), followed by homogenously mixing, to prepare pet food (hereinafter may be referred to as "Test Product IV").

-Control 4-

Cat food for animal experiments (free of a palatability enhancer, product of KITAYAMA LABES CO. LTD) was used as "Control 4".

-2-Point Comparison Test-

In the same manner as in the 2-point comparison test of Test Example 10 except that Test Product I was changed to Test Product IV, Control 1 was changed to Control 4, the beagle dogs were changed to cats (n=20, 2 years to 17 years after birth, a mixed group of females (F), males (M), or castracepted ones (XF), KITAYAMA LABES CO. LTD.), and the feeding amount in the first test and the second test was changed from 250 g to 70 g, an average first choice ratio and an average feeding intake ratio were tested. The results are presented in Tables 17-1 to 17-5. In Table 17-1, "○" indicates the product that was first chosen.

Cats used in the above 2-point comparison test are cats that underwent identification training for a palatability test and passed it, and regularly received training.

Table 17-1

| Sex | Age (Year) | First Test | | Second Test | |
|---|---|---|---|---|---|
| | | Test Product I | Control 1 | Control 1 | Test Product I |
| | | FC1 | FC1 | FC2 | FC2 |
| M | 2 | ○ | | | ○ |
| M | 10 | ○ | | ○ | |
| M | 15 | ○ | | | ○ |
| XF | 16 | ○ | | | ○ |
| F | 9 | | ○ | | ○ |
| XF | 15 | | ○ | | ○ |
| M | 11 | | ○ | ○ | |
| M | 8 | ○ | | | ○ |
| F | 12 | ○ | | | ○ |
| M | 7 | | ○ | | ○ |
| M | 10 | ○ | | ○ | |
| F | 11 | ○ | | | ○ |
| XF | 16 | ○ | | ○ | |
| F | 7 | | ○ | | ○ |
| F | 7 | ○ | | | ○ |
| F | 8 | ○ | | | ○ |
| M | 10 | ○ | | | ○ |
| F | 7 | ○ | | | ○ |
| XF | 16 | | ○ | | ○ |
| M | 17 | ○ | | | ○ |
| FC (Number) | | 14 | 6 | 4 | 16 |
| FC ratio (%) | | 70 | 30 | 20 | 80 |

Table 17-2

| Sex | Age (Year) | First Test | | | | | |
|---|---|---|---|---|---|---|---|
| | | Test Product I | | | Control 1 | | |
| | | Feeding amount (FA) (g) | Residual feed amount (g) | Feeding intake (FI1) (g) | Feeding amount (FA) (g) | Residual feed amount (g) | Feeding intake (GI1) (g) |
| M | 2 | 70 | 13 | 57 | 70 | 70 | 0 |
| M | 10 | 70 | 13 | 57 | 70 | 70 | 0 |
| M | 15 | 70 | 3 | 67 | 70 | 70 | 0 |
| XF | 16 | 70 | 66 | 4 | 70 | 62 | 8 |

(continued)

| Sex | Age (Year) | First Test | | | | | |
|---|---|---|---|---|---|---|---|
| | | Test Product I | | | Control 1 | | |
| | | Feeding amount (FA) (g) | Residual feed amount (g) | Feeding intake (FI1) (g) | Feeding amount (FA) (g) | Residual feed amount (g) | Feeding intake (GI1) (g) |
| F | 9 | 70 | 36 | 34 | 70 | 69 | 1 |
| XF | 15 | 70 | 48 | 22 | 70 | 69 | 1 |
| M | 11 | 70 | 11 | 59 | 70 | 62 | 8 |
| M | 8 | 70 | 37 | 33 | 70 | 67 | 3 |
| F | 12 | 70 | 36 | 34 | 70 | 66 | 4 |
| M | 7 | 70 | 14 | 56 | 70 | 63 | 7 |
| M | 10 | 70 | 16 | 54 | 70 | 48 | 22 |
| F | 11 | 70 | 16 | 54 | 70 | 46 | 24 |
| XF | 16 | 70 | 51 | 19 | 70 | 69 | 1 |
| F | 7 | 70 | 69 | 1 | 70 | 24 | 46 |
| F | 7 | 70 | 43 | 27 | 70 | 68 | 2 |
| F | 8 | 70 | 23 | 47 | 70 | 70 | 0 |
| M | 10 | 70 | 9 | 61 | 70 | 70 | 0 |
| F | 7 | 70 | 33 | 37 | 70 | 58 | 12 |
| XF | 16 | 70 | 62 | 8 | 70 | 55 | 15 |
| M | 17 | 70 | 8 | 62 | 70 | 70 | 0 |

Table 17-3

| Sex | Age (Year) | Second Test | | | | | |
|---|---|---|---|---|---|---|---|
| | | Control 1 | | | Test Product I | | |
| | | Feeding amount (FA) (g) | Residual feed amount (g) | Feeding intake (FI2) (g) | Feeding amount (FA) (g) | Residual feed amount (g) | Feeding intake (FI2) (g) |
| M | 2 | 70 | 70 | 0 | 70 | 33 | 37 |
| M | 10 | 70 | 69 | 1 | 70 | 32 | 38 |
| M | 15 | 70 | 57 | 13 | 70 | 7 | 63 |
| XF | 16 | 70 | 61 | 9 | 70 | 66 | 4 |
| F | 9 | 70 | 70 | 0 | 70 | 44 | 26 |
| XF | 15 | 70 | 50 | 20 | 70 | 59 | 11 |
| M | 11 | 70 | 43 | 27 | 70 | 54 | 16 |
| M | 8 | 70 | 67 | 3 | 70 | 27 | 43 |
| F | 12 | 70 | 67 | 3 | 70 | 40 | 30 |
| M | 7 | 70 | 20 | 50 | 70 | 69 | 1 |
| M | 10 | 70 | 56 | 14 | 70 | 40 | 30 |
| F | 11 | 70 | 70 | 0 | 70 | 0 | 70 |

(continued)

| Sex | Age (Year) | Second Test | | | | | |
|---|---|---|---|---|---|---|---|
| | | Control 1 | | | Test Product I | | |
| | | Feeding amount (FA) (g) | Residual feed amount (g) | Feeding intake (FI2) (g) | Feeding amount (FA) (g) | Residual feed amount (g) | Feeding intake (FI2) (g) |
| XF | 16 | 70 | 53 | 17 | 70 | 70 | 0 |
| F | 7 | 70 | 39 | 31 | 70 | 69 | 1 |
| F | 7 | 70 | 67 | 3 | 70 | 42 | 28 |
| F | 8 | 70 | 70 | 0 | 70 | 28 | 42 |
| M | 10 | 70 | 69 | 1 | 70 | 32 | 38 |
| F | 7 | 70 | 58 | 12 | 70 | 29 | 41 |
| XF | 16 | 70 | 70 | 0 | 70 | 39 | 31 |
| M | 17 | 70 | 62 | 8 | 70 | 33 | 37 |

Table 17-4

| Sex | Age (Year) | Control 1 | Test Product I |
|---|---|---|---|
| | | Feeding intake ratio (%) | Feeding intake ratio (%) |
| M | 2 | 100 | 0 |
| M | 10 | 99 | 1 |
| M | 15 | 91 | 9 |
| XF | 16 | 32 | 68 |
| F | 9 | 99 | 1 |
| XF | 15 | 66 | 34 |
| M | 11 | 63 | 37 |
| M | 8 | 93 | 7 |
| F | 12 | 90 | 10 |
| M | 7 | 45 | 55 |
| M | 10 | 70 | 30 |
| F | 11 | 85 | 15 |
| XF | 16 | 48 | 53 |
| F | 7 | 3 | 97 |
| F | 7 | 92 | 8 |
| F | 8 | 100 | 0 |
| M | 10 | 99 | 1 |
| F | 7 | 76 | 24 |
| XF | 16 | 67 | 33 |
| M | 17 | 91 | 9 |
| Avg. feeding intake ratio (%) | | 75 | 2 5 |

Table 17-5

|  | Control 4 | Test Product IV |
|---|---|---|
| Avg. first choice ratio (%) | 25 75 | |
| Avg. feeding intake ratio (%) | 25 | 75 |

From the results of Table 17-5, as compared with Control 4, Test Product IV was found to be significantly higher in average first choice ratio and average feeding intake ratio and also be significantly lower in residual feed amount, indicating higher palatability for cats.

<Test Example 15: Palatability test 2 for cat>

-Control 5-

The powdered yeast cell wall (autolysis-type yeast cell wall) (product name: yeast cell wall, product of Asahi Group Foods, Ltd.), which had been used in Preparation Example 1-1, was sprinkled in an amount of 0.3% by mass onto cat food for animal experiments (free of a palatability enhancer, product of KITAYAMA LABES CO. LTD.), followed by homogenously mixing, to prepare pet food (hereinafter may be referred to as "Control 5").

-2-Point Comparison Test-

In the same manner as in the 2-point comparison test of Test Example 14 except that Control 4 was changed to Control 5, an average first choice ratio and an average feeding intake ratio were tested. The results are presented in Table 18.

Table 18

|  | Control 5 | Test Product IV |
|---|---|---|
| Avg. first choice ratio (%) | 20 | 80 |
| Avg. feeding intake ratio (%) | 30 | 70 |

From the results of Table 18, as compared with Control 5, Test Product IV was found to be significantly higher in average first choice ratio and average feeding intake ratio and also be significantly lower in residual feed amount, indicating higher palatability for cats.

<Test Example 16: Palatability test 3 for cat>

-Preparation of Test Product V-

The autolysis-type yeast cell wall-derived decomposition product-containing composition (1E) having a beer-like fragrance, which had been prepared in Preparation Example 3-1, was sprinkled in an amount of 0.3% by mass onto cat food for animal experiments (free of a palatability enhancer, product of KITAYAMA LABES CO. LTD.), followed by homogenously mixing, to prepare pet food (hereinafter may be referred to as "Test Product V").

-2-Point Comparison Test-

In the same manner as in the 2-point comparison test of Test Example 15 except that Test Product VI was changed to Test Product V, an average first choice ratio and an average feeding intake ratio were tested. The results are presented in Table 19.

Table 19

|  | Control 5 | Test Product V |
|---|---|---|
| Avg. first choice ratio (%) | 32 | 68 |
| Avg. feeding intake ratio (%) | 35 | 65 |

From the results of Table 19, as compared with Control 5, Test Product V was found to be significantly higher in average first choice ratio and average feeding intake ratio and also be significantly lower in residual feed amount, indicating higher palatability for cats.

<Test Example 17: Palatability test 4 for cat>

-Preparation of Test Product VI-

The hot-water extraction-type yeast cell wall-derived decomposition product-containing composition (2M) having a meat flavor-like roast flavor, which had been prepared in Preparation Example 6-2, was sprinkled in an amount of 0.3% by mass onto cat food for animal experiments (free of a palatability enhancer, product of KITAYAMA LABES CO. LTD.), followed by homogenously mixing, to prepare pet food (hereinafter may be referred to as "Test Product IV").

-Control 6-

The powdered yeast cell wall (hot-water extraction-type yeast cell wall) (product name: HG-YCW, product of Asahi Group Foods, Ltd.), which had been used in Preparation Example 2-1, was sprinkled in an amount of 0.3% by mass onto cat food for animal experiments (free of a palatability enhancer, product of KITAYAMA LABES CO. LTD.), followed by homogenously mixing, to prepare pet food (hereinafter may be referred to as "Control 6").

-2-Point Comparison Test-

In the same manner as in the 2-point comparison test of Test Example 14 except that Test Product IV was changed to Test Product VI and Control 4 was changed to Control 6, an average first choice ratio and an average feeding intake ratio were tested. The results are presented in Table 20.

Table 20

|  | Control 6 | Test Product VI |
|---|---|---|
| Avg. first choice ratio (%) | 27 | 73 |
| Avg. feeding intake ratio (%) | 28 | 72 |

From the results of Table 20, as compared with Control 6, Test Product VI was found to be significantly higher in average first choice ratio and average feeding intake ratio and also be significantly lower in residual feed amount, indicating higher palatability for cats.

In Test Examples 10 to 17, comparison between the pet foods supplemented with nothing (Control 1 or Control 4) and the pet food supplemented with the yeast cell wall (Control 2, Control 3, Control 5, or Control 6) indicates that the addition of the yeast cell wall to the pet foods somewhat improved palatability for animals. It is considered that this is because the yeast cell wall of beer yeast contains, for example, yeast odor to some extent. However, both when adding the yeast cell wall-derived decomposition product-containing composition (enzymatically treated product) to the pet food and when adding the yeast cell wall-derived decomposition product-containing composition (heat-treated product) to the pet food, not only did palatability improve remarkably as compared with typical pet foods, but also palatability improved remarkably even as compared with the pet foods supplemented with the yeast cell wall. In particular, it was found that the yeast cell wall-derived decomposition product-containing composition (heat-treated product) was able to suitably improve palatability for cats.

Industrial Applicability

The method of the present invention for producing a yeast cell wall-derived decomposition product-containing composition can increase the solubility ratio and the dispersion stability of a yeast cell wall. The method of the present invention does not require any separation/removal operation of the insoluble fraction of the yeast cell wall, has high working efficiency and production efficiency, and is low cost. Thus, this method can be suitably used as a method of effectively using a yeast cell wall that has conventionally been disposed of as the insoluble matter after the extraction of a yeast extract.

The cell wall-derived decomposition production-containing composition of the present invention is excellent in solubility and dispersion stability, and thus can be suitably used in various fields such as a food field, a bio field, and a cosmetic field. In particular, it can be suitably used as, for example, foods or drinks, alcohols, raw materials for pet foods, palatability enhancers for pet foods, and media.

The yeast cell wall-derived decomposition production-containing composition, the roast flavor-imparting composition, and the roast color-imparting composition of the present invention are excellent in solubility and dispersion stability, and have a favorable roast flavor and a favorable fat and oil feeling, and a favorable roast color. Thus, they can be used for at least one selected from the group consisting of roast flavor enhancement, roast flavor impartment, roast color enhancement, roast color impartment, fat and oil feeling enhancement, and fat and oil feeling impartment for, for example, food or drink, pet food, and a pet food palatability enhancer. Also, they can be used as additives or raw materials thereof such as perfumes and perfume raw materials for at least one selected from the group consisting of roast flavor enhancement, roast flavor impartment, roast color enhancement, roast color impartment, fat and oil feeling enhancement, and fat and oil feeling impartment.

The beer-like fragrance-imparting composition of the present invention is excellent in solubility and dispersion stability, has less off-flavor, and has favorable beer-like fragrance. Thus, it can be suitably used for at least one selected from the group consisting of beer-like fragrance enhancement and beer-like fragrance impartment of food or drink. Also, it can be used as additives or raw materials thereof such as perfumes, perfume raw materials, food raw materials, and extraction raw materials for at least one selected from the group consisting of beer-like fragrance enhancement and beer-like fragrance impartment.

The food or drink of the present invention includes the yeast cell wall-derived decomposition production-containing composition of the present invention, and thus can be suitably used as food or drink having, for example, a beer-like fragrance, a roast flavor, a fat and oil feeling, and a roast color.

The pet food palatability enhancer of the present invention includes the yeast cell wall-derived decomposition production-containing composition of the present invention, and thus can be suitably used as additives or raw materials thereof for pet foods.

## Claims

1. A method for producing a yeast cell wall-derived decomposition product-containing composition, the method comprising
enzymatically treating a cell wall of yeast with exoglucanase.

2. The method according to claim 1, wherein the enzymatically treating further comprises a protease treatment of the cell wall of yeast.

3. The method according to claim 2, wherein the enzymatically treating further comprises an exopeptidase treatment of the cell wall of yeast.

4. The method according to any one of claims 1 to 3, further comprising performing a heat treatment to an enzymatically treated product obtained in the enzymatically treating.

5. The method according to claim 4, further comprising adjusting pH of the enzymatically treated product to be alkaline, wherein the heat treatment is heat-treating an alkaline enzymatically treated product obtained in the adjusting.

6. A yeast cell wall-derived decomposition product-containing composition comprising

a decomposition product derived from a cell wall of yeast,
wherein a volume of a precipitate is 50 mL or less, the precipitate forming when 100 mL of the yeast cell wall-derived decomposition product-containing composition having a solid content concentration of 10% by mass is left to stand still at 25°C under normal pressure for 48 hours.

7. The yeast cell wall-derived decomposition product-containing composition according to claim 6, wherein relative to a solid content mass of the yeast cell wall-derived decomposition product-containing composition, a content ratio of a total free amino acid is 7% by mass or more and a content ratio of a reducing sugar is 1% by mass or more.

8. The yeast cell wall-derived decomposition product-containing composition according to claim 6 or 7, wherein relative to a solid content mass of the yeast cell wall-derived decomposition product-containing composition, a content ratio of a total free amino acid is 12% by mass or more and a content ratio of a reducing sugar is 10% by mass or more.

9. The yeast cell wall-derived decomposition product-containing composition according to any one of claims 6 to 8, wherein the yeast cell wall-derived decomposition product-containing composition comprises at least one compound selected from the group consisting of ethyl caproate, ethyl caprylate, ethyl caprate, phenylethyl alcohol, and capric acid.

10. The yeast cell wall-derived decomposition product-containing composition according to claim 9, wherein the decomposition product derived from the cell wall of yeast is a decomposition product derived from a cell wall of beer yeast.

11. A yeast cell wall-derived decomposition product-containing composition comprising

a decomposition product derived from a cell wall of yeast,
wherein the yeast cell wall-derived decomposition product-containing composition comprises at least one compound selected from the group consisting of pyrazine, methylpyrazine, 2,5-dimethylpyrazine, 2,6-dimethylpyrazine, 2,3-dimethylpyrazine, and furaneol.

12. A beer-like fragrance-imparting composition comprising
the yeast cell wall-derived decomposition product-containing composition according to claim 9 or 10.

13. A roast flavor-imparting composition comprising
the yeast cell wall-derived decomposition product-containing composition according to claim 11.

14. A roast color-imparting composition comprising
the yeast cell wall-derived decomposition product-containing composition according to claim 11.

15. Food or drink comprising
the yeast cell wall-derived decomposition product-containing composition according to any one of claims 6 to 11.

16. A pet food palatability enhancer comprising
the yeast cell wall-derived decomposition product-containing composition according to any one of claims 6 to 11.

FIG. 1

FIG. 2

FIG. 3

FIG. 4

FIG. 5

FIG. 6

EP 4 029 945 A1

FIG. 7

EP 4 029 945 A1

FIG. 8

+EI TIC Scan sample07.D

8.0

11.0

13.7

16.8

20.2

Counts vs. Measurement period (min)

# FIG. 9

# FIG. 10

## INTERNATIONAL SEARCH REPORT

| International application No. |
|---|
| PCT/JP2020/034473 |

**A. CLASSIFICATION OF SUBJECT MATTER**

C12P 1/00(2006.01)i; A23K 10/14(2016.01)i; A23K 10/16(2016.01)i; A23K 10/38(2016.01)i; A23K 20/121(2016.01)i; A23K 20/137(2016.01)i; A23K 20/142(2016.01)i; A23K 20/158(2016.01)i; A23K 20/163(2016.01)i; A23K 50/40(2016.01)i; A23L 5/40(2016.01)i; A23L 27/00(2016.01)i; A23L 27/10(2016.01)i; A23L 27/20(2016.01)i; C12N 1/16(2006.01)i

FI: C12P1/00 A; A23L5/40; C12N1/16 J; A23L27/00 D; A23L27/00 Z; A23L27/10 H; A23L27/20 G; A23K50/40; A23K10/16; A23K10/14; A23K20/142; A23K20/163; A23K20/158; A23K10/38; A23K20/137; A23K20/121

According to International Patent Classification (IPC) or to both national classification and IPC

**B. FIELDS SEARCHED**

Minimum documentation searched (classification system followed by classification symbols)

C12P1/00; A23K10/14; A23K10/16; A23K10/38; A23K20/121; A23K20/137; A23K20/142; A23K20/158; A23K20/163; A23K50/40; A23L5/40; A23L27/00; A23L27/10; A23L27/20; C12N1/16

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

JSTPlus/JMEDPlus/JST7580 (JDreamIII); CAplus/FSTA/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2014-515273 A (DSM IP ASSETS B.V.) 30 June 2014 | 1–4 |
| Y | (2014-06-30) claims, paragraphs [0002]–[0034] | 1–16 |
| Y | JP 2018-531586 A (DSM IP ASSETS B.V.) 01 November 2018 (2018-11-01) claims 1, 2, paragraphs [0007], [0051] | 1–16 |
| Y | JP 2010-99001 A (THE KITASATO INSTITUTE) 06 May 2010 (2010-05-06) claims, paragraph [0022] | 5–16 |
| Y | JP 2011-526781 A (NESTEC SA) 20 October 2011 (2011-10-20) claims, paragraph [0003] | 5–16 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 30 October 2020 (30.10.2020) | 17 November 2020 (17.11.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2020/034473

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2000-312562 A (AJINOMOTO CO., INC.) 14 November 2000 (2000-11-14) paragraph [0011] | 5-16 |
| Y | JP 2012-161268 A (TABLEMARK CO., LTD.) 30 August 2012 (2012-08-30) claims | 9-16 |
| Y | JP 2012-16301 A (AKITA PREFECTURAL UNIVERSITY et al.) 26 January 2012 (2012-01-26) abstract, paragraph [0011] | 9-16 |
| Y | WO 2013/140901 A1 (ASAHI GROUP HOLDINGS, LTD.) 26 September 2013 (2013-09-26) claims | 9-16 |

Form PCT/ISA/210 (continuation of second sheet) (January 2015)

**INTERNATIONAL SEARCH REPORT**

| International application No. |
| --- |
| PCT/JP2020/034473 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

---

Form PCT/ISA/210 (continuation of first sheet (2)) (January 2015)

**INTERNATIONAL SEARCH REPORT**

| International application No. |
|---|
| PCT/JP2020/034473 |

<Continuation of Box No. III>

    (Invention 1) Claims 1-5
    The invention in claims 1-4 lacks novelty in light of document 1, and thus does not have a special technical feature. However, a special technical feature was found in the invention in claim 5. Therefore, claims 1-5 are classified as invention 1.

    (Invention 2) Claims 6-10, 12, and 15-16
    The invention in claims 6-10, 12, and 15-16 share the common technical feature of a "composition containing yeast cell wall-derived decomposition products" with claims 1-5 classified as invention 1, but since said technical feature does not make a contribution over the prior art in light of document 1, this technical feature cannot be considered a special technical feature. Therefore, claims 6-10, 12, and 15-16 cannot be classified as invention 1.
    In addition, claims 6-10, 12, and 15-16 are classified as invention 2.

    (Invention 3) Claims 11 and 13-16
    The invention in claims 11 and 13-16 share the common technical feature of a "composition containing yeast cell wall-derived decomposition products" with claims 1-5 classified as invention 1, but since said technical feature does not make a contribution over the prior art in light of document 1, this technical feature cannot be considered a special technical feature. Therefore, claims 11 and 13-16 cannot be classified as invention 1.
    In addition, claims 11 and 13-16 are classified as invention 3.

Form PCT/ISA/210 (extra sheet) (January 2015)

## INTERNATIONAL SEARCH REPORT
Information on patent family members

International application No.

PCT/JP2020/034473

| Patent Documents referred in the Report | Publication Date | Patent Family | Publication Date |
|---|---|---|---|
| JP 2014-515273 A | 30 Jun. 2014 | US 2014/0099402 A1 claims, paragraphs [0002]-[0052] EP 2713774 A1 | |
| JP 2018-531586 A | 01 Nov. 2018 | US 2018/0271130 A1 claims 1, 2, paragraphs [0007], [0071] | |
| JP 2010-99001 A | 06 May 2010 | (Family: none) | |
| JP 2011-526781 A | 20 Oct. 2011 | US 2011/0189367 A1 claims, paragraph [0005] EP 2296483 A1 | |
| JP 2000-312562 A | 14 Nov. 2000 | (Family: none) | |
| JP 2012-161268 A | 30 Aug. 2012 | (Family: none) | |
| JP 2012-16301 A | 26 Jan. 2012 | (Family: none) | |
| WO 2013/140901 A1 | 26 Sep. 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5982696 B
- JP 2018531586 A

- JP 2017086079 A

**Non-patent literature cited in the description**

- Palatability for Pet Foods. Latest Trends in Pet Foods and Drugs. CMC Publishing, 16 December 2013, 29-35